# EUROPÄISCHE PATENTANMELDUNG

(11) **EP 2 269 724 A1**
(43) Veröffentlichungstag der Anmeldung: **05.01.2011**
(21) Anmeldenummer: 10171762.7
(22) Anmeldetag: 29.10.2005
(51) Int. Cl.: B01J 19/00, G01N 33/53, G01N 33/543

(54) **Verfahren zur Bestimmung eines oder mehrerer Analyten in komplex zusammengesetzten Proben biologischen Ursprungs und dessen Verwendung**

(62) Teilanmeldung aus: 05797778.7
(71) Anmelder: Bayer Technology Services GmbH, 51368 Leverkusen (DE)
(72) Erfinder: PAWLAK, Michael, 72147, Nehren (DE); SCHICK, Eginhard, 79618, Rheinfelden (DE); VENTURI, Miro, 20017, Rho/Milano (IT); EHRAT, Markus, 4312, Magden (DE)

(57) **Zusammenfassung**

Die vorliegende Erfindung betrifft ein Mikroarray zur quantitativen Bestimmung eines oder mehrerer Analyten in komplex zusammengesetzten Proben biologischen Ursprungs, welche in Messbereichen des Mikroarrays ein erste Vielzahl von jeweils kleinen Mengen komplex zusammengesetzter Proben biologischen Ursprungs immobilisiert sind, und mindestens eine zweite Vielzahl von Messbereichen in besagtem Array auf dem festen Träger bereitgestellt ist, in welchen Messbereichen den nachzuweisenden Analyten gleichartige Stoffe in unterschiedlichen Konzentrationen immobilisiert sind, welche geeignet sind, mittels Kontaktierung des Mikroarrays mit einer ersten Lösung enthaltend ein oder mehrere Bindungsreagentien als spezifische Bindungspartner für die in der ersten Vielzahl von diskreten Messbereichen in den aufgebrachten komplex zusammengesetzten Proben biologischen Ursprungs enthaltenen, nachzuweisenden Analyten und die in der zweiten Vielzahl von diskreten Messbereichen enthaltenen, besagten nachzuweisenden Analyten gleichartigen Stoffe und nachfolgende ortsaufgelöste Messung von ersten optischen Signalen, welche von diskreten Messbereichen eines oder mehrerer Arrays, welche mit der ersten Lösung in Kontakt gebracht wurden, ausgehen, sowie Aufzeichnung dieser ersten optischen Signale, eine Kalibrationskurve für besagte in den immobiliserten komplex zusammengesetzten Proben enthaltenen, quantitativ nachzuweisenden Analyten zu erzeugen.

Die vorliegende Erfindung betrifft außerdem ein darauf beruhendes quantitatives Nachweisverfahren.

## Beschreibung

Die vorliegende Erfindung betrifft ein Verfahren zum Nachweis eines oder mehrerer Analyten in einer oder mehreren komplex zusammengesetzten Proben biologischen Ursprungs

### Technischer Hintergrund

Für zahlreiche Anwendungsgebiete ist es erforderlich, eine Vielzahl von biologisch relevanten Analyten in einer komplexen Probe zu bestimmen, beispielsweise in diagnostischen Verfahren zur Bestimmung des Gesundheitszustandes eines Individuums oder des Effekts therapeutischer Behandlung oder in der Pharmaforschung und -entwicklung zur Bestimmung der Beeinflussung biologischer Systeme, wie beispielsweise eines Organismus, und deren komplexer Funktionsweise durch äußere Einwirkungen, wie beispielsweise mittels Zuführung biologisch aktiver Verbindungen.

Während bekannte analytische Trennverfahren im allgemeinen dahingehend optimiert sind, innerhalb möglichst kurzer Zeit eine möglichst große Anzahl von in einer gegebenen Probe enthaltenen Verbindungen gemäß eines vorgegebenen physikalisch-chemischen Parameters, wie beispielsweise des Molekulargewichts oder des Quotienten aus molekularer Ladung und Masse, aufzutrennen, beruhen Bioaffinitäts-Nachweisverfahren darauf, mit jeweils einem biologischen oder biochemischen oder synthetischen Erkennungselement möglichst hoher Spezifizität den entsprechenden (einzelnen) Analyten hochselektiv in einer komplex zusammengesetzten Probe zu erkennen und zu binden. Der Nachweis einer Vielzahl unterschiedlicher Verbindungen erfordert also die Verwendung einer entsprechenden Anzahl unterschiedlicher spezifischer Erkennungselemente.

Ein Nachweisverfahren basierend auf Bioaffinitätsreaktionen kann sowohl in einer homogenen Lösung als auch an der Oberfläche eines festen Trägers erfolgen. Je nach dem spezifischen Aufbau des Verfahrens sind nach Bindung der Analyten an die entsprechenden Erkennungselemente und gegebenenfalls weitere Nachweissubstanzen sowie gegebenenfalls zwischen verschiedenen Verfahrensschritten jeweils Waschschritte notwendig, um die gebildeten Komplexe aus den Erkennungselementen und den nachzuweisenden Analyten sowie gegebenenfalls weiteren Nachweissubstanzen vom Rest der Probe und der gegebenenfalls eingesetzten zusätzlichen Reagentien zu trennen.

Relativ weit verbreitet sind inzwischen Verfahren zum gleichzeitigen Nachweis einer Vielzahl unterschiedlicher Nukleinsäuren in einer Probe mithilfe entsprechender komplementärer, auf einem festen Träger in diskreten, räumlich getrennten Messbereichen immobilisierter Nukleinsäuren als Erkennungselementen. Beispielsweise sind, basierend auf einfachen Glas- oder Mikroskop-Plättchen, Arrays von Oligonukleotiden als Erkennungselementen mit einer sehr hohen Feature-Dichte (Dichte von Messbereichen auf einem gemeinsamen festen Träger) bekannt. Beispielsweise werden in der US-Patentschrift No. 5,445,934 (Affymax Technologies) Arrays von Oligonukleotiden mit einer Dichte von mehr als 1000 Features pro Quadratzentimeter beschrieben und beansprucht.

Seit kurzem häufen sich auch Beschreibungen von Arrays und damit ausgeführter Nachweisverfahren ähnlicher Art zur gleichzeitigen Bestimmung einer Vielzahl von Proteinen, beispielsweise in der US-Patentschrift No. 6,365,418 B1, insbesondere mit Arrays von immobilisierten Antikörpern als Erkennungselementen für die nachzuweisenden Analyten.

In den Patentschriften für derartige sogenannte "Mikroarrays", sowohl für den Nachweis von Nukleinsäuren als auch anderer Biopolymere, wie beispielsweise Proteine, wird jeweils beschrieben, dass eine Vielzahl unterschiedlicher spezifischer Erkennungselemente in diskreten Messbereichen zur Erzeugung eines Arrays für die Analyterkennung immobilisiert und anschließend die zu untersuchende Probe mit den darin (gegebenenfalls in einer komplexen Mischung) vorhandenen Analyten mit diesem sogenannten "Capture-Array" in Kontakt gebracht wird. Den bekannten Beschreibungen folgend, liegen unterschiedliche spezifische Erkennungselemente dabei jeweils in einer möglichst hochreinen Form in unterschiedlichen diskreten Messbereichen vor, so dass an Messbereiche mit unterschiedlichen Erkennungselementen im allgemeinen unterschiedliche Analyten aus der Probe binden.

Diese Art bekannter Assays erfordert, dass die in möglichst hochreiner Form zu immobilisierenden spezifischen Erkennungselemente mittels teilweise sehr aufwendiger Arbeitsschritte gereinigt und angereichert und werden. Da sich unterschiedliche Erkennungselemente mehr oder minder stark in ihren physikalisch-chemischen Eigenschaften (z. B. in ihrer Polarität) unterscheiden, gibt es auch entsprechende Unterschiede in den Bedingungen für eine optimale Immobilisierung dieser Erkennungselemente, beispielsweise durch Adsorption oder kovalente Bindung, in diskreten Messbereichen auf einem gemeinsamen festen Träger, gegebenenfalls auf einer darauf aufgebrachten Haftvermittlungsschicht. Demzufolge können die zur Immobilisierung einer Vielzahl unterschiedlicher Erkennungselemente gewählten Immobilisierungsbedingungen (wie z. B. Art der Haftvermittlungsschicht) kaum gleichzeitig für alle Erkennungselemente ein Optimum, sondern lediglich einen Kompromiss zwischen den Immobilisierungseigenschaften der verschiedenen Erkennungselemente darstellen. Auf dieser Art von "Capture-Arrays" kann außerdem jeweils nachteilig nur eine zugeführte Probe pro Array auf darin enthaltene Analyten untersucht werden.

Es bestand daher das Bedürfnis nach einem geänderten Assay-Aufbau, welcher es ermöglicht, eine Vielzahl von Proben in einem Array oder mehreren Arrays auf einem gemeinsamen Träger gleichzeitig oder in mehreren Arrays auf mehreren Trägern sequentiell auf in den Proben enthaltene Analyten zu untersuchen. Dazu wäre es zweckmäßig, nicht die unterschiedlichen spezifischen Erkennungselemente, sondern die zu untersuchenden Proben selbst direkt, d.h. unbehandelt, oder nach möglichst wenigen Vorbereitungsschritten in diskreten Messbereichen in einem oder mehreren Arrays auf einem oder mehreren Trägern aufzutragen. Ein solcher Assay-Aufbau soll nachfolgend als eine "invertierte Assay-Architektur" bezeichnet werden.

Zur Befriedigung dieses Bedürfnisses wurden vor kurzem, z. B. in Paweletz et al., Oncogene 2001, Vol. 20, 1981 - 1989, derartige Arrays zum Proteinnachweis basierend auf einer invertierten Assay-Architektur unter der Bezeichnung "Reverse Phase Protein Microarrays" vorgeschlagen.

Ein gemeinsames Problem solcher Verfahren zum Analytnachweis mithilfe von Mikroarrays wie auch in noch allgemeinerer Form von an Oberflächen durchgeführten Nachweisverfahren, welche auf der spezifischen Bindung von Bindungsreagentien als spezifischen Bindungspartnern an die nachzuweisenden Analyten beruhen, ist das Auftreten unspezifischer Bindungsereignisse, welche nicht auf der spezifischen Wechselwirkung zwischen den Analyten und den zu ihrem Nachweis eingesetzten Bindungsreagentien und gegebenenfalls weiteren Detektionsreagentien beruhen.

In der US-Patentschrift Nr. 5,726,064 werden verschiedene Methoden zur Kompensation von Störungen der Assay-Signale durch Hintergrundsignale, wie beispielsweise Hintergrundfluoreszenz, welche insbesondere auf unspezifischen Bindungsereignissen beruhen kann, sowie Änderungen von Temperatur oder pH, welche die beobachteten Assay-Signale beeinträchtigen könnten, beschrieben. Im wesentlichen beruhen diese Methoden auf der Bereitstellung zusätzlicher für derartige Kompensationszwecke ausgewiesener Bereiche, neben den für die Erzeugung der Assay-Signale ausgewiesenen Bereichen, auf einem gemeinsamen festen Träger.

In der US-Patentanmeldung 2004/0043508 A1 wird der Umfang spezifischer und unspezifischer Bindung an unterschiedliche Oberflächen für die Herstellung von "Capture Arrays", welche mit unterschiedlichen Materialien zur Minimierung unspezifischer Bindung behandelt wurden, beschrieben. Vorteilhaft zur Verminderung unspezifischer Bindung werden dabei elektrostatisch wirkende Beschichtungen genannt.

Unterschiedliche Oberflächenbeschichtungen zur Minimierung unspezifischer Bindung, insbesondere solche umfassend Polyethylenglycole, und Messungen des absoluten und des relativen Anteils unspezifischer Bindung werden auch in US-Patent Nr. 5,677,196 beschrieben, allerdings wieder in einem (Sensor-) Format entsprechend Capture-Arrays.

Den genannten Methoden zur Minimierung der Auswirkungen unspezifischer Bindung auf die Assay-Resultate ist gemeinsam, dass sie auf der Veränderung der Beschaffenheit der Oberfläche des Trägers beruhen, um dadurch die Bindung von Analyten oder anderer im Assay eingesetzter Bindungs- und Nachweisreagentien außerhalb der Messbereiche mit dort immobilisierten analytspezifischen Erkennungselementen zu verhindern oder zu minimieren. Zugleich wird stillschweigend vorausgesetzt, dass in besagten Messbereichen selbst eine unspezifische Bindung nicht stattfindet; denn solche Effekte könnten mithilfe der beschriebenen Verfahren nicht berücksichtigt werden. Im Falle von Capture-Arrays, mit einer wohldefinierten Zusammensetzung der in den Messbereichen aufgebrachten Verbindungen, nämlich im allgemeinen einer einheitlichen Form von Erkennungselementen innerhalb eines Messbereichs, kann diese genannte Voraussetzung unter anderem durch sorgfältige Auswahl der Erkennungselemente und der im Assay eingesetzten Bindungs- und Detektionsreagentien weitgehend erfüllt werden.

Im Falle von Arrays für Assays mit einer invertierten Assay-Architektur, d.h. mit in den Messbereichen immobilisierten, komplex zusammengesetzten Proben biologischen Ursprungs, ist die genannte Voraussetzung schwer und kaum verlässlich zu erfüllen, da die aufgebrachten Proben eine unbekannte Zusammensetzung mit einer Vielzahl unterschiedlicher Verbindungen der biologischen Probenmatrix haben. Daher ist zu erwarten, dass auch innerhalb der Messbereiche unspezifische Bindung von Bindungs- und gegebenenfalls eingesetzten Detektionsreagentien in einem erheblichen Ausmaß stattfinden kann.

Die Aufgabe der vorliegenden Erfindung ist es, für derartige Assays mit Arrays von Messbereichen, in denen komplex zusammengesetzte Proben biologischen Ursprungs mit darin enthaltenen, nachzuweisenden Analyten immobilisiert wurden, ein Verfahren bereitzustellen, welches ermöglicht, den Anteil von durch unspezifische Wechselwirkung mit den zugegebenen Bindungsreagentien und mit den gegebenenfalls zugegebenen Detektionsreagentien erzeugten optischen Signalen an den von den Messbereichen ausgehenden Signalen zu bestimmen.

Außerdem löst die vorliegende Erfindung das noch allgemeinere Problem der Bestimmung der absoluten Mengen eines oder mehrerer Analyten in immobilisierten, komplex zusammengesetzten Proben biologischen Ursprungs und der Kalibration der infolge von Bindung von Bindungsreagentien an die nachzuweisenden Analyten entstehenden Signale. Die Erstellung von Kalibrationskurven für aus der Bindung in zugeführten Proben enthaltener Analyten an ihre in "Capture-Arrays" immobiliserten Erkennungselemente entstehenden Signale, mittels Zugabe einer geeigneten Anzahl von Kalibrationslösungen, welche die entsprechenden Analyten in geeigneten Konzentrationen enthalten, ist wohlbekannt. Nachteilig erfordert diese Methode die Zugabe einer Vielzahl von Lösungen auf eine entsprechende Vielzahl zueinander gleichartiger Arrays von Messbereichen. In den internationalen Anmeldungen WO 01/092870 und WO 02/40998 wird vorgeschlagen, dass in einem oder mehreren Arrays jeweils mehrere Messbereiche mit dort in einer unter-schiedlichen, kontrollierten Dichte immobilisierten biologischen oder biochemischen oder synthetischen Erkennungselementen zum Nachweis eines für diese Messbereiche gemein-samen Analyten vorgesehen sind. Dabei wird besonders bevorzugt, dass bei bekannter Kon-zentrationsabhängigkeit der Bindungssignale zwischen einem Analyten und seinen biolo-gischen oder biochemischen oder synthetischen Erkennungselementen und einer ausreichend grossen "Variation" dieser in unterschiedlicher kontrollierter Dichte in verschiedenen Messbereichen eines Arrays immobilisierten Erkennungselemente bereits mittels Zugabe einer einzigen Kalibrationslösung zu diesem Array eine Kalibrationskurve für diesen Analyten erstellt werden kann." Diese verschiedenen Kalibrationsmethoden für "Capture-Arrays" dienen aber immer der Kalibration der Signale infolge von Bindung der in unbekannter Konzentration in Lösung befindlichen Analyten an die immobilisierten Erkennungselemente des Arrays. Für Arrays mit in diskreten Messbereichen immobiolisierten, komplex zusammengesetzten Proben biologischen Ursprungs stellt sich demgegenüber die Aufgabe einer Kalibration der in unbekannter Konzentration in der Immobilisierungsmatrix enthaltenen Analyten. Diese Aufgabe wird mit einem erfindungsgemäßen Array von Messbereichen und einem darauf beruhenden erfindungsgemäßen quantitativen Nachweisverfahren gelöst. Durch Kombination mit dem vorangehend genannten erfindungsgemäßen Verfahren zur Unterscheidung von durch spezifische und unspezifische Bindung erzeugten Signalanteilen wird dadurch eine verlässliche Bestimmung absoluter Analytmengen und - konzentrationen ermöglicht.

Die Erfindung erlaubt es überraschenderweise, auch für die hier verwendete Art von Arrays die relative und/oder absolute Menge oder relative und/oder absolute Konzentration von in den immobilisierten, komplex zusammengesetzten Proben enthaltenen Analyten mit hoher Genauigkeit zu bestimmen.

### Kurzbeschreibung der Abbindung

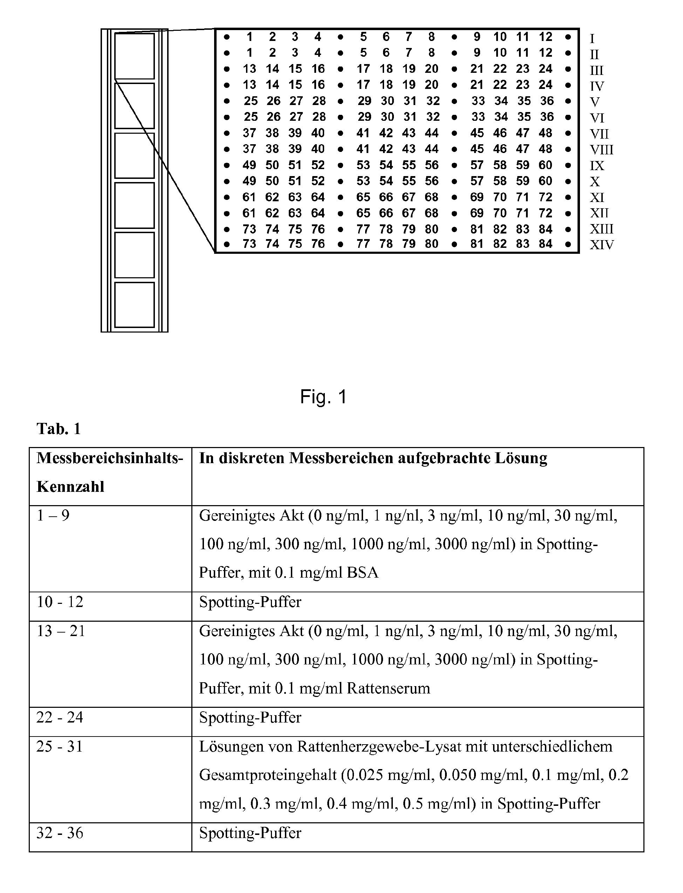

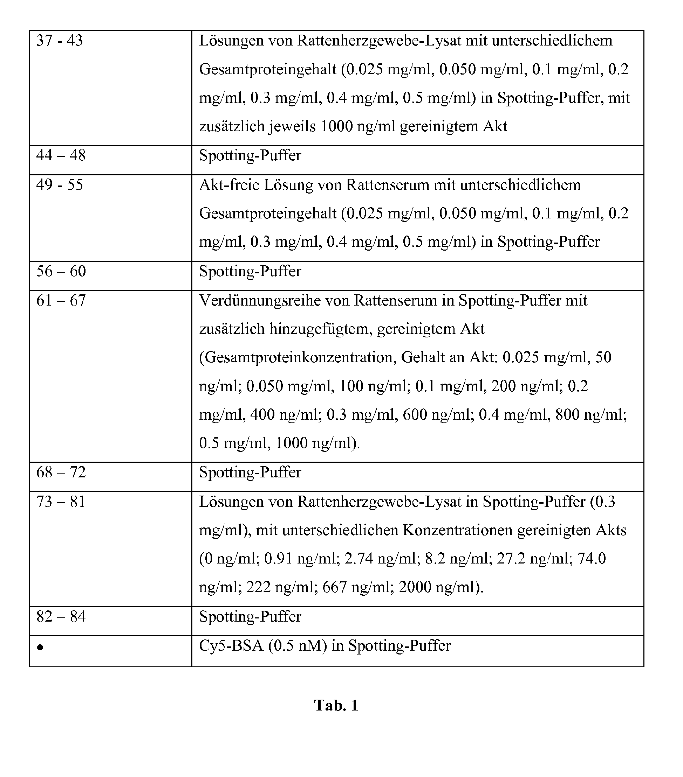

Fig. 1: Träger mit 6 Arrays von Messbereichen für das erfindungsgemäße Verfahren. Gitterstrukturen zur Lichteinkopplung und Lichtauskopplung sind links und rechts von den Arrays angedeutet. Die Ausschnittsvergrößerung zeigt die geometrische Anordnung der Messbereiche in einem einzelnen Array. - Zur Bedeutung der Messbereichsinhalts-Kennzahlen siehe Tab. 1.

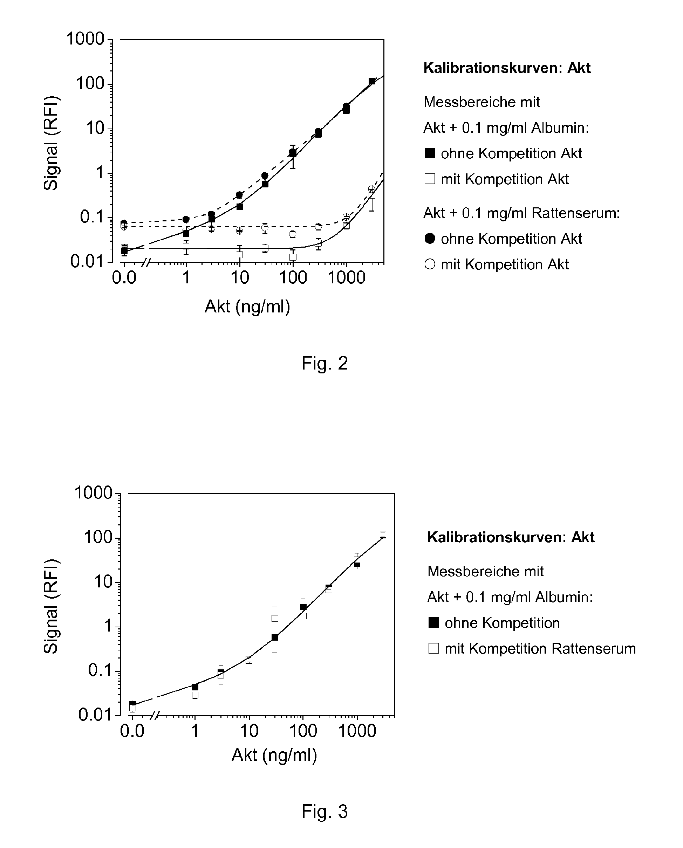

Fig. 2: referenzierte Fluoreszenzintensitäten (RFI) aus Messbereichen mit darin aufgebrachtem, gereinigtem Akt unter zusätzlicher Anwesenheit von 0.1 mg/ml BSA bzw. 0.1 mg/ml Rattenserum in den Immobilisierungslösungen, als Funktion der Akt-Konzentration der Immobilisierungslösungen. Ausgefüllte Symbole: Kalibrationskurven erzeugt ohne Kompetitor in der Lösung des Bindungsreagens (Antikörper "anti-Akt", 5 nM), leere Symbole: Messkurve erzeugt mit 100 nM Akt als Kompetitor in der Lösung des Bindungsreagenz.
Fig. 3: referenzierte Fluoreszenzintensitäten (RFI) aus Messbereichen mit darin aufgebrachtem, gereinigtem Akt unter zusätzlicher Anwesenheit von 0.1 mg/ml BSA bzw. 0.1 mg/ml Rattenserum in den Immobilisierungslösungen, als Funktion der Akt-Konzentration der Immobilisierungslösungen. Ausgefüllte Symbole: Kalibrationskurven erzeugt ohne Kompetitor in der Lösung des Bindungsreagens (Antikörper "anti-Akt", 5 nM), leere Symbole: Messkurve erzeugt mit 0.1 mg/ml Rattenserum als Kompetitor um unspezifische Bindung in der Lösung des Bindungsreagenz.

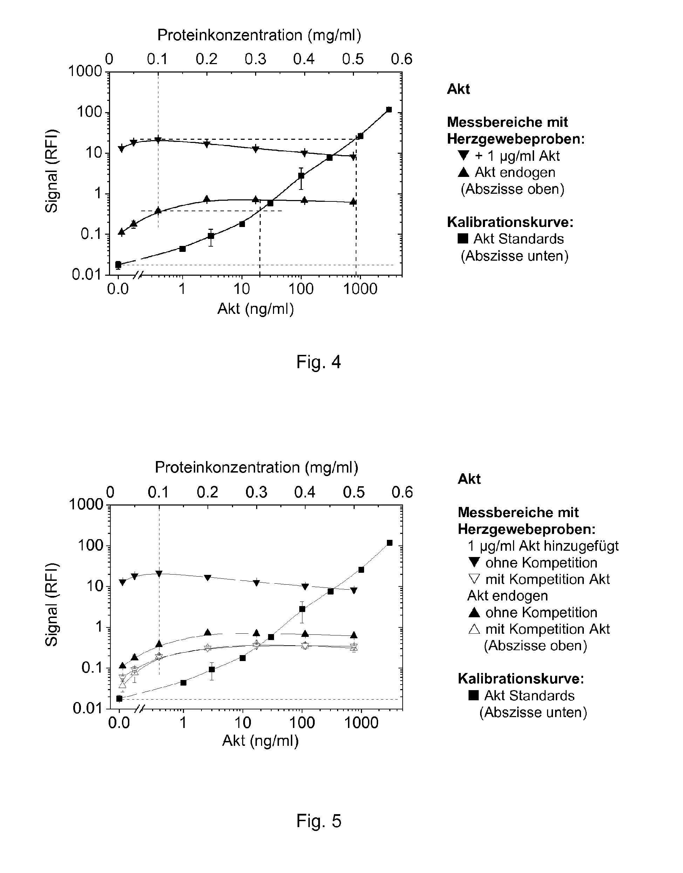

Fig. 4: referenzierte Fluoreszenzintensitäten für den Nachweis von endogenem Akt in aus Rattenherzgewebe hergestellten Proben, mit (obere Kurve) und ohne (mittlere Kurve) zusätzlich hinzugegebenes gereinigtes Akt (1000 ng/ml), als Funktion der Gesamtproteinkonzentration der Immobilisierungslösungen (obere Abszisse). Zusätzlich eingetragen ist die Kalibrationskurve aus Fig. 2 für den Nachweis von Akt, erstellt mit Messbereichen zusätzlich enthaltend 0.1 mg/ml BSA (als Funktion der Akt-Konzentration, untere Abszisse).
Fig. 5: referenzierte Fluoreszenzintensitäten für den Nachweis von endogenem Akt in aus Rattenherzgewebe hergestellten Proben, mit (obere Kurve) und ohne (mittlere Kurve) zusätzlich hinzugegebenes gereinigtes Akt (1000 ng/ml), als Funktion der Gesamtproteinkonzentration der Immobilisierungslösungen (obere Abszisse). Ausgefüllte Symbole: Messwerte erhalten aus Messung ohne Kompetitor in der Lösung des Bindungsreagenz (Antikörper "anti-Akt", 5 nM); leere Symbole: Messwerte erhalten aus Messung mit 100 nM gereinigtem Akt als Kompetitor in der Lösung des Bindungsreagenz (Antikörper "anti-Akt", 5 nM ). Zusätzlich eingetragen sind die Kalibrationskurve aus Fig. 2 für den Nachweis von Akt, erstellt mit Messbereichen zusätzlich enthaltend 0.1 mg/ml BSA (ausgefüllte Symbole; als Funktion der Akt-Konzentration, untere Abszisse) sowie eine entsprechende Kalibrationskurve, erstellt mit 100 nM Akt als Kompetitor in der Lösung des Bindungsreagenz (leere Symbole; als Funktion der Akt-Konzentration, untere Abszisse).

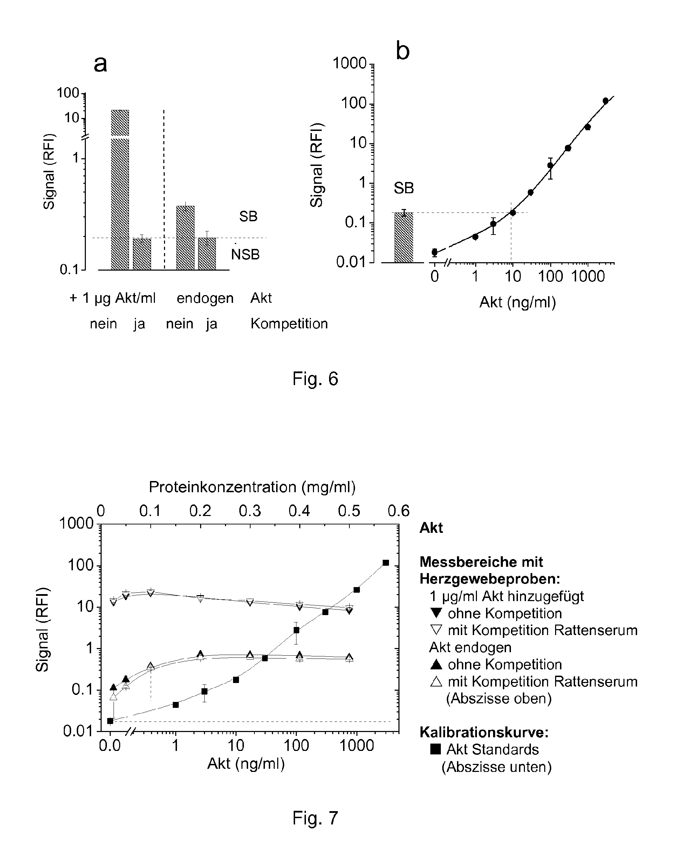

Fig. 6: Bestimmung der durch spezifische Bindung verursachten Anteile an den Signalen aus Fig. 5 bei einer Proteinkonzentration von 0.1 mg/ml aus dem Vergleich der Messwerte in Anwesenheit und Abwesenheit des Kompetitors in Lösung (Fig. 6a) und die Bestimmung des Gehalts an endogenem Akt aus dem Vergleich des durch spezifische Bindung ("SB") verursachten Signalanteils mit der Kalibrationskurve (Fig. 62b).
Fig. 7: referenzierte Fluoreszenzintensitäten für den Nachweis von endogenem Akt in aus Rattenherzgewebe hergestellten Proben, mit (obere Kurven) und ohne (mittlere Kurven) zusätzlich hinzugegebenes gereinigtes Akt (1000 ng/ml), als Funktion der Gesamtproteinkonzentration der Immobilisierungslösungen (obere Abszisse). Ausgefüllte Symbole: Messwerte erhalten aus Messung ohne Kompetitor in der Lösung des Bindungsreagenz (Antikörper "anti-Akt", 5 nM); leere Symbole: Messwerte erhalten aus Messung mit 0.1 mg/ml Rattenserum als Kompetitor um unspezifische Bindung in der Lösung des Bindungsreagenz (Antikörper "anti-Akt", 5 nM). Zusätzlich eingetragen sind die Kalibrationskurve aus Fig. 2 für den Nachweis von Akt, erstellt mit Messbereichen zusätzlich enthaltend 0.1 mg/ml BSA (ausgefüllte Symbole; als Funktion der Akt-Konzentration, untere Abszisse) sowie eine entsprechende Kalibrationskurve, erstellt mit 0.1 mg/ml Rattenserum als Kompetitor um unspezifische Bindung in der Lösung des Bindungsreagenz (leere Symbole; als Funktion der Akt-Konzentration, untere Abszisse).

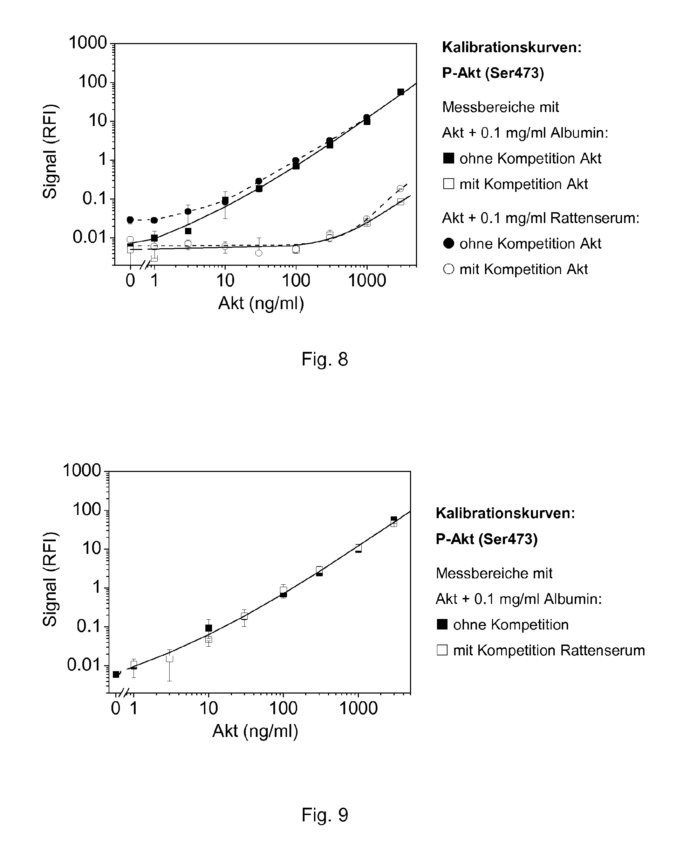

Fig. 8: referenzierte Fluoreszenzintensitäten (RFI) aus Messbereichen mit darin aufgebrachtem, gereinigtem Akt unter zusätzlicher Anwesenheit von 0.1 mg/ml BSA bzw. 0.1 mg/ml Rattenserum in den Immobilisierungslösungen, als Funktion der Konzentration der Immobilisierungslösungen an P-Akt (Ser473). Ausgefüllte Symbole: Kalibrationskurven erzeugt ohne Kompetitor in der Lösung des Bindungsreagens (Antikörper "anti- P-Akt (Ser473)", 5 nM), leere Symbole: Messkurve erzeugt mit 100 nM Akt als Kompetitor in der Lösung des Bindungsreagenz.
Fig. 9: referenzierte Fluoreszenzintensitäten (RFI) aus Messbereichen mit darin aufgebrachtem, gereinigtem Akt unter zusätzlicher Anwesenheit von 0.1 mg/ml BSA bzw. 0.1 mg/ml Rattenserum in den Immobilisierungslösungen, als Funktion der Konzentration der Immobilisierungslösungen an P-Akt (Ser473). Ausgefüllte Symbole: Kalibrationskurven erzeugt ohne Kompetitor in der Lösung des Bindungsreagens (Antikörper "anti- P-Akt (Ser473)", 5 nM), leere Symbole: Messkurve erzeugt mit 0.1 mg/ml Rattenserum als Kompetitor um unspezifische Bindung in der Lösung des Bindungsreagenz.

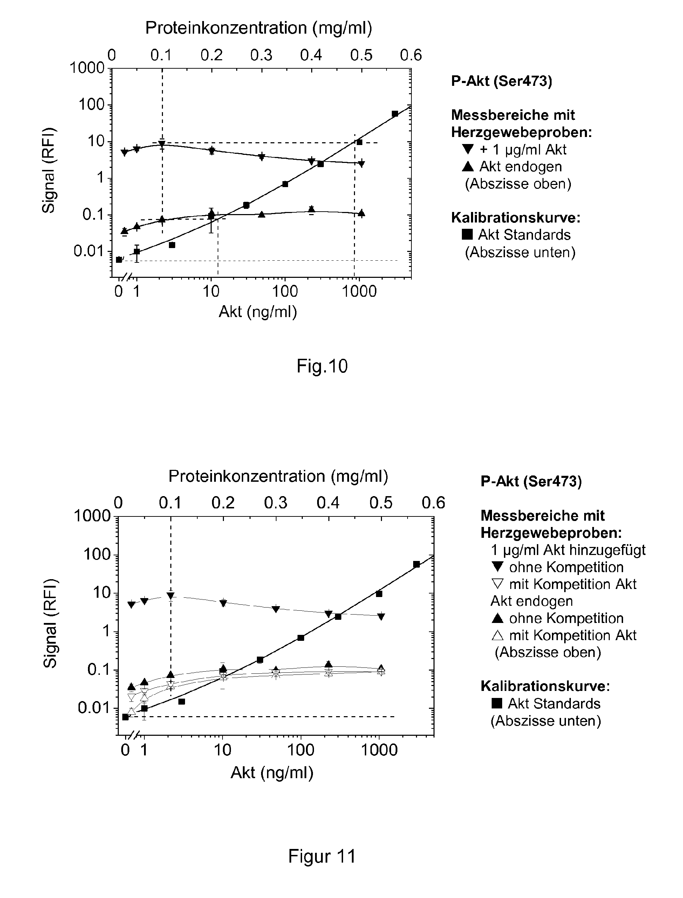

Fig. 10: referenzierte Fluoreszenzintensitäten für den Nachweis von endogenem P-Akt (Ser473) in aus Rattenherzgewebe hergestellten Proben, mit (obere Kurve) und ohne (mittlere Kurve) zusätzlich hinzugegebenes gereinigtes Akt (1000 ng/ml), als Funktion der Gesamtproteinkonzentration der Immobilisierungslösungen (obere Abszisse). Zusätzlich eingetragen ist die Kalibrationskurve aus Fig. 7 für den Nachweis von P-Akt (Ser473), erstellt mit Messbereichen zusätzlich enthaltend 0.1 mg/ml BSA (als Funktion der Konzentration an P-Akt (Ser473), untere Abszisse).
Fig. 11: referenzierte Fluoreszenzintensitäten für den Nachweis von endogenem P-Akt (Ser473) in aus Rattenherzgewebe hergestellten Proben, mit (obere Kurve) und ohne (mittlere Kurve) zusätzlich hinzugegebenes gereinigtes Akt (1000 ng/ml), als Funktion der Gesamtproteinkonzentration der Immobilisierungslösungen (obere Abszisse). Ausgefüllte Symbole: Messwerte erhalten aus Messung ohne Kompetitor in der Lösung des Bindungsreagenz (Antikörper "anti- P-Akt (Ser473)", 5 nM); leere Symbole: Messwerte erhalten aus Messung mit 100 nM gereinigtem Akt als Kompetitor in der Lösung des Bindungsreagenz (Antikörper "anti- P-Akt (Ser473)", 5 nM). Zusätzlich eingetragen sind die Kalibrationskurve aus Fig. 7 für den Nachweis von P-Akt (Ser473), erstellt mit Messbereichen zusätzlich enthaltend 0.1 mg/ml BSA (ausgefüllte Symbole; als Funktion der Konzentration an P-Akt (Ser473), untere Abszisse) sowie eine entsprechende Kalibrationskurve, erstellt mit 100 nM Akt als Kompetitor in der Lösung des Bindungsreagenz (leere Symbole; als Funktion der Akt-Konzentration, untere Abszisse).

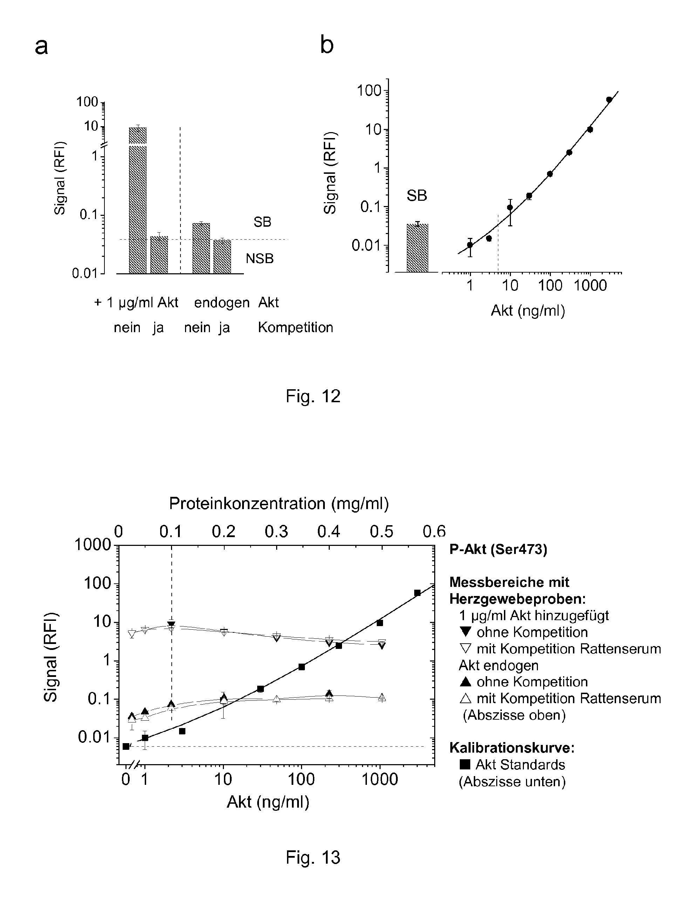

Fig. 12: Bestimmung der durch spezifische Bindung verursachten Anteile an den Signalen aus Fig. 11 bei einer Proteinkonzentration von 0.1 mg/ml aus dem Vergleich der Messwerte in Anwesenheit und Abwesenheit des Kompetitors in Lösung (Fig. 12a) und die Bestimmung des Gehalts an endogenem Akt aus dem Vergleich des durch spezifische Bindung ("SB") verursachten Signalanteils mit der Kalibrationskurve (Fig. 12b).
Fig. 13: referenzierte Fluoreszenzintensitäten für den Nachweis von endogenem P-Akt (Ser473) in aus Rattenherzgewebe hergestellten Proben, mit (obere Kurven) und ohne (mittlere Kurven) zusätzlich hinzugegebenes gereinigtes Akt (1000 ng/ml), als Funktion der Gesamtproteinkonzentration der Immobilisierungslösungen (obere Abszisse). Ausgefüllte Symbole: Messwerte erhalten aus Messung ohne Kompetitor in der Lösung des Bindungsreagenz (Antikörper "anti- P-Akt (Ser473)", 5 nM); leere Symbole: Messwerte erhalten aus Messung mit 0.1 mg/ml Rattenserum als Kompetitor um unspezifische Bindung in der Lösung des Bindungsreagenz (Antikörper "anti- P-Akt (Ser473)", 5 nM). Zusätzlich eingetragen sind die Kalibrationskurve aus Fig. 8 für den Nachweis von P-Akt (Ser473), erstellt mit Messbereichen zusätzlich enthaltend 0.1 mg/ml BSA (ausgefüllte Symbole; als Funktion der Konzentration an P-Akt (Ser473), untere Abszisse) sowie eine entsprechende Kalibrationskurve, erstellt mit 0.1 mg/ml Rattenserum als Kompetitor um unspezifische Bindung in der Lösung des Bindungsreagenz (leere Symbole; als Funktion der Konzentration an P-Akt (Ser473), untere Abszisse).

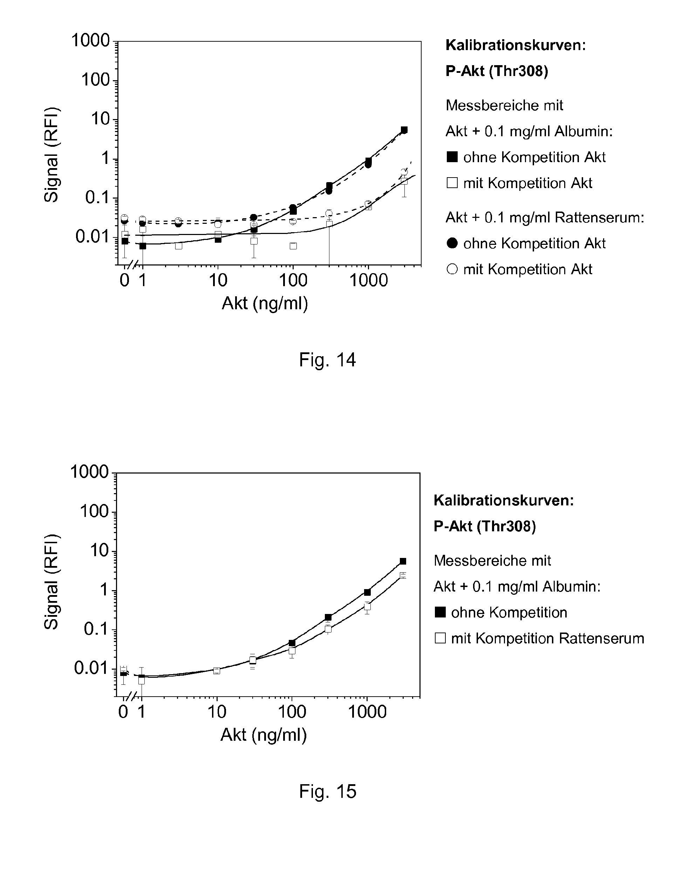

Fig. 14: referenzierte Fluoreszenzintensitäten (RFI) aus Messbereichen mit darin aufgebrachtem, gereinigtem Akt unter zusätzlicher Anwesenheit von 0.1 mg/ml BSA bzw. 0.1 mg/ml Rattenserum in den Immobilisierungslösungen, als Funktion der angenommenen Konzentration der Immobilisierungslösungen an P-Akt (Thr308). Ausgefüllte Symbole: Kalibrationskurven erzeugt ohne Kompetitor in der Lösung des Bindungsreagens (Antikörper "anti- P-Akt (Thr308)", 5 nM), leere Symbole: Messkurve erzeugt mit 100 nM Akt als Kompetitor in der Lösung des Bindungsreagenz.
Fig. 15: referenzierte Fluoreszenzintensitäten (RFI) aus Messbereichen mit darin aufgebrachtem, gereinigtem Akt unter zusätzlicher Anwesenheit von 0.1 mg/ml BSA bzw. 0.1 mg/ml Rattenserum in den Immobilisierungslösungen, als Funktion der angenommenen Konzentration der Immobilisierungslösungen an P-Akt (Thr308). Ausgefüllte Symbole: Kalibrationskurven erzeugt ohne Kompetitor in der Lösung des Bindungsreagens (Antikörper "anti- P-Akt (Thr308)", 5 nM), leere Symbole: Messkurve erzeugt mit 0.1 mg/ml Rattenserum als Kompetitor um unspezifische Bindung in der Lösung des Bindungsreagenz.

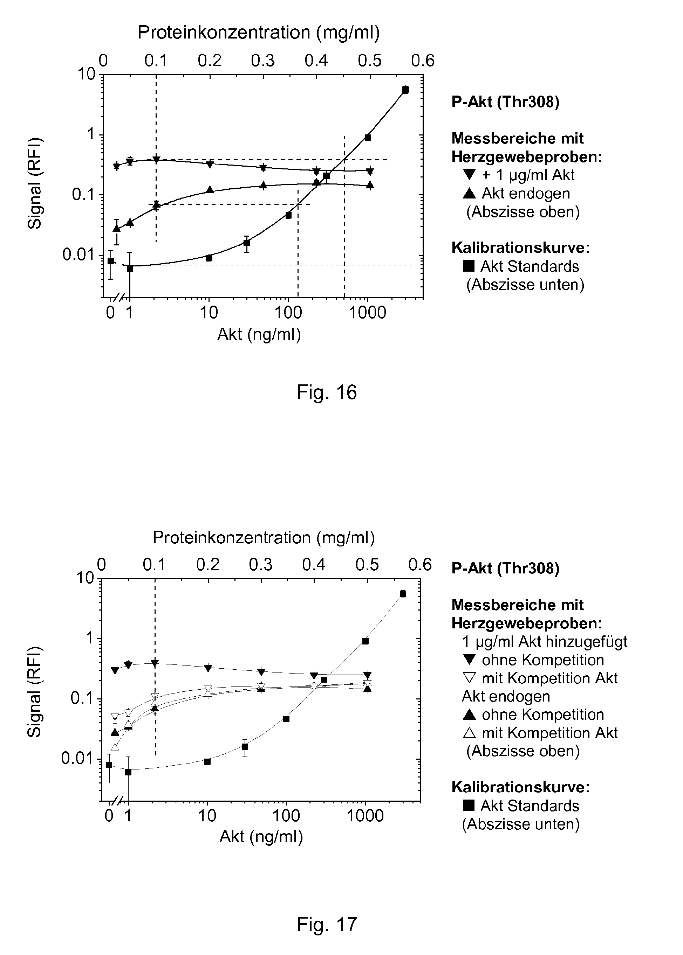

Fig. 16: referenzierte Fluoreszenzintensitäten für den Nachweis von endogenem P-Akt (Thr308) in aus Rattenherzgewebe hergestellten Proben, mit (obere Kurve) und ohne (mittlere Kurve) zusätzlich hinzugegebenes gereinigtes Akt (1000 ng/ml), als Funktion der Gesamtproteinkonzentration der Immobilisierungslösungen (obere Abszisse). Zusätzlich eingetragen ist die Kalibrationskurve aus Fig. 12 für den Nachweis von P-Akt (Thr308), erstellt mit Messbereichen zusätzlich enthaltend 0.1 mg/ml BSA (als Funktion der angenommenen Konzentration an P-Akt (Thr308), untere Abszisse).
Fig. 17: referenzierte Fluoreszenzintensitäten für den Nachweis von endogenem P-Akt (Thr308) in aus Rattenherzgewebe hergestellten Proben, mit (obere Kurve) und ohne (mittlere Kurve) zusätzlich hinzugegebenes gereinigtes Akt (1000 ng/ml), als Funktion der Gesamtproteinkonzentration der Immobilisierungslösungen (obere Abszisse). Ausgefüllte Symbole: Messwerte erhalten aus Messung ohne Kompetitor in der Lösung des Bindungsreagenz (Antikörper "anti- P-Akt (Thr308)", 5 nM); leere Symbole: Messwerte erhalten aus Messung mit 100 nM gereinigtem Akt als Kompetitor in der Lösung des Bindungsreagenz (Antikörper "anti- P-Akt (Thr308)", 5 nM). Zusätzlich eingetragen sind die Kalibrationskurve aus Fig. 14 für den Nachweis von P-Akt (Thr308), erstellt mit Messbereichen zusätzlich enthaltend 0.1 mg/ml BSA (ausgefüllte Symbole; als Funktion der angenommenen Konzentration an P-Akt (Thr308), untere Abszisse) sowie eine entsprechende Kalibrationskurve, erstellt mit 100 nM Akt als Kompetitor in der Lösung des Bindungsreagenz (leere Symbole; als Funktion der angenommenen Konzentration an P-Akt (Thr308), untere Abszisse).

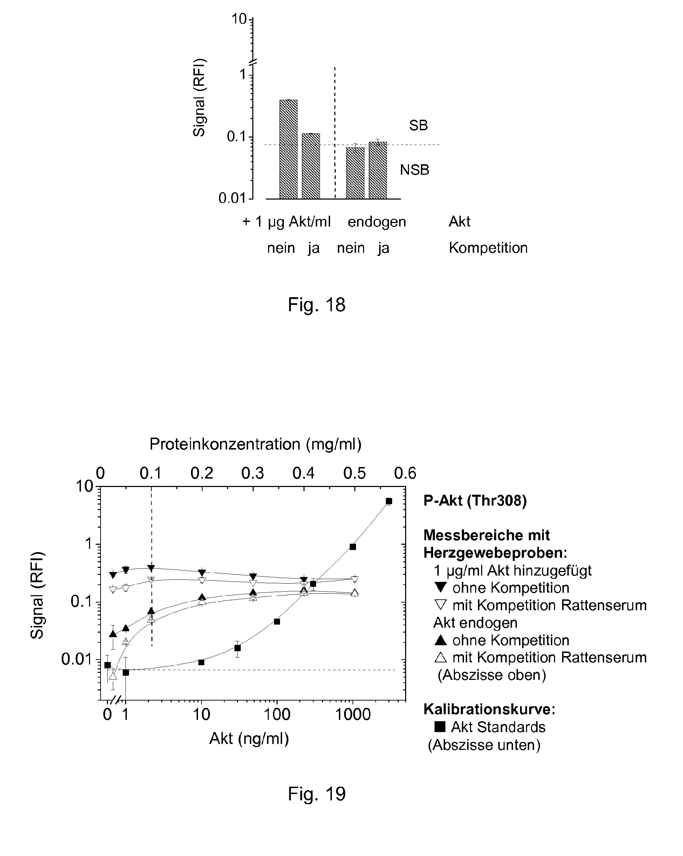

Fig. 18: Bestimmung der durch spezifische Bindung verursachten Anteile an den Signalen aus Fig. 17 bei einer Proteinkonzentration von 0.1 mg/ml aus dem Vergleich der Messwerte in Anwesenheit und Abwesenheit des Kompetitors in Lösung.
Fig. 19: referenzierte Fluoreszenzintensitäten für den Nachweis von endogenem P-Akt (Thr308) in aus Rattenherzgewebe hergestellten Proben, mit (obere Kurven) und ohne (mittlere Kurven) zusätzlich hinzugegebenes gereinigtes Akt (1000 ng/ml), als Funktion der Gesamtproteinkonzentration der Immobilisierungslösungen (obere Abszisse). Ausgefüllte Symbole: Messwerte erhalten aus Messung ohne Kompetitor in der Lösung des Bindungsreagenz (Antikörper "anti-P-Akt (Thr308)", 5 nM); leere Symbole: Messwerte erhalten aus Messung mit 0.1 mg/ml Rattenserum als Kompetitor um unspezifische Bindung in der Lösung des Bindungsreagenz (Antikörper "anti- P-Akt (Thr308)t", 5 nM). Zusätzlich eingetragen sind die Kalibrationskurve aus Fig. 12 für den Nachweis von P-Akt (Thr308), erstellt mit Messbereichen zusätzlich enthaltend 0.1 mg/ml BSA (ausgefüllte Symbole; als Funktion der angenommenen Konzentration an P-Akt (Thr308), untere Abszisse) sowie eine entsprechende Kalibrationskurve, erstellt mit 0.1 mg/ml Rattenserum als Kompetitor um unspezifische Bindung in der Lösung des Bindungsreagenz (leere Symbole; als Funktion der angenommenen Konzentration an P-Akt (Thr308), untere Abszisse).

### Genaue Beschreibung der Erfindung

Im Sinne der vorliegenden Erfindung sollen räumlich getrennte oder diskrete Messbereiche auf einem festen Träger durch die geschlossene Fläche definiert werden, die dort aufgebrachte komplex zusammengesetzte Proben biologischen Ursprungs oder aufgebrachte Referenzierungsreagentien (wie beispielsweise fluoreszenzmarkiertes Albumin) oder Kalibrationsreagentien oder deren aufgebrachte Mischungen einnehmen. Diese Flächen können dabei eine beliebige Geometrie, beispielsweise die Form von Kreisen, Rechtecken, Dreiecken, Ellipsen etc., haben.

Erster Gegenstand der vorliegenden Erfindung ist ein Verfahren zum Nachweis eines oder mehrerer Analyten in einer oder mehreren komplex zusammengesetzten Proben biologischen Ursprungs, umfassend die folgenden Schritte:
(1) Bereitstellung einer oder mehrerer komplex zusammengesetzter Proben biologischen Ursprungs,
(2) Bereitstellung mindestens eines festen Trägers,
(3) Aufbringung kleiner Mengen der komplex zusammengesetzten Proben biologischen Ursprungs, in verdünnter oder unverdünnter Form, an diskreten Orten direkt auf dem festen Träger oder nach vorheriger Aufbringung einer Haftvermittlungsschicht auf besagter Haftvermittlungsschicht auf dem festen Träger, wodurch ein oder mehr Arrays von diskreten Messbereichen auf dem mindestens einen festen Träger erzeugt werden,
(4) Kontaktierung mindestens eines Arrays von diskreten Messbereichen mit einer ersten Lösung enthaltend ein oder mehrere Bindungsreagentien als spezifische Bindungspartner für die in diskreten Messbereichen in den aufgebrachten komplex zusammengesetzten Proben biologischen Ursprungs enthaltenen, nachzuweisenden Analyten und, gegebenenfalls falls erforderlich, ein oder mehr Detektionsreagentien, wobei die Aufbringung von Bindungs- und Detektionsreagentien gleichzeitig oder sequentiell erfolgen kann,
(5) ortsaufgelöste Messung von ersten optischen Signalen, welche von diskreten Messbereichen eines oder mehrerer Arrays, welche in Schritt (4) mit der ersten Lösung in Kontakt gebracht wurden, ausgehen,
(6) Aufzeichnung dieser ersten optischen Signale,
   **dadurch gekennzeichnet, dass** der Anteil von durch unspezifische Wechselwirkung mit den zugegebenen Bindungsreagentien und mit den gegebenenfalls zugegebenen Detektionsreagentien erzeugten optischen Signalen an den gemessenen ersten optischen Signalen durch Ausführung der weiteren Schritte bestimmt wird:
   (7a) Aufbringung einer zweiten Lösung, enthaltend, zusätzlich zu den ein oder mehr Bindungsreagentien und gegebenenfalls ein oder mehr Detektionsreagentien der in Schritt (4) hinzugegebenen ersten Lösung, eine bekannte hohe Konzentration von Verbindungen, welche gleichartig zu den in diskreten Messbereichen aufgebrachten komplex zusammengesetzten Proben biologischen Ursprungs enthaltenen, nachzuweisenden Analyten sind, als Kompetitoren zu den in diskreten Messbereichen aufgebrachten komplex zusammengesetzten Proben biologischen Ursprungs enthaltenen, nachzuweisenden Analyten um die spezifische Bindung besagter Bindungsreagentien und gegebenenfalls zusätzlich hinzugegebener Detektionsreagentien, auf ein oder mehr der in Schritt (3) erzeugten Arrays von diskreten Messbereichen und/oder
   (7b) Aufbringung einer dritten Lösung, enthaltend, zusätzlich zu den ein oder mehr Bindungsreagentien und gegebenenfalls ein oder mehr Detektionsreagentien der in Schritt (4) hinzugegebenen ersten Lösung, eine bekannte hohe Konzentration von Stoffen, welche ähnlicher Art wie in der Probenmatrix der in Schritt (3) aufgebrachten Proben enthaltene Stoffe sind, zwecks Kompetition zu den in diskreten Messbereichen aufgebrachten komplex zusammengesetzten Proben biologischen Ursprungs enthaltenen Stoffen der Probenmatrix um unspezifische Bindung besagter Bindungsreagentien und gegebenenfalls zusätzlich hinzugegebener Detektionsreagentien, auf ein oder mehr der in Schritt (3) erzeugten Arrays von diskreten Messbereichen,
(8) ortsaufgelöste Messung von zweiten und/oder dritten optischen Signalen, welche von diskreten Messbereichen eines oder mehrerer Arrays, welche in Schritt (7a) mit der zweiten Lösung bzw. in Schritt (7b) mit der dritten Lösung in Kontakt gebracht wurden, ausgehen,
(9) Aufzeichnung dieser zweiten und/oder dritten optischen Signale und
(10) Vergleich der ersten und zweiten und/oder dritten optischen Signale.

In den Schritten (7a) und (7b) kann jeweils die Zugabe der Bindungsreagentien und der zur Kompetition hinzugefügten Stoffe sowie der optionalen Detektionsreagentien in Form der Zugabe einer einzigen Lösung dieser drei Gruppen von Komponenten erfolgen. Es wird jedoch bevorzugt, dass im Falle der zusätzlichen Zugabe von Detektionsreagentien zunächst die Aufbringung zweiter bzw. dritter Lösungen von Mischungen aus den Bindungsreagentien und den zur Kompetition eingesetzten Stoffen erfolgt, optional gefolgt von ein oder mehreren Waschschritten und einem nachfolgenden separaten Teilschritt der Zugabe der Detektionsreagentien.

Im folgenden soll die Bezeichnung "des festen Trägers" oder "eines festen Trägers" die Bedeutung von "des mindestens einen festen Trägers" oder "mindestens eines festen Trägers" mit einschließen.

Die Bezeichnungen "erste Lösung", "zweite Lösung", "dritte Lösung" sollen die Bedeutung einer "Vielzahl erster Lösungen", "Vielzahl zweiter Lösungen" bzw. "Vielzahl dritter Lösungen" mit einschließen, wobei die Lösungen innerhalb einer solchen Vielzahl von Lösungen jeweils eine gleiche oder unterschiedliche Zusammensetzung haben können.

Der Begriff "Probe" wird im folgenden auch gleichbedeutend mit "komplex zusammengesetzte Probe biologischen Ursprungs" verwendet, sofern nicht anders vermerkt. Die Bezeichnung einer "Probe" im Singular schließt die Bezeichnung einer "Pluralität von Proben" mit ein, sofern nicht anders vermerkt.

Besagte komplex zusammengesetzte Proben biologischen Ursprungs können ausgewählt sein aus der Gruppe von Proben, welche von Lysaten von Zell-Populationen, Zell-Extrakten, Körperflüssigkeiten und Bestandteilen von Körperflüssigkeiten, wie beispielsweise Blut, Serum, Plasma, Gelenkflüssigkeit, Tränenflüssigkeit, Urin, Speichelflüssigkeit, Gewebeflüssigkeit und Lymphe, gebildet wird, wobei es sich um fraktionierte oder nicht fraktionierte Proben handeln kann.

Die Proben können gewonnen sein von gesunden und/oder krankhaften und/oder stimulierten und/oder unbehandelten Zellen aus der Gruppe, welche menschliche, tierische, bakterielle und pflanzliche Zellen umfasst. Insbesondere können besagte komplex zusammengesetzte Proben biologischen Ursprungs gewonnen sein von tierischem oder menschlichem Gewebe, wie beispielsweise Organ-, Haut-, Haar-, Muskel-, Fett- oder Knochengewebe.

Unterschiedliche Proben können dem gleichen Organismus oder der gleichen Zellkultur entnommen worden sein. Dann kann durch die Analyse auf mehreren Messbereichen mit darin enthaltenem Material aus dem gleichen (oder einem gleichartigen) Organismus bzw. der gleichen Zellkultur (bzw. gleichartigen Zellkulturen) beispielsweise statistische Information über die Reproduzierbarkeit der in diesen Messbereichen bestimmten relativen molekularen Zusammensetzung der aufgebrachten Proben gewonnen werden.

Unterschiedliche Proben können insbesondere an verschiedenen Positionen des gleichen Organismus entnommen werden. Dann kann aus den Analysen auf den entsprechenden diskreten Messbereichen beispielsweise Information über Inhomogenitäten der relativen molekularen Zusammensetzung der nachzuweisenden Analyten in dem Organismus, aus dem besagte Proben entnommen wurden, gewonnen werden. Ein solches Vorgehen ist beispielsweise für die Untersuchung krebsbehafteter Organismen von großer Bedeutung.

Unterschiedliche Proben können aber auch verschiedenen Organismen oder verschiedenen Zellkulturen entnommen sein. Beispielsweise kann es sich dabei um Proben von mit einem pharmazeutischen Wirkstoff behandelten und unbehandelten Organismen handeln. Ähnlich einer Expressionsanalyse in der Nukleinsäure-Analytik kann dann der Einfluss des jeweiligen Wirkstoffes auf die relative molekulare Zusammensetzung der Proben, d.h. insbesondere die Zusammensetzung der Vielzahl der von den zugrundeliegenden Zellpopulationen exprimierten Verbindungen, untersucht werden.

Im Falle von Zellen als Ausgangsmaterial für die Proben werden die Zellen typischerweise in einem ersten Aufbereitungsschritt lysiert. Die Lysate können in einem geeigneten Lösungsmittel, beispielsweise einer Pufferlösung, gelöst sein und bekannte zugesetzte Beimischungen enthalten, beispielsweise Stabilisatoren wie Protease-Inhibitoren, um einen Abbau enthaltener Biopolymere zu verhindern. Gemäß einer bevorzugten Ausführungsform des erfindungsgemäßen Verfahrens werden die Lysate so hergestellt und weiterbehandelt, dass eine daraus gewonnene Probe (d.h. komplex zusammengesetzte Probe biologischen Ursprungs) das gesamte Proteom von Zell-Linien, ZellKulturen oder Zell-Geweben enthält, aus dem sie gewonnen wurde.

Ein wichtiges Anwendungsgebiet des erfindungsgemäßen Verfahrens ist die Untersuchung von Proben, welche Zell-Lysate umfassen oder aus Zell-Lysaten hergestellt wurden, auf die zelluläre Analyt-Expression (d.h. insbesondere Protein-Expression) unter unterschiedlichen Bedingungen. In Abhängigkeit vom Ziel der jeweiligen Studie, können die in den Messbereichen aufzubringenden Proben in unterschiedlicher Weise ausgewählt sein.

Eine mögliche Variante beruht auf der Verwendung von Lysaten aus voneinander unabhängigen Zell-Populationen (d.h. Zell-Linien oder Zellkultur-Ansätzen). Dabei sollen solche Zell-Populationen als "voneinander unabhängig" bezeichnet werden, welche unabhängig voneinander gewachsen sind oder unabhängig voneinander kultiviert wurden (wie z. B. "In-vitro-Zellkulturen"). Demzufolge sollen unter dieser Bezeichnung beispielsweise Zell-Populationen eingeschlossen sein, welche von verschiedenen Menschen, Tieren, Pflanzen oder Organismen im allgemeinen sowie von verschiedenen Organen stammen, des weiteren Zell-Populationen, welche von verschiedenen Orten innerhalb eines Organismus oder Organs stammen, wie beispielsweise krebshaftes und gesundes Gewebe aus ein- und demselben Organ. Die Bezeichnung soll auch Zell-Populationen umfassen, welche von demselben Organismus oder Organ zu verschiedenen Zeitpunkten entnommen wurden und/oder nach Entnahme in einem In-vitro-Kultivationsprozess unterschiedlichen Behandlungen, Stimulationen oder anderen unterschiedlichen Beeinflussungen anderer Art ausgesetzt waren. Mithilfe des erfindungsgemäßen Verfahrens kann dann mithilfe von aus derartigen voneinander unabhängigen Zell-Populationen gewonnenen Proben beispielsweise ein differentielles Expressionsprofil erzeugt werden, um Unterschiede in der zellulären Expression und/oder Aktivierung zellulärer Signaltransduktionskaskaden, beispielsweise zwischen verschiedenen Organismen, zwischen gesunden und kranken gleichartigen Organismen, zwischen verschiedenen Organen etc. nachzuweisen. Von besonderem Interesse ist dabei der Einfluss der Behandlung oder Stimulation der Zell-Populationen auf die zelluläre Expression. Unter den Begriffen der "Behandlung" oder "Stimulation" soll dabei die Zugabe chemischer oder biochemischer Verbindungen (Reagentien oder "Drugs") zu den betreffenden Zellpopulationen als auch die Einwirkung unterschiedlicher äußerer physikalischer Bedingungen, beispielsweise in Form von Bestrahlung mit Licht aus dem ultravioletten bis infraroten Spektrum, Hitzeeinwirkung, Einwirkung elektromagnetischer Felder etc., verstanden werden.

Das erfindungsgemäße Verfahren eignet sich auch zur Untersuchung der Auswirkungen weniger genau definierbarer innerer oder äußerer Einflussfaktoren, wie Stress, Krankheit, Alterung, Art der Ernährung etc. auf die zelluläre Expression und/oder Aktivierung (siehe unten).

Aufgrund der hohen Genauigkeit der durch das erfindungsgemäße Verfahren erzielbaren Messergebnisse ist es dabei insbesondere auch geeignet für die Untersuchung der zeitlichen Entwicklung zellulärer Expression und/oder Aktivierung (siehe unten) unter den vorgenannten Bedingungen, über Zeiträume von beispielsweise Minuten, Stunden, Tagen, Wochen, Monaten oder Jahren.

Eine andere mögliche Variante beruht auf der Verwendung unterschiedlicher Zell-Lysate, welche von verschiedenene Zell-Subpopulationen erzeugt wurden, die ihrerseits von einer gemeinsamen Zell-Population gewonnen wurden. Verschiedene Zell-Subpopulationen können beispielsweise durch Entnahme von einer gemeinsamen Zell-Population zu verschiedenen Zeitpunkten erzeugt werden. Verschiedene Zell-Subpopulationen können auch durch Entnahme von einer gemeinsamen Zell-Population und nachfolgende Behandlung oder Stimulationen durch unterschiedliche Reagentien und/oder Kultivierung unter unterschiedlichen äußeren Bedingungen (z. B. zur Untersuchung des Einflusses von Bestrahlung durch UV-Licht, von Hitzeschock etc.) erzeugt werden.

Typische für die Behandlung von Zell-Populationen für die genannten und andere mögliche Varianten für die Anwendung des erfindungsgemäßen Verfahren eingesetzte Reagentien umfassen pharmazeutisch wirksame Verbindungen, Cytokine, Antigene zur Zell-Stimulation, Zelltodinduzierende Stimulatoren, Hormone etc.

Vorzugsweise werden komplex zusammengesetzte Proben, welche von Zell-Populationen gewonnen wurden, deren Expression mithilfe des erfindungsgemäßen Verfahrens verglichen werden soll, jeweils in einem gemeinsamen Array von Messbereichen aufgebracht, um die Proben dann unter möglichst einheitlichen Bedingungen auf die nachzuweisenden enthaltenen Analyten untersuchen zu können.

Eine Probe kann Zusätze von bekannten Konzentrationen gleichartiger Verbindungen (als Standards) wie der nachzuweisenden Analyten, vergleichbar mit einem "Spiken" von Proben in der Chromatographie, enthalten. Solche Zusätze können beispielsweise für Kalibrationszwecke dienlich sein. Außerdem können die Proben Zusätze von der Probenmatrix ähnlichen, aber von den nachzuweisenden Analyten verschiedenen Verbindungen, wie beispielsweise Albumine (z. B. Rinderserumalbumin (BSA)), Immunoglobuline oder verdünntes Serum, enthalten, welche beispielsweise der kontrollierten Einstellung der Oberflächendichte immobilisierter Analytmoleküle in einem Messbereich dienen können. In den Proben oder deren Fraktionen oder den Verdünnungen besagter Proben oder Fraktionen enthaltene Analyten, d.h. insbesondere Biopolymere wie beispielsweise Nukleinsäuren oder Proteine, können in nativer Form oder denaturierter Form, nach Behandlung beispielsweise mit Harnstoff oder Tensiden (z. B. SDS), vorliegen. Falls notwendig, können insbesondere unlösliche Bestandteile des Ursprungsmaterials oder Zwischenmaterials im Falle mehrerer Vorbereitungsschritte vor Bereitstellung der komplex zussammengesetzten Proben biologischen Ursprungs durch geeignete Maßnahmen, beispielsweise Zentrifugation im Falle von Lysaten als Zwischenmaterial, entfernt werden. Vorzugsweise werden die Ausgangsmaterialien zur Herstellung einer "komplex zusammengesetzten Probe biologischen Ursprungs" keinen weiteren Vorbehandlungsschritten als Filtration und/oder Fraktionierung und/oder Verdünnung unterzogen.

Bevorzugt liegen die in den Proben oder deren Fraktionen oder in den Verdünnungen besagter Proben oder Fraktionen enthaltenen Analyten, d.h. insbesondere Biopolymere wie beispielsweise Proteine, z. B. nach Behandlung mit Harnstoff, in denaturierter Form vor, wobei die Epitope dieser Analyten für die Bindung ihrer jeweiligen Nachweissubstanzen, beispielsweise von Antikörpern, möglichst frei zugänglich sind. Dieses wird beispielsweise ermöglicht durch die Zerstörung der tertiären oder quarternären Struktur infolge der Behandlung mit Harnstoff. Denaturierte Proben zeichnen sich außerdem durch den Vorteil aus, dass aus ihnen erzeugte Arrays sehr stabil sind und über längere Zeiträume (bis zu Jahren) gelagert und archiviert werden können für später durchzuführende Analysen.

Im Falle von fraktionierten Proben können diese mit einem Trennverfahren beispielsweise aus der Gruppe von Trennverfahren gewonnen sein, welche Fällungen, Filtration, Zentrifugation, HPLC und micro-HPLC ("High Pressure Liquid Chromatography") mittels der Methode von "Normal Phase"-, "Reverse Phase"-, Ionenaustausch- und Hydrophobe Interaktionschromatographie (HIC), "Size Exclusion Chromatography", Gelchromatographie, Elektrophorese, Kapillarelektrophorese, Elektrochromatographie, sowie "Free-Flow Electrophoresis" umfasst.

Insbesondere können besagte komplex zusammengesetzte Proben biologischen Ursprungs abgereichertes Serum umfassen. Als "abgereichertes Serum" werden solche aus Serum gewonnene Proben bezeichnet, denen, beispielsweise mittels Affinitätschromatographie Inhaltsstoffe wie Albumine, Immunoglobuline und Apolipoproteine in hohem Maße entzogen wurden.

Das Material für eine zu analysierende komplex zusammengesetzte Probe biologischen Ursprungs kann beispielsweise mittels einer Methode aus der Gruppe von Entnahmemethoden gewonnen worden sein, welche Gewebeschnitte, Biopsie und "Laser Capture Micro Dissection" umfasst.

Das erfindungsgemäße Verfahren ermöglicht es, auch nur geringe eingesetzte Probenvolumina und - mengen mit hoher Genauigkeit zu analysieren. Als Probenmenge soll dabei die Gesamtmenge verstanden werden, welche in einem diskreten Messbereich aufgebracht ist. Beispielsweise kann das Material einer in einem Messbereich aufzubringenden Probe dem Material von weniger als 100 Zellen entsprechen. Es kann sogar dem Material von weniger als 10 Zellen entsprechen. Außerdem ist es möglich, dass das in einem Messbereich aufgebrachte Material einer zu analysierenden komplexen zusammengesetzten Probe biologischen Ursprungs ein Volumen von weniger als 100 nl, bevorzugt von weniger als 1 nl, darstellt.

Die Analyten, welche in den in diskreten Messbereichen aufgebrachten komplex zusammengesetzten Proben biologischen Ursprungs nachgewiesen werden sollen, können Verbindungen aus der Gruppe sein, welche Proteine und deren posttranslational modifizierte Proteinformen, wie beispielsweise phosphorylierte, glycolisierte, methylierte und acetylierte Formen von Proteinen, insbesondere an zellulären Signaltransduktionswegen beteiligte und wechselwirkende Proteine, wie z. B. Kinasen, Kinasesubstrate, Rezeptoren und Bindungsproteine für Peptide, Hormone, Cofaktoren, Membranrezeptoren, Kanalrezeptoren, T-Zell-Rezeptoren und Enzyme, sowie Proteine und deren posttranslational modifizierte Proteinformen, die aus unterschiedlichen Zellkompartimenten entstammen, wie beispielsweise cytosolische Proteine, Zellkernproteine, Membranproteine, mitochondriale Proteine, und extrazelluläre Proteine, wie beispielweise in Körperflüssigkeiten ausgeschiedene Proteine, und insbesondere auch unter dem Einfluss von zellulärer Behandlung oder Stimulation über- oder unterexprimierte Proteine sowie künstlich modifizierte oder exprimierte Proteine, wie beispielsweise funktionalisierte Proteine mit zusätzlichen Bindungsstellen ("Tag-Proteine", wie beispielsweise "Histidin-Tag-Proteine"), mono- oder polyklonale Antikörper und Antikörperfragmente, Peptide, erzeugte Peptidfragmente von ganzen Proteinen, Glycopeptide, Lektine, fluoreszente Proteine (wie beispielsweise "Green Fluorescent Protein", GFP und dergleichen), Avidin, Streptavidin, Biotin, biotinylierte Proteine und anders konjugierte Proteine, Oligosaccharide sowie Nukleinsäuren (beispielsweise DNA, RNA) umfasst.

Als "Analyt" soll im Rahmen der vorliegenden Erfindung eine solche molekulare Spezies bezeichnet werden, welche mithilfe eines hierfür eingesetzten Bindungsreagenz als spezifischem Bindungspartner und gegebenenfalls eines zusätzlich eingesetzten Detektionsreagenz von anderen in einer zu analysierenden Probe enthaltenen Verbindungen unterschieden und gebunden wird. Erfolgt beispielsweise die Bindung eines entsprechenden Bindungsreagenz nur an die phosphorylierte, aber nicht an die unphosphorylierte Form einer nachzuweisenden Verbindung oder Spezies, so stellen gemäß dieser Definition beide Formen dieser Verbindung bzw. Spezies zwei unterschiedliche Analyten dar. Werden von einem entsprechenden Bindungsreagenz jegliche Verbindungen oder Spezies erkannt und gebunden, wenn sie phosphoryliert sind, so stellen entsprechend unter dieser Bedingung die entsprechenden phosphorylierten Verbindungen oder Spezies gemeinsam einen Analyten dar. Bindungsreagentien als spezifische Bindungspartner eines Analyten gemäß dieser Definition können beispielsweise so ausgewählt sein, dass sie ausschließlich die phosphorylierte Form oder die glykolisierte Form (oder entsprechend die nicht phosphorylierte bzw. nicht glykolisierte Form) einer nachzuweisenden Verbindung erkennen und daran binden. Die Aktivität eines biologischen Signalwegs in einer Zelle oder einem Organismus kann mit dem Anteil phosphorylierter, methylierter, acetylierter oder glykolisierter Verbindungen (abhängig von der Natur des Signalwegs), welche diesen Signalweg steuern, korreliert werden. Der relative Anteil der phosphorylierten, methylierten, acetylierten bzw. glykolisierten Form an der Gesamtmenge, d.h. der Quotient der Menge einer Verbindung in ihrer phosphorylierten, methylierten, acetylierten bzw. glykolisierten Form und der Gesamtmenge dieser Verbindung in phosphorylierter und nicht phosphorylierter Form bzw. methylierter und nicht methylierter Form bzw. acetylierter und nicht acetylierter Form bzw. glykolisierter und nicht glykolisierter Form, in einer Probe wird nachfolgend als Phosphorylierungssgrad bzw. Methylierungsgrad bzw. Acetylierungsgrad bzw. Glykolisierungsgrad dieser Verbindung in der Probe bezeichnet. Phosphorylierungsgrad, Methylierungsgrad, Acetylierungsgrad und Glykolisierungsgrad können unter dem Oberbegriff des Aktivierungsgrads einer Verbindung zusammengefasst werden. Der Aktivierungsgrad einer Verbindung kann aber auch andere chemisch veränderte Formen einer Verbindung bezeichnen. Das erfindungsgemäße Verfahren eignet sich besonders gut auch zur Bestimmung des Aktivierungsgrades exprimierter Proteine.

Bindungsreagentien als spezifische Bindungspartner können auch so ausgewählt sein, dass sie nur dann an eine nachzuweisende Verbindung binden, wenn diese in einer bestimmten dreidimensionalen Struktur vorliegt. Beispielsweise erkennen und binden viele Antikörper nur an spezielle Teilbereiche (Epitope) einer nachzuweisenden Substanz mit einer speziellen dreidimensionalen Struktur. Je nach Konformationszustand der entsprechenden nachzuweisenden Verbindung können diese Teilbereiche (Epitope) für die Bindung des entsprechenden Bindungsreagenz zugänglich oder verborgen sein. Die Bindungsreagentien können jedoch auch so ausgewählt sein, dass sie an Bereiche der nachzuweisenden Verbindung binden, deren Zugänglichkeit unabhängig von der dreidimensionalen Struktur dieser Verbindung ist. Durch den Einsatz entsprechend ausgewählter Bindungsreagentien ist es daher möglich, den relativen Anteil an der Gesamtmenge einer in einer Probe nachzuweisenden Verbindung, welche einen spezifischen Konformationszustand aufweist, zu bestimmen.

Das erfindungsgemäße Verfahren ermöglicht bei geeigneter Auswahl der eingesetzten Bindungsreagentien und gegebenenfalls zusätzlicher Detektionsreagentien, dass Proteine, welche als Analyten in den in diskreten Messbereichen aufgebrachten komplex zusammengesetzten Proben biologischen Ursprungs nachgewiesen werden sollen, gemäß ihres Vorkommens in phosphorylierter und/oder glycolisierter und/oder methylierter und/oder acetylierter Form in den aufgebrachten komplex zusammengesetzten Proben biologischen Urspungs, im Zuge des Schritts (4) gemäß Anspruch 1 nach der Bindung damit in Kontakt gebrachter Bindungsreagentien als spezifischen Bindungspartnern und gegebenenfalls zusätzlicher Detektionsreagentien, unterschieden und im Detektionsschritt (5) gemäß Anspruch 1 separat als unterschiedliche Analyten im Sinne der vorangehenden Definition detektiert werden.

Es ist auch möglich, dass Proteine, welche als Analyten in den in diskreten Messbereichen aufgebrachten komplex zusammengesetzten Proben biologischen Urspungs nachgewiesen werden sollen, gemäß ihres Vorkommens in phosphorylierter und/oder glycolisierter, und/oder methylierter und/oder acetylierter Form in den aufgebrachten komplex zusammengesetzten Proben biologischen Urspungs, im Zuge des Schritts (4) gemäß Anspruch 1 nach der Bindung damit in Kontakt gebrachter Bindungsreagentien als spezifischen Bindungspartnern und gegebenenfalls zusätzlicher Detektionsreagentien, nicht unterschieden und im Detektionsschritt (5) gemäß Anspruch 1 nicht separat als unterschiedliche Analyten, sondern gemeinsam als ein einzelner Analyt detektiert werden.

Das erfindungsgemäße Verfahren ermöglicht es, den Aktivierungsgrad (gemäß obiger Definition) eines oder mehrerer in einer aufgebrachten komplex zusammengesetzten Probe biologischen Ursprungs enthaltenen Analyten (d.h. insbesondere von enthaltenen Proteinen) zu bestimmen. Insbesondere kann mit besagtem Verfahren der Phosphorylierunsgrad und/oder Methylierungsgrad und/oder Acetylierungsgrad und/oder Glykolisierungsgrad eines oder mehrerer in einer aufgebrachten Probe enthaltenen Analyten (insbesondere Proteinen) bestimmt werden.

Die eingesetzten Bindungsreagentien als spezifische Bindungspartner für die in diskreten Messbereichen in den aufgebrachten komplex zusammengesetzten Proben biologischen Ursprungs enthaltenen, nachzuweisenden Analyten können beispielsweise ausgewählt sein aus der Gruppe von Verbindungen, welche Proteine, beispielsweise mono- oder polyklonale Antikörper und Antikörperfragmente, Peptide, Enzyme, Enzyminhibitoren, Kinasesubstrate, Aptamere, synthetische Peptidstrukturen, Glycopeptide, Hormone, Cofaktoren, Oligosaccharide, Lektine, Antigene für Antikörper oder T-Zell-Rezeptoren, Biotin, Avidin, Streptavidin, mit zusätzlichen Bindungsstellen funktionalisierte Proteine ("Tag-Proteine", wie beispielsweise "Histidin-Tag-Proteinen") und deren Komplexbildungspartner sowie Nukleinsäuren (beispielsweise DNA, RNA, Oligonukleotide) und Nukleinsäureanaloge (z. B. PNA) sowie deren Derivate mit künstlichen Basen umfasst.

Eingesetzte Detektionsreagentien können ausgewählt sein aus einer ersten Gruppe, welche polyklonale oder monoklonale Antikörper und Antkörperfragmente, Nukleinsäuren und Nukleinsäurederivate sowie deren Derivate mit künstlichen Basen, Biotin, Avidin, Streptavidin und Neutravidin umfasst. Die Detektionsreagentien können auch ausgewählt sein aus einer zweiten Gruppe, welche Massenlabel, beispielsweise in der Form von Nanopartikeln, Beads oder Kolloiden, und Lumineszenzlabel, beispielsweise in der Form von Lumineszenzfarbstoffen oder lumineszenten Nanopartikeln wie "Quantum Dots" mit Anregungs- und Emissionswellenlängen zwischen 200 nm und 1000 nm, umfasst, wobei besagte Massen- oder Lumineszenzlabel an die Bindungsreagentien gebunden sind oder sich daran anlagern oder binden oder an Detektionsreagentien der vorgenannten ersten Gruppe von Detektionsreagentien gebunden sind oder in spezifischer Weise an besagte Detektionsreagentien der ersten Gruppe von Detektionsreagentien binden oder anlagern oder an die Komplexe zwischen den nachzuweisenden Analyten, welche in den in diskreten Messbereichen aufgebrachten komplex zusammengesetzten Proben biologischen Ursprungs enthalten sind, und daran gebundenen Bindungsreagentien als spezifischen Bindungspartnern, gebildet werden, binden oder anlagern. Die Bindungsreagentien können die Funktion der Detektionsreagentien mit umfassen.

Mit dem Begriff "Lumineszenz" wird in dieser Anmeldung die spontane Emission von Photonen im ultravioletten bis infraroten Bereich nach optischer oder nichtoptischer, wie beispielsweise elektrischer oder chemischer oder biochemischer oder thermischer Anregung, bezeichnet. Beispielsweise sind Chemilumineszenz, Biolumineszenz, Elektrolumineszenz und insbesondere Fluoreszenz und Phosphoreszenz unter dem Begriff "Lumineszenz" mit eingeschlossen.

Eine bevorzugte Variante des erfindungsgemäßen Verfahrens besteht darin, dass besagte Bindungsreagentien als spezifische Bindungspartner und optionale Detektionsreagentien einer ersten Lösung und/oder

Bindungsreagentien als spezifische Bindungspartner, Verbindungen, welche gleichartig zu den in diskreten Messbereichen aufgebrachten komplex zusammengesetzten Proben biologischen Ursprungs enthaltenen, nachzuweisenden Analyten und optionale Detektionsreagentien einer zweiten Lösung und/oder

Bindungsreagentien als spezifische Bindungspartner, Stoffe, welche ähnlicher Art wie in der Probenmatrix der in Schritt (3) in diskreten Messbereichen aufgebrachten komplex zusammengesetzten Proben biologischen Ursprungs enthaltene Stoffe sind, und optionale Detektionsreagentien einer dritten Lösung
enthalten, jeweils miteinander vorinkubiert werden und besagte erste, zweite oder dritte Lösung dann in einem einzigen Zugabeschritt mit besagten Arrays von Messbereichen in Kontakt gebracht wird.

Eine mögliche Variante des erfindungsgemäßen Verfahrens ist **dadurch gekennzeichnet, dass** unterschiedliche Analyten in einem gemeinsamen Array von Messbereichen nachgewiesen werden durch Zugabe von unterscheidbaren Detektionsreagentien zu besagtem Array. Dabei wird bevorzugt, dass die Anzahl unterschiedlicher nachzuweisender Analyten gleich der Anzahl eingesetzter unterscheidbarer Detektionsreagentien ist. Es wird besonders bevorzugt, wenn unterscheidbare Detektionsreagentien sich in der Anregungs- und/oder Emissionswellenlänge einer Lumineszenz unterscheiden.

Charakteristisch für eine andere Variante des erfindungsgemäßen Verfahrens ist, dass eine Vielzahl unterschiedlicher Analyten in einer Vielzahl von Arrays von diskreten Messbereichen durch Zugabe von unterschiedlichen Bindungsreagentien als spezifischen Bindungspartnern zur Bestimmung unterschiedlicher Analyten auf verschiedene Arrays von diskreten Messbereichen und/oder durch Zugabe von unterscheidbaren Detektionsreagentien zu besagten Arrays von Messbereichen nachgewiesen werden.

Kennzeichen einer weiteren möglichen Variante ist, dass unterschiedliche Bindungsreagentien als spezifische Bindungspartner für unterschiedliche Analyten auf verschiedene Arrays aufgebracht werden für jeden unterschiedlichen nachzuweisenden Analyten.

Es ist vorteilhaft, wenn Arrays von Messbereichen mit darin aufgebrachten komplex zusammengesetzten Proben biologischen Ursprungs solche Messbereiche umfassen, in denen dem aufgebrachten Material als Standards bekannte Konzentrationen von Verbindungen hinzugefügt wurden, welche gleicher Art wie die nachzuweisenden Analyten sind. Dabei wird besonders bevorzugt, dass Arrays von Messbereichen eine Anzahl von solchen Messbereichen umfassen, in denen dem aufgebrachten Material als Standards unterschiedliche bekannte Konzentrationen von Verbindungen hinzugefügt wurden, welche gleicher Art wie die nachzuweisenden Analyten sind, wobei die Anzahl solcher Messbereiche und Höhe besagter unterschiedlicher bekannter Konzentrationen ausreichend ist, um mittels eines einzigen Zugabeschritts einer ersten Lösung mit darin enthaltenen Bindungsreagentien als spezifischen Bindungspartnern und gegebenenfalls ebenfalls enthaltenen Detektionsreagentien, gemäß Schritt (4) nach Anspruch 1 des erfindungsgemäßen Verfahrens, und der nachfolgenden Schritte (5) und (6) nach Anspruch 1 eine Kalibrationskurve für die Bestimmung unbekannter Konzentrationen besagter nachzuweisender Analyten in dem Array zu erzeugen. Dabei kann wiederum die Zugabe der Bindungsreagentien und der optionalen Detektionsreagentien gemäß Schritt (4) des erfindungsgemäßen Verfahrens in einem einzigen Zugabeschritt oder in separaten Teilschritten, gegebenenfalls mit dazwischen erfolgenden Waschschritten, erfolgen. Das Material, welchem dabei die Standards zur Analytbestimmung hinzugefügt wurden, kann beispielsweise nur die Komponenten der benutzten Pufferlösung, zugesetzte der Probenmatrix ähnliche Verbindungen wie Albumine (insbesondere Rinderserumalbumin), Immunoglobuline oder verdünntes Serum umfassen.

Es wird bevorzugt, dass auf einem festen Träger mehrere gleichartige Arrays von Messbereichen angeordnet sind, wobei gleiche Positionen von Messbereichen in verschiedenen Arrays, bezüglich Anordnung in Reihen und Spalten, bedeuten, dass dort gleichartige Proben aufgebracht worden sind.

Für die Applikation der verschiedenen Lösungen, nämlich der ersten Lösung mit darin enthaltenenen Bindungsreagentien und gegebenenfalls zusätzlich enthaltenen Detektionsreagentien, der zweiten Lösung mit darin zusätzlich enthaltenen, den nachzuweisenden Analyten gleichartigen Verbindungen als Kompetitoren sowie einer dritten Lösung mit hinzugefügten Verbindungen, welche in der Probenmatrix der in den Messbereichen aufgebrachten Proben enthalten sind, auf Arrays von Messbereichen, welche auf einem gemeinsamen festen Träger angeordnet sind, gibt es verschiedene mögliche Ausführungsformen.

Eine erste, bevorzugte mögliche Ausführungsform zeichnet sich dadurch aus, dass die Zugabe einer ersten Lösung gemäß Schritt (4) und Messung und Aufzeichnung erster erster optischer Signale aus den Messbereichen dieses Arrays gemäß den Schritten (5) und (6) nach Anspruch 1 und Zugabe zweiter und/oder dritter Lösungen, gemäß den Schritten (7a) und (7b) und nachfolgende Messung und Aufzeichnungen der von den Messbereichen der betreffenden Arrays ausgehenden Signale, gemäß den Schritten (8) und (9) nach Anspruch 1, auf verschiedenen gleichartigen Arrays von Messbereichen erfolgt, wobei gleiche Positionen von Messbereichen in verschiedenen Arrays, bezüglich Anordnung in Reihen und Spalten, bedeuten, dass dort gleichartige Proben aufgebracht worden sind.

Es ist aber auch möglich, dass erste, zweite und dritte Lösungen sequentiell, jeweils nach Ausführung einer ausreichenden Anzahl von Regenerations- und Waschschritten, auf dem gleichen Array von Messbereichen (oder der gleichen Pluralität von Messbereichen) aufgebracht werden. Unter "Regeneration" soll dabei ein solcher Zwischenschritt verstanden werden, bei dem durch Zugabe geeigneter Komplex-zerstörender Reagentien, wie beispielsweise sogenannten "chaotropen" Reagentien mit einem hohen Salzgehalt / hoher Ionenstärke und/oder stark sauren Charakters zur Dissoziation von Antigen-Antikörper-Komplexen oder Harnstoff-Lösungen zur Dissoziation von hybridisierten Nukleinsäure-Strängen, die zwischen den Analyten und ihren Bindungsreagentien und gegebenenfalls zusätzlich hinzugefügten Detektionsreagentien nach Zugabe der ersten Lösung bzw. zweiten oder dritten Lösung gebildeten Komplexe dissoziiert werden, so dass die immobilisierten Analytmoleküle vor Zugabe einer zweiten bzw. dritten Lösung jeweils wieder für die Bindung von Bindungsreagentien und gegebenenfalls weiteren Detektionsreagentien zugänglich sind. Vorzugsweise wird diese Variante des erfindungsgemäßen Verfahrens aber nur dann durchgeführt, wenn eine Regenerierbarkeit in hohem Maße (beispielsweise von mehr als 80 %, bevorzugt von mehr als 90 %) gewährleistet ist.

Die bevorzugt einzusetzenden Konzentrationen von Verbindungen, welche gleichartig zu den in diskreten Messbereichen aufgebrachten komplex zusammengesetzten Proben biologischen Ursprungs enthaltenen, nachzuweisenden Analyten sind und als Kompetitoren zu den in diskreten Messbereichen aufgebrachten komplex zusammengesetzten Proben biologischen Ursprungs enthaltenen, nachzuweisenden Analyten um die spezifische Bindung besagter Bindungsreagentien und gegebenenfalls zusätzlich hinzugegebener Detektionsreagentien eingesetzt sind, in der aufzubringenden zweiten Lösung sind abhängig von den zu erwartenden Oberflächenkonzentrationen besagter Analyten in den Messbereichen und den Gleichgewichtskonstanten der Bindungsreaktionen zwischen besagten Analyten und ihren Bindungs- bzw. Detektionsreagentien. Typischerweise werden die Bindungsreagentien in hundertfacher bis tausendfacher Verdünnung der vom Anbieter bezogenen Stammlösungen. Für Antikörper sind solche Stammlösungen mit einem Gehalt von typischerweise 0.5-1 mg/ml erhältlich, entsprechend Konzentrationen im Bereich von 1-10 µM. Die Detektionsreagentien werden in einem vergleichbaren Konzentrationsbereich eingesetzt, also typischerweise 1-10 nM, ebenso wie die Bindungsreagentien. In Falle von Kompetitionsexperimente werden die Kompetitoren (z.B. phosphorylierte Peptideepitope) in mindestens einem zehnfachen, besser einem hundertfachen Überschuss, im Vergleich zu den Konzentrationen der Bindungsreagentien, eingesetzt. Die als Kompetitoren um unspezifische Verbindung eingesetzten Stoffen ähnlicher Art wie in der Probenmatrix der komplex zusammengesetzten Proben biologischen Ursprungs enthaltenene Stoffe (z. B. Albumine, Immunoglobuline oder verdünntes Serum) werden typischerweise mit einem Gesamtproteingehalt von 10 µg/ml bis 500 µg/ml dieser dritten Lösungen (entsprechend Schritt (7b) des erfindungsgemäßen Verfahrens) eingesetzt.

Das erfindungsgemäße Verfahren ermöglicht es, die durch unspezifische Wechselwirkung oder unspezifische Bindung verursachten Signalanteile an in den Messbereichen gemessenen, aufgrund von spezifische Bindung und zusätzlicher unspezifischer Bindung erzeugten Gesamtsignalen, auf zweierlei Art zu bestimmen.

Die erste Möglichkeit besteht darin, dass der Anteil von durch unspezifische Wechselwirkung mit den zugegebenen Bindungsreagentien und gegebenenfalls mit den zugegebenen Detektionsreagentien erzeugten optischen Signalen an den gemessenen ersten optischen Signalen gemäß Anspruch 1 aus der Differenz der gemäß Schritt (8), nach Zugabe der zweiten Lösung gemäß Schritt (7a), gemessenen optischen Signale und der gemäß Schritt (5), nach Zugabe der ersten Lösung gemäß Schritt (4), gemessenen optischen Signale bestimmt wird.

Die zweite Möglichkeit ist **dadurch gekennzeichnet, dass** der Anteil von durch unspezifische Wechselwirkung mit den zugegebenen Bindungsreagentien und gegebenenfalls mit den zugegebenen Detektionsreagentien erzeugten optischen Signalen an den gemessenen ersten optischen Signalen gemäß Anspruch 1 aus der Differenz der gemäß Schritt (8), nach Zugabe der dritten Lösung gemäß Schritt (7b), gemessenen optischen Signale und der gemäß Schritt (5), nach Zugabe der ersten Lösung gemäß Schritt (4), gemessenen optischen Signale bestimmt wird.

In Kombination mit der bereits genannten, bevorzugten Ausführungsform des erfindungsgemäßen Verfahrens, gemäß derer Arrays von Messbereichen eine Anzahl von solchen Messbereichen umfassen, in denen dem aufgebrachten Material als Standards unterschiedliche bekannte Konzentrationen von Verbindungen hinzugefügt wurden, welche gleicher Art wie die nachzuweisenden Analyten sind, wobei die Anzahl solcher Messbereiche und Höhe besagter unterschiedlicher bekannter Konzentrationen ausreichend ist, um mittels eines einzigen Zugabeschritts einer ersten Lösung mit darin enthaltenen Bindungsreagentien als spezifischen Bindungspartnern und gegebenenfalls ebenfalls enthaltenen Detektionsreagentien, gemäß Schritt (4) nach Anspruch 1, und der nachfolgenden Schritte (5) und (6) nach Anspruch 1 eine Kalibrationskurve für die Bestimmung unbekannter Konzentrationen besagter nachzuweisender Analyten in dem Array zu erzeugen, ermöglicht das erfindungsgemäße Verfahren, die (relative und/oder absolute) Konzentration oder (relative und/oder absolute) Menge eines Analyten in einer in einem Messbereich aufgebrachten komplex zusammengesetzten Probe biologischen Ursprungs aus der Differenz zwischen dem für diesen Messbereich gemessenen optischen Signal (d.h. dem gemessenen ersten optischen Signal) und dem Anteil von durch unspezifische Wechselwirkung mit den zugegebenen Bindungsreagentien und gegebenenfalls mit den zugegebenen Detektionsreagentien erzeugtem optischem Signal und Vergleich dieser Differenz mit einer Kalibrationskurve für den betreffenden Analyten zu bestimmen.

Die genannten Verfahrensschritte werden in Ausführungsbeispielen noch genauer erläutert.

Charakteristisch für das erfindungsgemäße Vefahren ist außerdem aufgrund seiner hohen Empfindlichkeit und hohen Genauigkeit und Reproduzierbarkait, insbesondere aufgrund einer Vielzahl gleichzeitig oder alternativ einsetzbarer, voneinander unabhängiger Referenzierungs- und Kalibrationsmethoden, dass Unterschiede, bevorzugt von weniger als 20 %, besonders bevorzugt von weniger als 10 %, in der (relativen und/oder absoluten) Konzentration oder (relativen und/oder absoluten) Menge eines Analyten in verschiedenen komplex zusammengesetzten Proben biologischen Ursprungs, welche in verschiedenen Messbereichen aufgebracht sind, bestimmt werden können.

Aufgrund der nur geringen Variation der gewonnenen Messergebnisse ist das erfindungsgemäße Verfahren auch geeignet zur Untersuchung des zeitlichen Verlaufs (d. h. der Veränderungen) der Mengen oder Konzentrationen von (in den komplex zusammengesetzten Proben biologischen Ursprungs nachzuweisenden) Analyten unter Einfluss der Erkrankung eines biologischen Organismus oder einer Zellkultur und/oder der äußeren Beeinflussung eines Organismus oder einer Zellkultur, beispielsweise durch Behandlung oder Stimulation mit einer biologischen wirksamen Verbindung ("drug") oder durch äußere physikalische Einwirkung wie beispielsweise Bestrahlung mit Licht einer Wellenlänge des ultravioletten bis infraroten Spektrums, Einfluss von Radioaktivität oder durch Hitzeeinwirkung.

Eine weitere mögliche Ausführungsform des erfindungsgemäßen Verfahrens ist daher **dadurch gekennzeichnet, dass** eine Probe und eine oder mehrere Vergleichsproben dem gleichen Ursprungsort zu unterschiedlichen Zeitpunkten entnommen sind und dass zeitliche Veränderungen der in diesen Proben enthaltenen Mengen oder Konzentrationen eines oder mehrerer Analyten bestimmt werden. Unter "dem gleichen Ursprungsort" soll dabei der gleiche Organismus oder ein gleichartiger Organismus oder die gleiche Zellkultur bzw. gleichartige Zellkultur (jeweils nach unterschiedlich langer gleichartiger Erkrankung oder Beeinflussung) verstanden werden. Vorzugsweise ermöglicht das erfindungsgemäße Verfahren, zeitliche Änderungen der Konzentration bzw. Menge besagter Analyten von weniger als 20 %, bevorzugt von weniger als 10 %, nachzuweisen.

Die einfachste Form der Immobilisierung der zu untersuchenden komplex zusammengesetzten Proben biologischen Ursprungs auf einem festen Träger besteht in physikalischer Adsorption, beispielsweise infolge hydrophober Wechselwirkungen mit der Oberfläche des festen Trägers. Diese Wechselwirkungen können jedoch durch die Zusammensetzung der im weiteren Verlauf des Verfahrens aufzubringenden Lösungen und deren physikalisch-chemische Eigenschaften, wie beispielsweise Polarität und Ionenstärke, in ihrem Ausmaß stark verändert werden. Insbesondere im Falle sequentieller Zugabe verschiedener Reagentien in einem mehrstufigen Assay kann das Haftvermögen der komplex zusammengesetzten Proben oder ihrer Bestandteile nach rein adsorptiver Immobilisierung auf der Oberfläche unzureichend.sein. Es wird daher bevorzugt, dass auf dem festen Träger, zur Verbesserung des Haftvermögens der in diskreten Messbereichen aufzubringenden Proben, eine Haftvermittlungsschicht aufgebracht ist, auf welcher die Proben dann aufgetragen werden.

Vorzugsweise beträgt dabei die Dicke der Haftvermittlungsschicht weniger als 200 nm, besonders bevorzugt weniger als 20 nm.

Für die Herstellung der Haftvermittlungsschicht eignen sich eine Vielzahl von Materialien. Beispielsweise kann die Haftvermittlungsschicht Verbindungen umfassen aus der Gruppe, welche Silane, funktionalisierte Silane, Epoxide, funktionalisierte, geladene oder polare Polymere und "selbstorganisierte passive oder funktionalisierte Mono- und Mehrfachschichten", Thiole, Alkylphosphate und -phosphonate sowie multifunktionelle Block-Copolymere, wie beispielsweise Poly(L)lysin/Polyethylenglycole, umfasst.

Es ist von Vorteil, wenn Bereiche zwischen den diskreten Messbereichen zur Minimierung unspezifischer Bindung von Bindungs- oder Detektionsreagentien "passiviert werden", d.h. dass zwischen den räumlich getrennten Messbereichen gegenüber besagten Bindungsreagentien und/oder Detektionsreagentien "chemisch neutrale", d.h. diese nicht bindende, Komponenten aufgebracht werden.

Besagte gegenüber den Bindungsreagentien und/oder Detektionsreagentien "chemisch neutrale", d.h. diese nicht bindende, Komponenten können ausgewählt sein aus den Gruppen, die von Albuminen, insbesondere Rinderserumalbumin und Humanserumalbumin, Casein, unspezifischen, polyklonalen und monoklonalen, artfremden und empirisch für den oder die nachzuweisenden Analyten unspezifischen Antikörpern (insbesondere für Immunoassays), Detergentien - wie beispielsweise Tween 20 - nicht mit zu analysierenden Polynukleotiden hybridisierender, fragmentierter natürlicher und synthetischer DNA, wie beispielsweise ein Extrakt von Herings- oder Lachssperma (insbesondere für Polynukleotid-Hybridisierungsassays), und auch ungeladenen, aber hydrophilen Polymeren, wie beispielsweise Polyethylenglycolen oder Dextranen, gebildet werden.

Für die Aufbringung der komplex zusammengesetzten Proben biologischen Ursprungs direkt auf dem festen Träger oder auf einer auf dem Träger zuvor aufgebrachten Haftvermittlungsschicht eignen sich eine Vielzahl bekannter Verfahren, welche beispielsweise ausgewählt sein können aus der Gruppe von Verfahren, die von "Ink jet spotting", mechanischem Spotting mittels Stift, Feder oder Kapillare, "Micro contact printing", fluidischer Kontaktierung der Messbereiche mit besagten Proben durch deren Zufuhr in parallelen oder gekreuzten Mikrokanälen, unter Einwirkung von Druckunterschieden oder elektrischen oder elektromagnetischen Potentialen sowie photochemischen und photolithographischen Immobilisierungsverfahren gebildet wird.

Die Arrays von Messbereichen stellen vorzugsweise ein- oder zweidimensionale Anordnungen diskreter Messbereiche dar. Die erreichbare Dichte von Messbereichen innerhalb eines Arrays von Messbereichen und Anzahl von Messbereichen auf einem gemeinsamen festen Träger ist wesentlich durch die Ortsauflösung der eingesetzten Aufbringungsmethode bestimmt. Typischerweise umfasst ein Array mehr als 50, bevorzugt mehr als 500, besonders bevorzugt mehr als 5000 Messbereiche. Dabei kann jeder Messbereich eine zu anderen Messbereichen gleiche oder unterschiedliche immobilisierte Probe enthalten. Die Messbereiche eines Arrays sind in einer Dichte von mehr als 10, bevorzugt von mehr als 100, besonders bevorzugt von mehr als 1000 Messbereichen pro Quadratzentimeter angeordnet.

Vorteilhaft ist eine solche Ausführungsform des erfindungsgemässen Verfahrens, welche dadurch gekennzeichnet ist, dass auf dem festen Träger eine Vielzahl von Arrays von Messbereichen angeordnet sind. Insbesondere können auf dem Träger mindestens 5, bevorzugt mindestens 50 Arrays von Messbereichen angeordnet sein. Besonders vorteihaft ist es, wenn unterschiedliche Arrays von Messbereichen in unterschiedlichen Probenbehältnissen angeordnet sind. Beispielsweise in den internationalen Patentanmeldungen WO 01/13096 und WO 01/43875 ist beschrieben, wie ein als Evaneszentfeld-Sensorplattform ausgebildeter fester Träger als Bodenplatte mit einem geeigneten Aufsatzkörper zur Erzeugung eines geeigneten Arrays von Probenbehältnissen, jeweils für die Aufnahme eines Arrays von Messbereichen, kombiniert werden kann.

Es wird bevorzugt, dass auf dem gemeinsamen, durchgehenden festen Träger 2 - 2000, vorzugsweise 2 - 400, besonders bevorzugt 2 - 100 Probenbehältnisse angeordnet sind.

Besonders bevorzugt wird, dass die Probenbehältnisse in einem Raster, d.h. einer Aufeinanderfolge in Zeilen und/oder Spalten angeordnet sind, welches kompatibel ist mit dem Raster von StandardMikrotiterplatten. Als industrieller Standard ist dabei eine Anordnung von 8 x 12 Wells mit einem (Zentrum-zu-Zentrum) Abstand von ca. 9 mm etabliert. Hiermit kompatibel sind kleinere Arrays mit beispielsweise 3, 6, 12, 24 und 48 Probenbehältnissen in gleichem Abstand. Es können auch mehrere solche kleineren Arrays von Probenbehältnissen derart zusammengefügt werden, dass nach deren Zusammenfügung der gegenseitige Abstand ein ganzzahliges Vielfaches des Abstands von ca. 9 mm beträgt.

Seit einiger Zeit werden auch Platten mit 384 und 1536 Wells, als ganzzahligem Vielfachen von 96 Wells auf gleicher Grundfläche mit ensprechend reduziertem Wellabstand (ca. 4.5 mm bzw. 2.25 mm), verwendet, welche ebenfalls als Standardmikrotiterplatten bezeichnet werden sollen. Die Anordnung von Probenbehältnissen als Teil des erfindungsgemässen Kits kann auch an diese Geometrie angepasst sein.

Durch die Anpassung des Rasters der Probenbehältnisse an diese Standards können eine Vielzahl kommerziell eingeführter und erhältlicher Laborpipettoren und Laborroboter für die Zugabe der ersten, zweiten oder dritten Lösungen verwendet werden.

Mit solchen Ausführungsformen eines festen Trägers für das erfindungsgemäße Verfahren wird eine experimentelle Konzeption ermöglicht, welche man als "multidimensional" bezeichnen kann: Beispielsweise können in den Zeilen und Spalten eines Arrays verschiedene Proben, beispielsweise von unterschiedlichen Organismen (z. B. entsprechend Spalten), in unterschiedlicher Verdünnung (z. B. entsprechend Zeilen) aufgebracht sein. Unterschiedliche Arrays von Messbereichen mit darin immobilisierten Proben, in unterschiedlichen Probenbehältnissen, können dann mit unterschiedlichen ersten und/oder zweiten oder dritten Lösungen mit darin enthaltenenen Bindungsreagentien und gegebenenfalls Detektionsreagentien zur Bestimmung unterschiedlicher immobilisierter Analyten in unterschiedlichen Arrays in Kontakt gebracht werden. Es ist offensichtlich, dass sich mit einer solchen Variante eines Trägers eine nahezu unbegrenzte Anzahl verschiedener Experimente durchführen lässt.

Für das erfindungsgemäße Verfahren eignen sich eine Vielzahl von Ausführungsformen eines festen Trägers. Es wird bevorzugt, dass der feste Träger, auf welchem die Arrays von diskreten Messbereichen erzeugt werden, im wesentlichen planar ist. Unter "im wesentlichen planar" soll verstanden werden, dass, abgesehen von beispielsweise eventuell auf der den Messbereichen zugewandten Oberfläche erzeugten Strukturen wie beispielsweise Vertiefungen oder Erhöhungen zur Erzeugung von Vorrichtungen für Probenbehältnisse, diese Oberfläche eine makroskopische Welligkeit von weniger als 100 Mikrometern pro Zentimeter Ausdehnung entlang einer beliebigen Achse in der Ebene seiner Oberfläche hat.

Für viele Anwendungen ist es auch vorteilhaft, wenn der feste Träger nicht porös ist. "Nicht porös" soll hier bedeuten, dass besagter Träger keine durchgehenden porösen Strukturen aufweist und seine (mikroskopische) Oberflächenrauhigkeit weniger als 1 µm beträgt. Bevorzugt beträgt die Oberflächenrauhigkeit des festen Trägers weniger als 20 nm, besonders bevorzugt weniger als 2 nm.

Für viele Anwendungen ist es auch erwünscht, dass der optische Träger mindestens bei der Wellenlänge eines während der Nachweisschritte des erfindungsgemäßen Verfahrens in Richtung von Messbereichen geleiteten Anregungslichts oder Messlichts im wesentlichen optisch transparent ist.

Unter einem eingestrahlten "Anregungslicht" soll dabei verstanden werden, dass dieses Licht als Energiequelle für eine Sekundäremission (zusammenfassend bezeichnet als "Emissionslicht"), wie beispielsweise Fluoreszenz oder allgemeiner Lumineszenz oder eine Ramanstrahlung oder beispielsweise zur Anregung eines Oberflächenplasmons in einer Metallschicht, dient, welche mit einem geeigneten Detektor gemessen werden kann. Unter einem eingestrahlten "Messlicht" soll verstanden werden, dass dieses ebenfalls der Wechselwirkung mit dem Träger und/oder mit darauf nachzuweisenden Analyten oder deren Bindungspartnern zwecks eines Analytnachweises dient, dass aber keine spektralen Änderungen dieses Messlichts oder einer Sekundäremission zu untersuchen sind, sondern beispielsweise Änderungen der Einstellparameter (wie beispielsweise des Resonanzwinkels für die Einkopplung in einen Schichtwellenleiter mittels einer Gitterstruktur, siehe unten) oder der Ausbreitungsparameter dieses Lichts (wie beispielsweise der Phasendifferenz zwischen Lichtanteilen, welche unterschiedliche optische Wege, wie den Messpfad eines Interferometers und den Referenzpfad, ohne Wechselwirkung mit einer Probe, durchlaufen) gemessen werden.

Unter "im wesentlichen optischer Transparenz" eines Materials, einer Schicht oder eines festen Trägers bei einer bestimmten Wellenlänge soll verstanden werden, dass die Lauflänge eines in besagtem Material oder in besagter Schicht oder in besagtem Träger oder in der (hochbrechenden) wellenleitenden Schicht eines als optischer Wellenleiter ausgebildeten Trägers geführten Lichts bei der betreffenden Wellenlänge grösser als 2 mm ist, sofern diese Lauflänge nicht durch Strukturen zur Änderung der Ausbreitungsrichtung besagten Lichts begrenzt wird. Beispielsweise kann die Lauflänge, beispielsweise von optisch sichtbarem Licht, d. h. die Strecke auf dem Weg des Lichts im entsprechenden Material bis zur Abnahme der Lichtintensität auf einen Wert 1/e der Ursprungsintensität beim Eintritt des Lichts in dieses Material, in der Größenordnung von etlichen Zentimetern (z. B. in Dünnschichtwellenleitern) bis zu Metern oder Kilometern (im Falle von Lichtleitern für die optische Signalübermittlung) liegen. Im Falle einer Gitter-Wellenleiter-Struktur, basierend auf einem Dünnschichtwellenleiter, kann die Ausbreitungslänge eines in der wellenleitenden Schicht geführten Lichts durch ein auskoppelndes diffraktives Gitter (ausgebildet in der wellenleitenden Schicht, siehe unten) auf wenige Mikrometer begrenzt werden. Diese Begrenzung der Lauflänge ist dann jedoch durch die Strukturierung, und nicht durch die Materialeigenschaften der Struktur, bedingt. Im Sinne der vorliegenden Erfindung soll eine solche Gitter-Wellenleiter-Struktur als "im wesentlichen optisch transparent" bezeichnet werden, wenn die Lauflänge des Lichts außerhalb der Bereiche der Gitterstrukturen mehr als 2 mm beträgt.

Vorzugsweise ist auch das Material einer optional auf dem festen Träger aufgebrachten Haftvermittlungsschicht mindestens bei der Wellenlänge eines eingestrahlten Anregungslichts oder Messlichts im wesentlichen optisch transparent.

Das Material des festen Trägers umfasst vorzugsweise ein Material aus der Gruppe umfassend Silikate, wie beispielsweise Glas oder Quarz, Keramiken, Metalloxide, Kunststoffe, insbesondere thermoplastische Kunststoffe, wie beispielsweise Polycarbonate, Acrylate, Polyacrylate, insbesondere Polymethylmethacrylate, Polystyrole, Cyclo-Olefinpolymere und Cyclo-Olefin-Copolymere und deren Kombinationen (Mischungen und/oder Schichtungen). Dabei wird bevorzugt, dass besagte Kunststoffe form-, präg-, spritz- und/oder fräsbar sind und - für Anwendungen unter Einsatz von Lumineszenzdetektion - eine möglichst niedrige Eigenfluoreszenz aufweisen. Es wird bevorzugt, dass die genannten Materialien die Anforderung nach im wesentlichen optischer Transparenz mindestens bei der Wellenlänge eines eingestrahlten Anregungslichts oder Messlichts erfüllen.

Für verschiedene Anwendungen des erfindungsgemäßen Verfahrens sind Ausführungsformen des festen Trägers erwünscht, welche dadurch gekennzeichnet sind, dass dieser mehrere Schichten mit unterschiedlichen optischen Eigenschaften umfasst.

Eine spezielle Ausführungsform ist **dadurch gekennzeichnet, dass** der feste Träger eine dünne Metallschicht, vorzugsweise umfassend Gold, Silber oder Aluminium, umfasst. Oft wird dabei zusätzlich bevorzugt, dass der Träger eine weitere Zwischenschicht mit einem Brechungsindex < 1.5, beispielsweise aus Siliciumdioxid oder Magnesiumfluorid umfasst, welche sich in Kontakt mit der Metallschicht befindet. Es wird für diese Gruppe von Ausführungsformen eines Trägers außerdem bevorzugt, dass die Dicke der Metallschicht und der eventuellen Zwischenschicht so ausgewählt ist, dass ein Oberflächenplasmon bei der Wellenlänge eines eingestrahlten Anregungslichts und/oder bei der Wellenlänge einer erzeugten Lumineszenz angeregt werden kann. Vorzugsweise beträgt die Dicke der Metallschicht zwischen 10 nm und 1000 nm, besonders bevorzugt zwischen 30 nm und 200 nm.

Für die meisten Anwendungen des erfindungsgemäßen Verfahrens ist es erwünscht, dass mindestens die direkt oder über eine Haftvermittlungsschicht mit den Messbereichen in Kontakt befindliche Schicht des festen Trägers mindestens bei der Wellenlänge eines eingestrahlten Anregungslichts oder Messlichts im wesentlichen optisch transparent ist

Der feste Träger kann Komponenten aus der Gruppe umfassen, welche Mikroskop-Plättchen, Mikrotiterplatten, Nanotiterplatten, Filter (z. B. umfassend Papier), Membranen (z. B. Nitrocellulosemembranen) und mikrostrukturierte Träger (z. B. Wabenstrukturen oder perforierte Strukturen aus Silicium) umfasst. Als Nanotiterplatten werden dabei analog zu den etablierten Mikrotiterplatten (mit typischerweise 96, 384 oder 1536 Probenbehältnissen) ähnlich strukturierte Anordnungen offener Probenbehältnisse, aber kleiner Abmessungen (typischerweise in der Größenordnung von Mikrometern anstelle von Millimetern) bezeichnet.

Eine besonders bevorzugte Ausführungsform eines für das erfindungsgemäße Verfahren geeigneten festen Trägers ist **dadurch gekennzeichnet, dass** der feste Träger einen durchgehenden oder in diskrete wellenleitende Bereiche aufgeteilten optischen Wellenleiter, umfassend eine oder mehrere Schichten, umfasst.

Für den einen oder die mehreren Detektionsschritte des erfindungsgemäßen Verfahrens wird bevorzugt, dass das Anregungs- oder Messlicht von einer oder mehreren polychromatischen oder monochromatischen Lichtquellen zu einem oder mehreren Messbereichen eines oder mehrerer Arrays von Messbereichen geleitet wird und optische Signale aus besagten Messbereichen und/oder Änderungen oder Unterschiede der von besagten Messbereichen ausgehenden optischen Signale ortsaufgelöst gemessen und aufgezeichnet werden.

Bevorzugt hat das Anregungs- oder Messlicht eine Wellenlänge zwischen 200 nm und 1200 nm.

Bevorzugt handelt es sich bei den verwendeten Lichtquellen um solche mit einem schmalen Emissionsspektrum. Besonders bevorzugte Lichtquellen sind Laserdioden und Laser.

Für die ortsaufgelöste Signaldetektion wird vorzugsweise ein ortsauflösender Detektor verwendet, welcher beispielsweise ausgewählt sein kann aus der Gruppe, welche CCD-Kameras, CCD-Chips, Photodioden-Arrays, Avalanche-Dioden-Arrays, Multichannel-Plates und Vielkanal-Photomultiplier umfasst. Für die Detektionsschritte des erfindungsgemäßen Verfahrens geeignete optische Systeme und deren Komponenten sowie optische Detektionsverfahren sind beispielsweise in den internationalen Anmeldungen WO 95/33197, WO 95/33198 und WO 96/35940 beschrieben, deren Inhalt hiermit vollumfänglich als Bestandteil der vorliegenden Erfindung eingeführt wird.

Abhängig von der physikalischen Ausgestaltung des festen Trägers gibt es für die messtechnische Art der Signalerzeugung beim Analytnachweis verschiedene Möglichkeiten. Eine mögliche Variante ist **dadurch gekennzeichnet, dass** die ortaufgelöst zu messenden Änderungen oder Unterschiede optischer Signale auf lokalen Unterschieden des effektiven Brechungsindex an der den Messbereichen zugewandten Oberfläche des festen Trägers oder innerhalb eines Abstandes von weniger als 1 µm von dieser Oberfläche besagten festen Trägers beruhen, welche durch Bindung von Bindungsreagentien und/oder Detektionsreagentien an in diskreten Messbereichen in den dort aufgebrachten komplex zusammengesetzten Proben biologischen Ursprungs enthaltene Analyten hervorgerufen werden.

Eine Untervariante dieses Ausführungsform des erfindungsgemäßen Verfahrens ist **dadurch gekennzeichnet, dass** die ortsaufgelöst zu messenden Änderungen oder Unterschiede optischer Signale auf lokalen Unterschieden der Resonanzbedingungen zur Erzeugung eines Oberflächenplasmons in einer dünnen Metallschicht als Teil besagten festen Trägers beruhen.

Die Bedingungen zur Erzeugung einer Oberflächenplasmonenresonanz sowie zur Kombination mit Lumineszenzmessungen sowie mit wellenleitenden Strukturen sind vielfach in der Literatur beschrieben, beispielsweise in den US-Patenten US-P 5,478,755, US-P 5,841,143, US-P 5,006,716 und US-P 4,649,280.

Messtechnisch sind für die Bestimmung von Änderungen der Resonanzbedingungen der Resonanzwinkel (bei Variation des Einstrahlungswinkels bei konstanter Wellenlänge des eingestrahlten Lichts) und die Resonanzwellenlänge (bei konstantem Einstrahlungswinkel und Variation der eingestrahlten Anregungswellenlänge) zugänglich. Entsprechend kann, bei konstanter Wellenlänge des eingestrahlten Lichts besagte Änderung der Resonanzbedingungen in einer zu messenden Änderung des Resonanzwinkels für die Einstrahlung eines Anregungslichts zur Erzeugung eines Oberflächenplasmons in einer dünnen Metallschicht des festen Trägers bestehen. Entsprechend kann auch besagte Änderung der Resonanzbedingungen in einer Änderung der Resonanzwellenlänge eines eingestrahlten Anregungslichts zur Erzeugung eines Oberflächenplasmons in einer dünnen Metallschicht als Teil des festen Trägers bestehen, wobei in diesem Fall der Einstrahlungswinkel (welcher mindestens bei einer Wellenlänge eines spektral zu variierenden auf den Träger eingestrahlten Lichts gleich dem Resonanzwinkel sein sollte) vorzugsweise konstant gehalten wird.

Besonders bevorzugt werden solche Varianten des erfindungsgemäßen Verfahrens, welche dadurch gekennzeichnet sind, dass der feste Träger einen optischen Dünnschichtwellenleiter umfasst mit einer ersten im wesentlichen optisch transparenten Schicht (a) auf einer zweiten im wesentlichen optisch transparenten Schicht (b), wobei Schicht (a) einen höheren Brechungsindex als Schicht (b) hat und mit den Messbereichen direkt oder vermittelt über eine Haftvermittlungsschicht in Kontakt ist.

Dabei kann die zweite optisch transparente Schicht (b) ein Material aus der Gruppe umfassen, welche Silikate, wie beispielsweise Glas oder Quarz, Keramiken, Metalloxide, Kunststoffe, insbesondere thermoplastische Kunststoffe, wie beispielsweise Polycarbonate, Acrylate, Polyacrylate, insbesondere Polymethylmethacrylate, Polystyrole, Cyclo-Olefinpolymere und Cyclo-Olefin-Copolymere und deren Kombinationen (Mischungen und/oder Schichtungen) umfasst. Dabei wird wiederum bevorzugt, dass besagte Kunststoffe form-, präg-, spritz- und/oder fräsbar sind und - für Anwendungen unter Einsatz von Lumineszenzdetektion - eine möglichst niedrige Eigenfluoreszenz aufweisen. Ausserdem wird bevorzugt, dass die genannten Materialien die Anforderung nach im wesentlichen optischer Transparenz mindestens bei der Wellenlänge eines eingestrahlten Anregungslichts oder Messlichts erfüllen.

Es wird bevorzugt, dass der Brechungsindex der ersten optisch transparenten Schicht (a) grösser als 1.8 ist. Es wird auch bevorzugt, dass die erste optisch transparente Schicht (a) ein Material aus der Gruppe von Silicium-Nitrid, TiO₂, ZnO, Nb₂O₅, Ta₂O₅, HfO₂, und ZrO₂, besonders bevorzugt aus TiO₂, Ta₂O₅ oder Nb₂O₅ umfasst.

Ausführungsformen von als fester Träger für das erfindungsgemäße Verfahren geeigneten (Dünn-)Schichtwellenleitern sind beispielsweise in den internationalen Patentanmeldungen WO 95/33197, WO 95/33198 und WO96/35940 beschrieben.

Für Ausführungsformen des erfindungsgemäßen Verfahrens mit einem festen Träger, welcher einen optischen Wellenleiter umfasst, wird bevorzugt, dass Anregungslicht oder Messlicht von einer oder mehreren Lichtquellen in eine wellenleitende Schicht des festen Trägers über eines oder mehrere optische Koppelemente eingekoppelt wird, welche ausgewählt sind aus der Gruppe, die von Prismenkopplern, evaneszenten Kopplern mit zusammengebrachten optischen Wellenleitern mit überlappenden evaneszenten Feldern, Stirnflächenkopplern mit vor einer Stirnseite der wellenleitenden Schicht angeordneten fokussierenden Linsen, vorzugsweise Zylinderlinsen, und Gitterkopplern gebildet wird.

Besonders bevorzugt wird, dass die Einkopplung von Anregungslicht oder Messlicht in eine wellenleitende Schicht des festen Trägers mithilfe von einer oder mehreren Gitterstrukturen (c) erfolgt, die in der besagten wellenleitenden Schicht als Oberflächenreliefgitter mit einer gewissen Gitterperiode und Gittertiefe ausgeprägt sind.

Vorteilhaft ist auch, wenn die Auskopplung von in einer wellenleitenden Schicht des festen Trägers geführtem Licht mithilfe von einer oder mehreren Gitterstrukturen (c') erfolgt, die in besagter wellenleitender Schicht ausgeprägt sind und gleiche oder unterschiedliche Periode und Gittertiefe wie Gitterstrukturen (c) haben.

Eine weitere auf refraktiven Messmethoden beruhende Variante des erfindungsgemäßen Verfahrens ist **dadurch gekennzeichnet, dass** die ortsaufgelöst zu messenden Änderungen oder Unterschiede optischer Signale auf lokalen Unterschieden der Resonanzbedingungen für die Einkopplung von Anregungslicht oder Messlicht einer oder mehrerer Lichtquellen in eine wellenleitende Schicht des festen Trägers mittels einer in dieser wellenleitenden Schicht ausgeprägten Gitterstruktur beruhen.

Analog zur Bestimmung von Änderungen der Resonanzbedingungen zur Erzeugung einer Oberflächenplasmonenresonanz sind messtechnisch für die Bestimmung von Änderungen der Resonanzbedingungen für die Lichteinkopplung in eine wellenleitende Schicht über ein darin ausgeprägtes Gitter der Resonanzwinkel (bei Variation des Einstrahlungswinkels bei konstanter Wellenlänge des eingestrahlten Lichts) und die Resonanzwellenlänge (bei konstantem Einstrahlungswinkel und Variation der eingestrahlten Anregungswellenlänge) zugänglich. Entsprechend kann, bei konstanter Wellenlänge des eingestrahlten Lichts besagte Änderung der Resonanzbedingungen in einer zu messenden Änderung des Resonanzwinkels für die Lichteinkopplung in eine wellenleitende Schicht des festen Trägers bestehen. Entsprechend kann auch besagte Änderung der Resonanzbedingungen in einer Änderung der Resonanzwellenlänge eines eingestrahlten Anregungslichts für die Lichteinkopplung in eine wellenleitende Schicht des festen Trägers bestehen., wobei in diesem Fall der Einstrahlungswinkel (welcher mindestens bei einer Wellenlänge eines spektral zu variierenden auf den Träger eingestrahlten Lichts gleich dem Resonanzwinkel sein sollte) vorzugsweise konstant gehalten wird.

In der internationalen Patentanmeldung WO 01/88511 sind ausführlich verschiedene Ausführungsformen von Gitter-Wellenleiter-Strukturen beschrieben, welche für den Einsatz als fester Träger für eine Kombination mit dem erfindungsgemäßen Verfahren geeignet sind. Ebenso eignen sich die darin beschriebenen Detektionsverfahren für den Einsatz im Detektionsschritt des erfindungsgemäßen Verfahrens für die vorangehend beschriebene Variante des Einsatz eines refraktiven Messverfahrens. Die WO 01/88511 wird daher hiermit vollumfänglich als Bestandteil der vorliegenden Beschreibung eingeführt.

Hinsichtlich der Technik der Signalerzeugung werden solche Varianten des erfindungsgemäßen Verfahrens bevorzugt, welche dadurch charakterisiert sind, dass die ortsaufgelöst zu messenden Änderungen oder Unterschiede optischer Signale auf lokalen Unterschieden oder Änderungen einer oder mehrerer Lumineszenzen beruhen, welche durch Bindung von Bindungsreagentien und/oder Detektionsreagentien an in diskreten Messbereichen in den dort aufgebrachten komplex zusammengesetzten Proben biologischen Ursprungs enthaltene Analyten hervorgerufen werden.

Von besonderem Vorteil ist, wenn zum Analytnachweis zwei oder mehr Lumineszenzlabel mit unterschiedlichen Emissionswellenlängen und/oder unterschiedlichen Anregungsspektren, bevorzugt mit unterschiedlichen Emissionswellenlängen und gleicher Anregungswellenlänge, eingesetzt werden. Wenn mehrere Lumineszenzlabel mit unterschiedlichen spektralen Eigenschaften, insbesondere unterschiedlichen Emissionswellenlängen, an unterschiedliche Bindungs- und/oder Detektionsreagentien, welche mit den Messbereichen in Kontakt gebracht werden, gebunden sind, können beispielsweise unterschiedliche Analyten innerhalb eines einzelnen Messbereichs in einem einzigen Nachweisschritt, d.h. Kontaktierung der Messbereiche mit besagten Bindungs- und/oder Detektionsreagentien und gleichzeitige oder nachfolgende Detektion der erzeugten Lumineszenzen, bestimmt werden.

Beispielsweise ist eine solche Variante des erfindungsgemässen Verfahrens besonders gut dafür geeignet, gleichzeitig zum Beispiel die phoshorylierte und die nicht phosphorylierte Form einer Verbindung, insbesondere auch innerhalb eines einzelnen (gemeinsamen) Messbereiches nachzuweisen, mithilfe zweier entsprechender unterschiedlicher, beispielsweise direkt (z. B. mit grün bzw. rot emittierenden Lumineszenzlabeln) markierter Bindungsreagentien als spezifischen Bindungspartnern.

In ähnlicher Weise können auch innerhalb eines einzelnen Messbereichs gleichzeitig zwei oder mehr verschiedene Analyten detektiert werden, wenn zum Analytnachweis zwei oder mehr Lumineszenzlabel mit unterschiedlichen Emissionsabklingzeiten eingesetzt werden und, bei geeigneten Anregungsbedingungen (d.h. gepulster oder modulierter Lichtanregung) die Detektion resultierender Lumineszenzen mit einer Zeitauflösung erfolgt, welche die Unterscheidung der unterschiedlich schnell abklingenden Lumineszenzen zulässt..

Von Vorteil ist, wenn die Einstrahlung des Anregungslichts in Pulsen mit einer Dauer zwischen 1 fsec und 10 Minuten erfolgt und das Emissionslicht aus den Messbereichen zeitlich aufgelöst gemessen wird.

Eine ganz besonders bevorzugte Ausführungsform des erfindungsgemäßen Verfahrens ist **dadurch gekennzeichnet, dass**
- der feste Träger einen optischen Schichtwellenleiter mit einer ersten bei mindestens der Wellenlänge eines eingestrahlten Anregungslichts im wesentlichen optisch transparenten Schicht (a) auf einer zweiten bei mindestens der Wellenlänge eines eingestrahlten Anregungslichts im wesentlichen optisch transparenten Schicht (b) mit niedrigerem Brechungsindex als Schicht (a) umfasst,
- Anregungslicht einer Lichtquelle mittels einer in der Schicht (a) ausgeprägten Gitterstruktur (c) in die Schicht (a) eingekoppelt wird,
- Dieses Anregungslicht als eine geführte Welle zu direkt auf der Schicht (a) oder vermittelt über eine Haftvermittlungsschicht auf der Schicht (a) befindlichen Messbereichen geleitet wird und
- Lumineszenzen von lumineszenzfähigen Verbindungen, welche im evaneszenten Feld des in der Schicht (a) geführten Lichts zur Lumineszenz angeregt werden, ortsaufgelöst gemessen werden.

Eine besondere Variante besteht dabei darin, dass neben der Bestimmung einer oder mehrerer Lumineszenzen Änderungen des effektiven Brechungsindex auf den Messbereichen bestimmt werden.

Für eine weitere Verbesserung der Empfindlichkeit kann dabei vorteilhaft sein, wenn die einen oder mehreren Lumineszenzen und/oder Bestimmungen von Lichtsignalen bei der Anregungswellenlänge polarisationsselektiv vorgenommen werden. Dabei wird bevorzugt, dass die einen oder mehreren Lumineszenzen bei einer anderen Polarisation als der des Anregungslichts gemessen werden.

Ein weiterer Gegenstand der vorliegenden Erfindung ist ein Mikroarray zur quantitativen Bestimmung eines oder mehrerer Analyten in einer oder mehreren komplex zusammengesetzten Proben biologischen Urspungs, umfassend
- einen festen Träger,
- eine erste Vielzahl von diskreten Messbereichen, in denen direkt oder vermittelt über eine Haftvermittlungsschicht jeweils kleine Mengen komplex zusammengesetzter Proben biologischen Ursprungs in verdünnter oder unverdünnter Form immobilisiert sind,
**dadurch gekennzeichnet,**
**dass** mindestens eine zweite Vielzahl von Messbereichen in besagtem Array auf dem festen Träger bereitgestellt ist, in welchen Messbereichen den nachzuweisenden Analyten gleichartige Stoffe in unterschiedlichen Konzentrationen immobilisiert sind, welche geeignet sind, mittels Kontaktierung des Mikroarrays mit einer ersten Lösung enthaltend ein oder mehrere Bindungsreagentien als spezifische Bindungspartner für die in der ersten Vielzahl von diskreten Messbereichen in den aufgebrachten komplex zusammengesetzten Proben biologischen Ursprungs enthaltenen, nachzuweisenden Analyten und die in der zweiten Vielzahl von diskreten Messbereichen enthaltenen, besagten nachzuweisenden Analyten gleichartigen Stoffe, und, gegebenenfalls falls erforderlich, ein oder mehr Detektionsreagentien, wobei die Aufbringung von Bindungs- und Detektionsreagentien gleichzeitig oder sequentiell erfolgen kann, und nachfolgende ortsaufgelöste Messung von ersten optischen Signalen, welche von diskreten Messbereichen eines oder mehrerer Arrays, welche mit der ersten Lösung in Kontakt gebracht wurden, ausgehen, sowie Aufzeichnung dieser ersten optischen Signale, eine Kalibrationskurve für besagte in den immobiliserten komplex zusammengesetzten Proben enthaltenen, quantitativ nachzuweisenden Analyten zu erzeugen.

Es kann von Vorteil sein, wenn eine dritte Vielzahl von Messbereichen in besagtem Array auf dem festen Träger bereitgestellt ist, in denen jeweils kleine Mengen komplex zusammengesetzter Proben biologischen Ursprungs in verdünnter oder unverdünnter Form und zusätzlich diesen zugesetzte bekannte Mengen von den nachzuweisenden Analyten gleichartigen Stoffen immobilisiert sind. Diese dritte Vielzahl kann beispielsweise für die Kontrollfunktion der Bestimmung des Grades der "Wiederfindung" ("Recovery") benutzt werden, wie dieses auch in dem nachfolgenden Ausführungsbeispiel der Fall ist.

Eine Weiterentwicklung des erfindungsgemäßen Mikroarrays besteht darin, dass eine vierte Vielzahl von Messbereichen in besagtem Array auf dem festen Träger bereitgestellt ist, in welchen Stoffe, welche ähnlicher Art wie in der Probenmatrix der in der ersten Vielzahl von Messbereichen aufgebrachten Proben enthaltene Stoffe sind, immobilisiert sind.

Es wird bevorzugt, dass auch die Messbereiche der zweiten Vielzahl von Messbereichen Stoffe umfassen, welche ähnlicher Art wie in der Probenmatrix der in der ersten Vielzahl von Messbereichen aufgebrachten Proben enthaltene Stoffe sind.

Die Stoffe, welche ähnlicher Art wie in der Probenmatrix der in der ersten Vielzahl von Messbereichen aufgebrachten Proben enthaltene Stoffe sind, können beispielsweise aus der Gruppe stammen, welche Albumine, insbesondere Rinderserumalbumin, Immunoglobuline, Transferrine und Fibrinogene umfasst.

Es wird außerdem bevorzugt, dass eine fünfte Vielzahl von Messbereichen in besagtem Array auf dem festen Träger bereitgestellt ist, welche Zwecken einer Referenzierung dienen. Dabei wird weiterhin bevorzugt, dass die Messbereiche der fünften Vielzahl von Messbereichen Stoffe umfassen, welche ausgewählt sind aus der Gruppe, welche Massenlabel, beispielsweise in der Form von Nanopartikeln, Beads oder Kolloiden, und Lumineszenzlabel, beispielsweise in der Form von Lumineszenzfarbstoffen oder lumineszenten Nanopartikeln wie "Quantum Dots" mit Anregungs-und Emissionswellenlängen zwischen 200 nm und 1000 nm, umfasst.

Für das erfindungsgemäße Mikroarray eignen sich alle Ausführungsformen fester Träger und deren Modifikationen (beispielsweise durch Aufbringung einer Haftvermittlungsschicht), wie sie für das vorangehend beschriebene erfindungsgemäße Verfahren (zur Unterscheidung der Anteile spezifischer und unspezifischer Bindung an den beobachteten Signalen) beschrieben wurden. Ebenso können alle für dieses erfindungsgemäße Verfahren beschriebenen komplex zusammengesetzten Proben, Bindungsreagentien, Detektionsreagentien und Arrays von Messbereichen in Verbindung mit einem erfindungsgemäßen Mikroarray eingesetzt werden.

Ein weiterer Gegenstand der vorliegenden Erfindung ist ein Verfahren zur quantitativen Bestimmung eines oder mehrerer Analyten in einer komplex zusammengesetzten Probe biologischen Ursprungs umfassend die folgenden Schritte:
- Bereitstellung einer oder mehrerer komplex zusammengesetzter Proben biologischen Ursprungs,
- Bereitstellung einer Vielzahl von Lösungen von den in den komplex zusammengesetzten Proben nachzuweisenden Analyten gleichartigen Stoffen in unterschiedlichen Konzentrationen,
- Bereitstellung mindestens eines festen Trägers,
- Erzeugung einer ersten Vielzahl von diskreten Messbereichen als Teil eines Mikroarrays durch Aufbringung kleiner Mengen der komplex zusammengesetzten Proben biologischen Ursprungs, in verdünnter oder unverdünnter Form, an diskreten Orten direkt auf dem festen Träger oder nach vorheriger Aufbringung einer Haftvermittlungsschicht auf besagter Haftvermittlungsschicht auf dem festen Träger,
- Erzeugung einer zweiten Vielzahl von diskreten Messbereichen als Teil besagten Mikroarrays durch Aufbringung jeweils kleiner Mengen der Vielzahl von Lösungen von den in den komplex zusammengesetzten Proben nachzuweisenden Analyten gleichartigen Stoffen in unterschiedlichen Konzentrationen,
- Kontaktierung des Mikroarrays mit einer ersten Lösung enthaltend ein oder mehrere Bindungsreagentien als spezifische Bindungspartner für die in der ersten Vielzahl von diskreten Messbereichen in den aufgebrachten komplex zusammengesetzten Proben biologischen Ursprungs enthaltenen, nachzuweisenden Analyten und die in der zweiten Vielzahl von diskreten Messbereichen enthaltenen, besagten nachzuweisenden Analyten gleichartigen Stoffe, und, gegebenenfalls falls erforderlich, ein oder mehr Detektionsreagentien, wobei die Aufbringung von Bindungs- und Detektionsreagentien gleichzeitig oder sequentiell erfolgen kann,
- ortsaufgelöste Messung von ersten optischen Signalen, welche von der ersten und zweiten Vielzahl diskreter Messbereiche des Mikroarrys ausgehen, Aufzeichnung der ersten optischen Signale aus der ersten Vielzahl diskreter Messbereiche als für deren Kontaktierung mit der ersten Lösung charakteristische Signale, Aufzeichung der ersten optischen Signale aus der zweiten Vielzahl diskreter Messbereiche als für deren Kontaktierung mit der ersten Lösung in Abhängigkeit von der Konzentration der in besagten Messbereichen enthaltenen, den in den komplex zusammengesetzten Proben enthaltenen Analyten gleichartigen Stoffen, charakteristische Signale,
- Erstellung von Kalibrationskurven für die in den komplex zusammengesetzten Proben nachzuweisenden Analyten aus den aufgezeichneten optischen Signalen aus der zweiten Vielzahl diskreter Messbereiche, sofern erforderlich nach vorheriger Subtraktion von Hintergrundsignalen und geeigneter Referenzierung,
quantitative Bestimmung der in den komplex zusammengesetzten Proben enthaltenen nachzuweisenden Analyten durch Vergleich der aufgezeichneten ersten optischen Signale aus der ersten Vielzahl diskreter Messbereiche mit den Kalibrationskurven für die jeweiligen Analyten, sofern erforderlich nach vorheriger Subtraktion von Hintergrundsignalen und geeigneter Referenzierung.

Dieses erfindungsgemäße Verfahren zur quantitativen Bestimmung eines oder mehrerer Analyten in einer komplex zusammengesetzten Probe biologischen Ursprungs kann mit den Ausführungsformen des vorangehend genannten erfindungsgemäßen Verfahrens nach Anspruch 1 und seinen Unteransprüchen kombiniert werden. Diese Kombinationen sind ebenfalls Bestandteil der vorliegenden Erfindung.

Die Erfindung umfasst außerdem die Verwendung eines erfindungsgemäßen Mikroarrays und/oder eines der erfindungsgemäßen Verfahren zur quantitativen Bestimmung eines oder mehrerer Analyten in einer komplex zusammengesetzten Probe biologischen Ursprungs zu quantitativen und/oder qualitativen Analysen zur Bestimmung chemischer, biochemischer oder biologischer Analyten in Verfahren des Screenings von Wirkstoffbibliotheken zur Effizienzbestimmung in der Pharmaforschung, der Kombinatorischen Chemie, der Klinischen und Präklinischen Entwicklung, zur Identifizierung, Validierung und Kontrolle ("monitoring") von biologischen oder chemischen Markerstoffen ("biomarkers"), zur Identifizierung und Verifizierung von Signaltransduktionswegen in der Proteomforschung und Systembiologie, zum Affinitätsscreening und insbesondere zum Antikörperscreening, zu Echtzeitbindungsstudien und zur Bestimmung kinetischer Parameter im Affinitätsscreening und in der Forschung, zu qualitativen und quantitativen Analytbestimmungen, insbesondere für die DNA- und RNA-Analytik und die Bestimmung von genomischen oder proteomischen Unterschieden im Genom bzw. Proteom, wie beispielsweise Einzelnukleotid-Polymorphismen, zur Messung von Protein-DNA-Wechselwirkungen, zur Bestimmung von Steuerungsmechanismen für die m-RNA-Expression und für die Protein(bio)synthese, für die Erstellung von Toxizitätsstudien sowie für die Bestimmung von Expressionsprofilen, insbesondere zur Erstellung von zellulären Expressionsprofilen von krankhaften und gesunden Zellpopulationen, mit und ohne äußere Stimulation, und deren Vergleich, zur Untersuchung der zeitlichen Entwicklung derartiger Expressionsprofile über Zeiträume von Minuten, Stunden, Tagen, Wochen, Monaten oder Jahren, zur Bestimmung von biologischen und chemischen Markerstoffen, wie mRNA, Proteinen, Peptiden oder niedermolekularen organischen (Boten)Stoffen, sowie zum Nachweis von Antikörpern, Antigenen, Pathogenen oder Bakterien in der pharmazeutischen Protduktforschung und -entwicklung, der Human- und Veterinärdiagnostik, der Agrochemischen Produktforschung und -entwicklung, der symptomatischen und präsymptomatischen Pflanzendiagnostik, zur Patientenstratifikation in der pharmazeutischen Produktentwicklung und für die therapeutische Medikamentenauswahl, zum Nachweis von Pathogenen, Schadstoffen und Erregern, insbesondere von Salmonellen, Prionen, Viren und Bakterien, insbesondere in der Lebensmittel- und Umweltanalytik.

Dabei ist das erfindungsgemäße Verfahren aufgrund der hohen Anzahl gleichzeitig auf einem gemeinsamen Träger unter gleichen Bedingungen durchführbarer Untersuchungen insbesondere für Affinitätsscreening, d.h. für den Vergleich der Affinitäten verschiedener Bindungspartner zu einem ihnen gemeinsamen spezifischen Bindungspartner, insbesondere von verschiedenen Antikörpern zu einen ihnen gemeinsamen Antigen, geeignet.

Besonders bevorzugt wird auch die Verwendung des erfindungsgemäßen Verfahrens zur Erstellung zellulärer Expressionsprofile und deren Vergleich. Dieses betrifft insbesondere den Vergleich zellulärer Expressionsprofile von krankhaften und gesunden Zellpopulationen, mit und ohne deren Stimulation. Dabei soll als "Stimulation" sowohl die Zugabe chemischer oder biochemischer Verbindungen zu besagten Zellpopulationen, als auch deren Behandlung mit unterschiedlichen physikalischen Bedingungen, wie beispielsweise Bestrahlung, Hitzeeinwirkung etc. verstanden werden. Aufgrund der hohen Genauigkeit der durch das erfindungsgemäße Verfahren erzielbaren Messergebnisse ist es dabei insbesondere auch geeignet für die Untersuchung der zeitlichen Entwicklung zellulärer Expression unter den vorgenannten Bedingungen, über Zeiträume von beispielsweise Minuten, Stunden, Tagen, Wochen, Monaten oder Jahren.

Außerdem ist das erfindungsgemäße Verfahren besonders gut geeignet zur Auffindung so genannter biologischer oder biochemischer Markerstoffe der vorgenannten Art, welche geeignet sind, Informationen zu liefern über krankhafte Zellpopulationen im Vergleich zu gesunden Zellpopulationen, mutierten oder modifizierten Zellpopulationen im Vergleich zu "wild type" Zellpopulationen oder über die Beeinflussung zellulärer Populationen durch deren Stimulation oder Behandlung.

Das erfindungsgemäße Mikroarray und die erfindungsgemäßen Verfahren werden nachfolgend beispielhaft erläutert.

### Beispiele:

### 1. Analyten und Proben

In dem nachfolgenden Ausführungsbeispiel handelt es sich bei den nachzuweisenden Analyten um das Markerprotein "Akt" des intrazellulären Akt-Signalwegs und um zwei unterschiedlich aktivierte (phosphorylierte) Formen dieses Proteins. Akt ist eine Proteinkinase und spielt eine Schlüsselrolle bei einer Vielzahl physiologischer Prozesse, wie beispielsweise im Glucose-Metabolismus, dem Zellwachstum, der Zelldifferenzierung und dem programmierter Zelltod (Apoptose). Die Aktivierung von Akt erfolgt über Phosphorylierung unterschiedlicher Aminosäureseitenketten, unter anderem Serin 473 und Threonin 308. Fehlregulationen des Akt-Signalwegs und die damit einhergehende Inhibition des programmierten Zelltods spielen eine entscheidende Rolle bei der Entstehung von Krebs, weshalb dieses Markerprotein von grossem therapeutischem Interesse ist.

Der analytische Nachweis von Akt und dessen phosphorylierten Form en P-Akt (Ser473) und P-Akt (Thr308) erfolgt mittels unterschiedlicher spezifischer Antikörper, die entweder an das Protein unabhängig von dessen Phosphorylierungsgrad binden, oder nur an bestimmte phosphorylierte Formen, wie nur an P-Akt (Ser473) oder nur an P-Akt (Thr308). Somit lässt sich durch die Messung einer Probe mittels Applikation dreier unterschiedlicher Antikörper der Gesamtgehalt an Akt sowie der jeweilige Gehalt dessen modifizierter Formen detektieren.

Der Nachweis von Akt und dessen phosphorylierten Form en erfolgt in diesem Ausführungsbeispiel in einem unfiltrierten Lysat von Rattenherzgewebe, welches in einem stark denaturierenden, Harnstoff und Detergentien enthaltenden Lysepuffer präpariert wurde und welches das gesamte Proteom der darin befindlichen Zellen enthielt. Weiterhin wurde Rattenserum, welches kein Akt enthielt, in Lysepuffer präpariert.

### 2. Im erfindungsgemäßen Verfahren verwendete Träger

Die für das erfindungsgemäße Verfahren verwendeten festen Träger, jeweils mit den Abmessungen 14 mm Breite x 57 mm Länge x 0.7 mm Dicke, sind als Dünnschichtwellenleiter ausgeprägt, jeweils umfassend ein Glasssubstrat (AF 45) als im wesentlichen optisch transparente Schicht (b) und eine darauf aufgebrachte 150 nm dünne, hochbrechende Schicht aus Tantalpentoxid, als im wesentliche optisch transparente Schicht (a). In dem Glassubstrat sind, parallel zur Länge, zwei Oberflächenreliefgitter (Gitterperiode: 318 nm, Gittertiefe: (12 +/- 2) nm) im Abstand von 9 mm moduliert. Bei der nachfolgenden Auftragung der hochbrechenden Schicht wurden diese Strukturen, die als diffraktive Gitter der Lichteinkopplung in die hochbrechende Schicht (a) dienen sollen, in die Oberfläche der Tantalpentoxidschicht übertragen.

Derartige Dünnschichtwellenleiter sind für das erfindungsgemäße Verfahren besonders gut geeignet, da sie es ermöglichen, oberflächennahe Bindungsereignisse mit einem hohen Verhältnis von Messignal zu Hintergrundsignal zu detektieren, so dass damit tiefe Nachweisgrenzen erreicht werden können. Prinzipiell sind jedoch auch alle anderen Ausführungsformen fester Träger, wie sie vorangehend genannt wurden, wie beispielsweise Mikrokop-Plättchen oder auch Mikrotiterplatten, für das erfindungsgemäße Verfahren geeignet.

Nach sorgfältiger Reinigung der Träger wird auf der Oberfläche der Metalloxidschicht eine Monoschicht aus Mono-Dodecylphosphat (DDP) durch spontane Selbstorganisation als Haftvermittlungsschicht erzeugt, mittels Abscheidung aus wässriger Lösung (0.5 mM DDP). Diese Oberflächenmodifikation der zuvor hydrophilen Metalloxidoberfläche führt zu einer hydrophoben Oberfläche (mit einem Kontaktwinkel von etwa 100° gegenüber Wasser), auf der die komplex zusammengesetzten Proben biologischen Ursprungs mit darin enthaltenen Analyten als spezifischen Bindungspartnern für den Analytnachweis in einer spezifischen Bindungsreaktion aufgetragen werden sollen.

### 3. Reagentien und Erzeugung der Arrays von Messbereichen

Für Kompetitionsexperimente (siehe unten) zur Bestimmung von durch unspezifische Bindung an den Analyten hervorgerufene Signalanteile wird gereinigtes Akt (Pharmacia Italia Spa ,Mailand, Italien) als Kompetitor in Lösung zu dem in den immobilisierten komplex zusammengesetzten Proben biologischen Ursprungs enthaltenen nachzuweisenden Akt ("endogenem Akt") als Analyt verwendet. Das gereinigte Akt wird auch zur Erstellung von Kalibrationskurven mittels Zusatz in unterschiedlichen zu aufzubringenden Immobilisierungslösungen unterschiedlicher Zusammensetzung eingesetzt (siehe unten).

Als Gesamtprotein-Konzentrationen der Stammlösung für die Proben (Lysat von Rattenherzgewebe in Lyse-Puffer), aus denen die auf dem festen Träger aufzubringenden komplex zusammengesetzten Proben biologischen Ursprungs hergestellt werden, sowie von Rattenserum als einem Medium mit vergleichbarer Zusammensetzung wie die Probenmatrix der aus dem Lysat des Rattenherzgewebes hergestellten Proben wird mithilfe eines modifizierten Bradford-Assays (PIERCE Coomassie Plus Kit (PIERCE # 23238) 17.3 mg/ml bzw. 54.0 mg/ml bestimmt.

Durch weitere Verdünnung mit einem zweiten, ebenfalls harnstoffhaltigen, aber von Detergentien freien Puffer ("Spotting-Puffer") werden Lösungen des Lysats des Rattenherzgewebes mit unterschiedlichem Gesamtproteingehalt (0.025 mg/ml, 0.050 mg/ml, 0.1 mg/ml, 0.2 mg/ml, 0.3 mg/ml, 0.4 mg/ml, 0.5 mg/ml) für die Aufbringung in einzelnen Messbereichen hergestellt, ohne die Proteinzusammensetzung der verdünnten Lösungen gegenüber der Stammlösung zu ändern. Die erhaltenen Lösungen unterschiedlicher Gesamtproteinkonzentration stellen zu untersuchende komplex zusammengesetzte Proben biologischen Ursprungs (ohne weiter hinzugefügte Zusätze) dar.

Außerdem werden Lösungen des Lysats des Rattenherzgewebes mit gleichen Gesamtproteinkonzentrationen (0.025 mg/ml, 0.050 mg/ml, 0.1 mg/ml, 0.2 mg/ml, 0.3 mg/ml, 0.4 mg/ml, 0.5 mg/ml), aber zusätzlich jeweils 1000 ng/ml Akt, hergestellt. Mit den aus diesen Lösungen zu erzeugenden Messbereichen soll überprüft werden, ob der Analytnachweis (von Akt bzw. seinen phosphorylierten Formen) unabhängig von der Gesamtproteinkonzentration im jeweiligen Messbereich ist.

Zwecks Erstellung von Kalibrationskurven von Akt und dessen phosphorylierten Formen werden des weiteren Lösungen von gereinigtem Akt (0 ng/ml, 1 ng/nl, 3 ng/ml, 10 ng/ml, 30 ng/ml, 100 ng/ml, 300 ng/ml, 1000 ng/ml, 3000 ng/ml) in Spotting-Puffer, jeweils unter Zusatz von 0.1 mg/ml Rinderserumalbumin (BSA), hergestellt. BSA dient hier als homogene und von Akt freie Probenmatrix, in der das Akt entsprechend den genannten unterschiedlichen Konzentrationen der Immobilisierungslösungen auf dem Träger in diskreten Messbereichen immobilisiert werden soll.

Ebenfalls zum Zweck der Erzeugung von Kalibrationskurven von Akt und den genannten phosphorylierten Formen, aber in einer anderen Probenmatrix, werden Lösungen mit gleichem Gehalt an gereinigtem Akt (0 ng/ml, 1 ng/nl, 3 ng/ml, 10 ng/ml, 30 ng/ml, 100 ng/ml, 300 ng/ml, 1000 ng/ml, 3000 ng/ml) in Spotting-Puffer, jedoch mit Zusatz von 0.1 mg/ml Rattenserum anstelle von 0.1 mg/ml BSA, hergestellt. Rattenserum dient hier als heterogene und dem Rattenhergewebe ähnliche, jedoch von Akt freie Probenmatrix, in der das Akt entsprechend den genannten unterschiedlichen Konzentrationen der Immobilisierungslösungen auf dem Träger in diskreten Messbereichen immobilisiert werden soll.

Auf den mit der hydrophoben Haftvermittlungsschicht versehenen Trägern werden jeweils 6 identische Mikroarrays von je 224 Messbereichen (Spots), ihrerseits in einer Anordnung von jeweils 14 Reihen und 16 Spalten, mit einem Inkjet-Spotter (GeSIM, Großerkmannsdorf, Deutschland) aufgetragen. Jeder Spot wird durch die Auftragung eines einzelnen Tröpfchens von etwa 400 pl Volumen auf die Oberfläche des Trägers erzeugt. Pro Lösung werden jeweils zwei gleichartige Messbereiche (zwei replikate Spots) erzeugt. Außerdem werden für Negativkontrollen auch Messbereiche mit darin aufgebrachtem Spotting-Puffer, ohne jegliche Proteine als Inhaltsstoffe, erzeugt.

Außer den vorgenannten "Proben-Spots" umfassen die Mikroarrays auch jeweils sogenannte "Referenz-Spots": Innerhalb jedes Mikroarrays ist in einem Teil der jeweils 224 Messbereiche mit Cy5 (Amersham) fluoreszenzmarkiertes Rinderserumalbumin (Cy5-BSA, Markierungsrate: 3 Cy5-Moleküle pro BSA-Molekül) immobilisiert. Diese Messbereiche dienen der Referenzierung von lokalen Unterschieden der Anregungslichtintensität innerhalb einzelner Arrays als auch zwischen verschiedenen Arrays. ("Referenz-Spots"). Cy5-BSA wird in diesen Messbereichen jeweils in einer Konzentration von 0.5 nM in harnstoffhaltigem Spottingpuffer aufgetragen.

Die geometrische Anordnung der Messbereiche in den jeweils gleichartigen Arrays ist in Fig. 1 dargestellt und in Tab. 1 erläutert.

Nach Fertigstellung der Arrays von Messbereichen werden die freien, nicht protein-bedeckten, hydrophoben Oberflächenbereiche der Träger zu deren Passivierung mit Rinderserumalbumin (BSA), als einer gegenüber einzusetzenden Bindungsreagentien und/oder Detektionsreagentien "chemisch neutralen", d.h. diese nicht bindende Komponente, abgesättigt, indem die Oberflächen mit einer BSA-haltigen Pufferlösung 30 Minuten lang inkubiert werden. Danach werden die Träger mit den darauf erzeugten Messbereichen mit Wasser (18 MΩ·cm) gewaschen und zuletzt in einem Stickstoffstrom getrocknet und bei 4°C bis zur Durchführung des erfindungsgemäßen Nachweisverfahrens gelagert.

### 4. Architektur von Assays zur Durchführung des erfindungsgemäßen Verfahrens

Zur weiteren Durchführung des erfindungsgemäßen Verfahrens werden die mit den Arrays von Messbereichen versehenen Träger jeweils mit einem Aufsatzkörper zur Erzeugung einer linearen Anordnung von 6 Probenbehältnissen (jeweils 15 µl Innenvolumen) mit den darin angeordneten Arrays von Messbereichen verbunden. Solche Anordnungen von Probenbehältnissen sind in den internationalen Anmeldungen WO 01/43875 und WO 02/103331 beschrieben, deren Inhalt hiermit vollumfänglich als Bestandteil der vorliegenden Anmeldung eingeführt wird.

Der Schritt des Analytnachweises (entsprechend Schritt (4) des erfindungsgemäßen Verfahrens) erfolgt in zwei Teilschritten, wobei jeweils ein Analyt pro Array von Messbereichen nachgewiesen wird.

### 4.1. Verwendung von Bindungsreagentien ohne Zusatz von Kompetitoren

Im ersten Teilschritt werden die Arrays von Messbereichen in den Probenbehältnissen mit einer Lösung eines primären Antikörpers als Bindungsreagenz ("anti-Akt" (# 9272) zur Detektion von Total-Akt (ohne Unterscheidung von phosphorylierten und nicht phosphorylierten Formen), "anti-P-Akt (Ser473)" (#9271) zur Detektion von phosphoryliertem P-Akt (Ser473), "anti-P-Akt (Thr308)"(#9275) zur Detektion von phosphoryliertem P-Akt (Thr308) (alle Antikörper von Cell Signaling Technologies, Beverly, USA) jeweils in 500-facher Verdünnung der Stammlösung in Assay-Puffer (entsprechend ca. 5nM), enthaltend über Nacht bei Raumtemperatur inkubiert.

Nach einem Waschschritt mit jeweils 200 µl Assay-Puffer, zur Entfernung nicht gebundener Bindungsreagentien, werden die Arrays von Messbereichen jeweils im zweiten Teilschritt mit dem Detektionsreagenz, nämlich fluoreszenzmarkiertem Alexa Fluor 647 anti-rabbit Fab Fragmenten (Molecular Probes; Eugene, USA), ebenfalls in 500-facher Verdünnung der Stammlösung in Assay-Puffer, sechzig Minuten lang im Dunkeln bei Raumtemperatur inkubiert. Danach werden die Arrays von Messbereichen nochmals mit je 200 µl Assay-Puffer gewaschen, um nicht gebundene Detektionsreagentien zu entfernen. Anschließend werden die diesen Verfahrensschritten unterzogenen Träger, verbunden mit Aufsatzkörpern und so erzeugten, mit Puffer gefüllten Probenbehältnissen, gelagert bis zum Detektionsschritt mittels Anregung und Detektion resultierender Fluoreszenzsignale im ZeptoREADER^{™} (siehe unten).

### 4.2. Verwendung von Bindungsreagentien mit Zusatz von Akt als Kompetitor

Entsprechend erfolgt der Analytnachweis in Gegenwart eines dem Analyten gleichartigen Kompetitors um die spezifische Bindung an die eingesetzten Bindungsreagentien und gegebenenfalls zusätzliche Nachweisreagentien ebenfalls in zwei Teilschritten der Zugabe auf den festen Träger mit den darauf befindlichen Arrays von Messbereichen: Zunächst werden die Lösungen der Bindungsreagentien ("anti-Akt", "anti-P-Akt (Ser473)" bzw. "anti-P-Akt (Thr308)", jeweils ca. 5 nM) jeweils mit einem etwa zwanzigfachen Überschuss von Akt (5 µg/ml entsprechend 100 nM) vorinkubiert. Es wird erwartet, dass dabei alle Antigen-Bindungsstellen der analytspezifischen Antikörper mit dem entsprechenden Kompetitor belegt werden und folglich keine spezifischen Bindungsstellen der Bindungsreagentien mehr für die Reaktion mit Analytmolekülen in den Messbereichen zur Verfügung stehen. Dann werden die so hergestellten, Akt enthaltenden Lösungen analog zum ersten Teilschritt von 4.1. in jeweils ein weiteres Probenbehältnis mit einem weiteren, aber gleichartigen Array von Messbereichen wie dem unter 4.1. benutzten Array von Messbereichen gefüllt und damit über Nacht inkubiert, gefolgt von einem Waschschritt, dem nachfolgenden Teilschritt der Zugabe des Detektionsreagenz und den weiteren Teilschritten wie unter 4.1. beschrieben. Das Vorgehen gemäß dieses Abschnittes 4.2. entspricht dem Teilschritt (7a) des erfindungsgemäßen Verfahrens.

### 4.3. Verwendung von Bindungsreagentien mit Zusatz von der Probenmatrix ähnlichen Stoffen als Kompetitoren

In zu 4.1. und 4.2. analoger Weise erfolgt der Analytnachweis in Gegenwart von zusätzlich zu den Bindungsreagentien hinzugefügten Stoffen, welche gleicher oder möglichst ähnlicher Art wie in der Probenmatrix enthaltene Stoffe sind und als Kompetitoren zu den in den Messbereichen immobilisierten Bestandteilen der Probenmatrix um unspezifische Bindung der Bindungsreagentien dienen. Dazu werden die Lösungen der Bindungsreagentien ("anti- Akt", "anti-P-Akt (Ser473)" bzw. "anti-P-Akt (Thr308)", jeweils ca. 5 nM) jeweils mit 0.1 mg/ml Rattenserum vorinkubiert. Dann werden die so hergestellten, Serum enthaltenden Lösungen analog zum ersten Teilschritt von 4.1. in jeweils ein weiteres Probenbehältnis mit einem weiteren, aber gleichartigen Array von Messbereichen wie dem unter 4.1. benutzten Array von Messbereichen gefüllt und damit über Nacht inkubiert, gefolgt von einem Waschschritt, dem nachfolgenden Teilschritt der Zugabe des Detektionsreagenz und den weiteren Teilschritten wie unter 4.1. beschrieben. Das Vorgehen gemäß dieses Abschnittes 4.3. entspricht dem Teilschritt (7b) des erfindungsgemäßen Verfahrens.

### 5. Anregung und Detektion von Fluoreszenzsignalen aus den Arrays von Messbereichen

Die Fluoreszenzsignale aus den verschiedenen Arrays von Messbereichen werden mit einem ZeptoREADER^{™} (Zeptosens AG, CH-4108 Witterswil, Schweiz) sequentiell automatisch gemessen. Dieses optische System wurde in der internationalen Patentanmeldung PCT/EP 01/10012 genauer beschrieben, welche hiermit vollumfänglich als Bestandteil dieser Anmeldung eingeführt wird.

### 6. Bildauswertung und Referenzierung

Die Bestimmung der Fluoreszenzsignale aus den Messbereichen (Spots) erfolgt mithilfe einer Bildanalyse-Software (ZeptoVIEW^{™}, Pro 2.0 Release 2.0, Zeptosens AG, CH-4108 Witterswil). Hierbei wird für jeden Spot die mittlere Signalintensität sowie die mittlere lokale Hintergrundsignalintensität in dessen unmittelbarer Umgebung bestimmt. Durch Subtraktion des mittleren lokalen Hintergrundsignals von der mittleren gemessenen Gesamtsignal-intensität des betreffenden Spots wird für jeden Spot die hintergrundbereinigte mittlere Netto-signalintensität bestimmt.

Die Referenzierung der Nettosignalintensität aller Spots erfolgt jeweils mithilfe der Cy5-BSA Referenzspots. Hierzu werden die Nettosignalintensitäten der "Proben-Spots" durch die auf die Position des jeweiligen "Proben-Spots" extrapolierte, gemittelte Signalintensität der beiden nächsten in einer Reihe benachbarten "Referenz-Spots" dividiert. Durch diese Referenzierung werden die lokalen Unterschiede der verfügbaren Anregungslichtintensität innerhalb eines jeden Arrays von Messbereichen als auch zwischen verschiedenen Arrays kompensiert.

ReferenzierteNetto-Signalintensitätender Spot-Duplikate (von jeweils zwei gleichartigen Messbereichen) werden zuletzt gemittelt und sind in den entsprechenden Figuren als "RFI"-Datenpunkte dargestellt (Referenced Fluorescence Intensity), angegebene Fehlerbalken entsprechen den jeweiligen Standardabweichungen

### 7. Durchführung des erfindungsgemäßen Verfahrens und Resultate

### 7.1. Bestimmung des Gesamtgehalts an Akt

### 7.1.1. Kalibrationskurven für den Nachweis von Akt

### a) Verwendung von Bindungsreagentien ohne Zusatz von Kompetitoren

Zwei Segmente von Messbereichen sind in den gleichartigen Arrays von Messbereichen jeweils vorgesehen für die Erstellung von Kalibrationskurven der Kinase Akt (siehe Fig. 1):
Segment 1: Array-Reihen I und II mit Messbereichs-Inhaltsnummern 1 - 9 mit aufgebrachten unterschiedlichen Konzentrationen (zwischen 0 ng/ml und 3000 ng/ml) von gereinigtem Akt in Spotting-Puffer mit zusätzlichen 0.1 mg/ml BSA,
Segment 2: Array-Reihen III und IV mit Messbereichs-Inhaltsnummern 13 - 21 mit aufgebrachten unterschiedlichen Konzentrationen (zwischen 0 ng/ml und 3000 ng/ml) von gereinigtem Akt in Spotting-Puffer mit zusätzlichen 0.1 mg/ml Rattenserum,

In ein erstes Probenbehältnis mit einem ersten Array von Messbereichen wird eine Lösung von "anti-Akt" (5 nM) aufgegeben. Die weiteren Verfahrensteilschritte wurden vorangehend unter 4.1 beschrieben. Die Kalibrationskurven des Segments 1 von Messbereichen (erzeugt mit Lösungen enthaltend unterschiedliche Konzentrationen von Akt und dem Spotting-Puffer zugesetztes 0.1 mg/ml BSA) (dargestellt durch ausgefüllte Quadrate) und des Segments 2 von Messbereichen (erzeugt mit Lösungen enthaltend unterschiedliche Konzentrationen von Akt und dem Spotting-Puffer zugesetztes 0.1 mg/ml Rattenserum) (dargestellt durch ausgefüllte Kreise) sind in Fig. 2 dargestellt.

Bei Konzentrationen oberhalb von 30 ng/ml Akt fallen die Kalibrationskurven von Messbereichen mit co-immobilisiertem BSA und mit co-immobilisiertem Rattenserum zusammen; bei Konzentrationen unterhalb von 30 ng/ml weist die Kalibrationskurve von Messbereichen mit co-immobilisiertem Rattenserum höhere Signalwerte als diejenige mit co-immobilisiertem BSA auf. Der Signalunterschied wird dem Beitrag unspezifischer Bindung des Bindungsreagenz an die Inhaltsstoffe des immobilisierten Rattenserums zugeordnet. Bei Hinzufügung noch höherer Konzentrationen von Rattenserum zur Immobiliserungslösung ist der Signalunterschied zu Messbereichen, deren erzeugenden Immobilisierungslösungen lediglich BSA hinzugefügt wurde, noch ausgeprägter.

Für die Bestimmung der unbekannten Akt-Konzentrationen in den aus Rattenherzgewebe hergestellten Proben werden die Kalibrationskurven benutzt, welche mithilfe von Immobilisierungslösungen, denen lediglich BSA hinzugefügt wurde, erzeugt wurden, da sie eine Konzentrationsbestimmung auch noch bei niedrigen Akt-Konzentrationen, unterhalb von 30 ng/ml, ermöglichen..

### b) Verwendung von Bindungsreagentien mit Zusatz von Akt als Kompetitor

Zur Überprüfung des Anteils von Signalen aufgrund unspezifischer Bindung an den Kalibrationskurven, welche mit den Immobilisierungslösungen mit unterschiedlicher Probenmatrix erstellt wurden, wird in ein zweites Probenbehältnis auf ein weiteres Array mit gleichartiger Anordnung der Messbereiche wie das zuerst untersuchte Array eine Mischung der Lösung des Antikörpers "anti-Akt" als Bindungsreagenz mit einem Überschuss von Akt (5 µg/ml entsprechend 100 nM Konzentration) gefüllt und mit den Messbereichen über Nacht inkubiert, wie unter 4.2. beschrieben. Es wird erwartet, dass unter diesen Bedingungen der Anteil an durch spezifische Bindung des Antikörpers an immobilisiertes Akt hervorgerufenen Signalen vollständig verschwindet, während der Anteil durch unspezifische Bindung hervorgerufener Signale verbleibt.

Erwartungsgemäß wird eine innerhalb der Messgenauigkeit komplette Unterdrückung des (durch spezifische Bindung verursachten) Signals innerhalb des relevanten Konzentrationsbereichs zur Bestimmung von endogenem Akt, nämlich unterhalb von 100 ng/ml, beobachtet (dargestellt durch leere Symbole in Fig. 2)). Dabei sind die Signale aus Messbereichen, deren Immobilisierungslösungen Rattenserum enthielten (dargestellt durch leere Kreise), höher als die Signale derjenigen Messbereiche, deren Immobilisierungslösungen, außer den definierten Akt-Konzentrationen, lediglich BSA enthielten (dargestellt durch leere Quadrate), entsprechend den erwarteten unterschiedlichen Anteilen unspezifischer Bindung an die Probenmatrixkomponenten. Der bei den höchsten Akt-Konzentrationen (1000 ng/ml und 3000 ng/ml in der Immobilisierungslösung) beobachtete Signalanstieg wird darauf zurückgeführt, dass die Kompetitor-Konzentration in Lösung unter diesen Bedingungen offensichtlich nicht ausreichend ist, um eine spezifische Bindung des Bindungsreagenz an immobilisiertes Akt vollständig zu verhindern.

### c) Verwendung von Bindungsreagentien mit Zusatz von der Probenmatrix ähnlichen Stoffen als Kompetitoren

Zur Überprüfung des Anteils unspezifischer Bindung, welche durch Bindung der Bindungsreagentien an Komponenten der Probenmatrix verursacht werden, wird der Lösung des Antikörpers Rattenserum mit einer Gesamtproteinkonzentration von 0.1 mg/ml hinzugesetzt. Diese Lösung wird dann in ein drittes Probenbehältnis mit einem dritten Array wiederum gleichartiger Anordnung der Messbereiche wie das vorgenannte erste und zweite Array gefüllt, entsprechend dem Vorgehen gemäß Abschnitt 4.3. Es wird erwartet, dass unter diesen Voraussetzungen die Anteile durch spezifische Bindung an immobilisiertes Akt erzeugter Signale erhalten bleiben und die Anteile durch unspezifische Bindung an die Probenmatrix erzeugter Signale weitgehend verschwinden.

Figur 3 zeigt mit ausgefüllten Symbolen wiederum die unter Abschnitt 7.1.1.a) besprochene Kalibrationskurve, die ohne Zusatz von Kompetitoren zu den Bindungsreagentien mit dem ersten Array erzeugt wurden. Die durch Zugabe der Rattenserum enthaltenden Lösung des Antikörpers als Bindungsreagenz mit dem dritten Array erhaltenen Signale (dargestellt durch leere Quadrate) unterscheiden sich von dieser Kalibrationskurven nicht messbar.

Hieraus ist zu schließen, dass im Falle des Antikörpers "anti-Total-Akt" die erstellte Kalibrationskurve mit hoher Genauigkeit den spezifischen Bindungsereignissen entspricht und eine unspezifische Bindung an die Probenmatrix nur in sehr geringem Umfang stattfindet.

### 7.1.2. Bestimmung des Gesamtgehalts von Akt in aus Rattenherzgewebe hergestellten Proben

### a) Verwendung von Bindungsreagentien ohne Zusatz von Kompetitoren

Der Nachweis des Gesamtgehalts von Akt in den aus Rattenherzgewebe hergestellten, komplex zusammengesetzten Proben biologischen Ursprungs erfolgt auf dem Segment von Messbereichen, welche mit den Messbereichs-Inhaltsnummern 25 bis 31 gekennzeichnet sind.

Die zur Erzeugung dieser Messbereiche erzeugten Immobilisierungslösungen stammen aus derselben Stammlösung und wurden durch Verdünnung (siehe Abschnitt 3.) auf unterschiedliche Gesamtproteinkonzentrationen (zwischen 0.025 mg/ml und 0.5 mg/ml) eingestellt.

Das Segment von Messbereichen mit den Messbereichs-Inhaltsnummern 37 bis 43 wurde durch Aufbringung von Immobilisierungsproben aus einer gleichartigen Verdünnungsreihe der Stammlösung aus dem Lysat des Rattenherzgewebes erzeugt, wobei den Immobilisierungsproben aber jeweils noch gereinigtes Akt in einer Konzentration von 1000 ng/ml zugesetzt wurde. Die mithilfe dieses Segments zu messenden Signale sollen der Kontrolle dienen, ob durch Vergleich der zu messenden Fluoreszenzsignale mit der Kalibrationskurve (erstellt mit Messbereichen mit co-immobilisiertem B SA) bei gleicher Gesamtproteinkonzentration (0.1 mg/ml) diese eingesetzte hohe Akt-Konzentration (welche wesentlich höher als der erwartete natürliche, endogene Akt-Gehalt ist) wiedergefunden wird.

Zusammen mit dem unter 7.1.1.a beschriebenen Teil des erfindungsgemäßen Verfahrens wird die Messung durchgeführt mittels Zugabe einer Lösung von "anti-Akt" (5 nM) in das erste Probenbehältnis mit dem darin befindlichen ersten Array von Messbereichen. Die weiteren Verfahrensteilschritte wurden vorangehend unter 4.1 beschrieben.

Die Ergebnisse (referenzierte Fluoreszenzintensitäten) sind in Fig. 4 dargestellt. Die Fluoreszenzsignale aus Messbereichen, in denen aus Rattenherzgewebe hergestellte Immobilisierungslösungen aufgebracht wurden, sind als Funktion der Gesamtproteinkonzentration dargestellt (Fig. 4, obere Abszissenachse), und die Werte der referenzierten Fluoreszenzsignale der Kalibrationsmessung mit co-immobilisiertem BSA (Proteinkonzentration: 0.1 mg/ml) sind als Funktion der Akt-Konzentrationen der für diese Messbereiche benutzten Immobilisierungslösungen (Fig. 4, untere Abszissenachse) dargestellt.

Die Signale aus den Messbereichen ohne zugesetztes Akt (d.h. mit in den immobilisierten Proben natürlich vorkommendem, endogenem Akt) steigen zunächst erwartungsgemäß mit zunehmender Proteinkonzentration an und erreichen ab einer Proteinkonzentration von 0.2 mg/ml einen Maximalwert. Bei weiter steigender Proteinkonzentration wird keine weitere Signalerhöhung beobachtet, ähnlich zu einem Sättigungseffekt.

Die Signale aus den Messbereichen mit 1000 ng/ml zugesetztem Akt (d.h. mit in den immobilisierten Proben natürlich vorkommendem, endogenem und zusätzlich 1000 ng/ml gereinigtem Akt) erreichen entsprechend sehr hohe Werte. Aus dem Vergleich des Signalwertes mit der gleichzeitig generierten Kalibrationskurve bei vergleichbarer Totalproteinkonzentration (0.1 mg/ml, illustriert durch die durchbrochene Linie bei einer Proteinkonzentration von 0.1 mg/ml sowie durch die vom Schnittpunkt dieser Linie mit der Messkurve für Signale aus Messbereichen mit 1000 ng/ml zugegebenem Akt in Richtung der Kalibrationskurve verlaufenden durchbrochenen Linie), kann die der Probe zugegebene 1000 ng/ml Akt-Konzentration mit (840 ± 70) ng/ml wiedergefunden und bestimmt werden. Die erreichte Wiederfindungsrate ("Recovery") von 84 % bzw. Präzision von 8% befindet sich im Rahmen der allgemein tolerierten Grenzen von 80 % - 120 % Wiederfindungsrate bzw. besser als 20 % Präzision in einem Assay. In diesem Beispiel wird also gezeigt, dass eine Analytbestimmung mit guter Assaygenauigkeit und Präzision durchgeführt werden kann. Der hohe Wiederauffindungswert bestätigt zugleich, dass die auf Messbereichen mit 0.1 mg/ml den Immobilisierungslösungen zugesetztem BSA (als Proteinmatrix) erzeugte Messkurve gut zur Kalibration der Messwerte von Spots, welche aus Lösungen mit Rattenherzgewebe-Lysaten hergestellt wurden, geeignet ist.

Durch Vergleich mit der Kalibrationskurve (illustriert durch die durchbrochene Linie bei einer Proteinkonzentration von 0.1 mg/ml sowie durch die vom Schnittpunkt dieser Linie mit der Messkurve für Signale aus Messbereichen mit nur endogenem Akt in Richtung der Kalibrationskurve verlaufenden durchbrochenen Linie) wird für die aus Rattenherzgewebe hergestellte Probe bei einem Proteingehalt von 0.1 mg/ml ein Gehalt von (20 ± 2) ng/ml endogenem Akt bestimmt.

### b) Verwendung von Bindungsreagentien mit Zusatz von Akt als Kompetitor

Zur Bestimmung des Anteils von Signalen unspezifischer Bindung an der Messkurve aus Messbereichen, welche mit den Immobilisierungslösungen basierend auf Rattenherzgewebe als Probenmatrix erzeugt wurden, wird in ein zweites Probenbehältnis auf ein weiteres Array mit gleichartiger Anordnung der Messbereiche wie das zuerst untersuchte Array eine Mischung der Lösung des Antikörpers "anti-Akt" als Bindungsreagenz mit einem Überschuss von Akt (5 µg/ml entsprechend 100 nM) gefüllt und mit den Messbereichen über Nacht inkubiert, wie unter 4.2. beschrieben. Es wird erwartet, dass unter diesen Bedingungen der Anteil durch spezifische Bindung des Antikörpers an immobilisiertes Akt hervorgerufener Signale vollständig verschwindet, während der Anteil durch unspezifische Bindung hervorgerufener Signale verbleibt.

Die Ergebnisse des Kompetitionsexperiments sind in Fig. 5 dargestellt, zusammen mit den Resultaten der entsprechenden, vorangehend unter 7.1.2.a) besprochenen Messungen ohne Anwesenheit eines Kompetitors in der Lösung des Bindungsreagenz. Ausgefüllte Symbole kennzeichnen jeweils die Ergebnisse ohne Anwesenheit eines Kompetitors (gemessen auf dem ersten Array), leere Symbole die in Gegenwart des Kompetitors gemessenen Signale (gemessen auf dem zweiten Array). Die Fluoreszenzsignale aus Messbereichen, deren Immobilisierungslösungen aus Lysaten von Rattenherzgewebe hergestellt wurden (sowohl zur Bestimmung des natürlich vorkommenden, endogenen Akt, ohne zusätzlich hinzugefügtes Akt, Messbereichsinhaltskennzahlen 25 bis 31, als auch mit 1000 ng/ml zusätzlich hinzugefügtem, gereinigtem Akt, Messbereichsinhaltskennzahlen 37 bis 43, gemäß Fig. 1) sind als Funktion der Proteinkonzentration der Immobilisierungslösung dargestellt (Fig. 5, obere Abszisse). Die in 7.1.1. besprochene Kalibrationskurve für den Nachweis von Akt (mit 0.1 mg/ml BSA in den Immobiliserungslösunge) ist aufgetragen als Funktion der Akt-Konzentration (Fig. 5, untere Abszisse).

In Anwesenheit des Kompetitors zeigen die Signale aus den Messbereichen zur Bestimmung des endogenen Akt eine deutliche Verminderung. Die Differenz zwischen den Messkurven in Abwesenheit und in Anwesenheit des Kompetitors stellt den Signalanteil dar, welcher durch spezifische Bindung hervorgerufen wird.

Für die Messbereiche, deren Immobilsierungslösung zusätzlich Akt im Überschuss (1000 ng/ml) enthielten, wird eine noch deutlichere Reduktion der referenzierten Fluoreszenzsignale beobachtet. Die in Anwesenheit von 100 nM Akt als Kompetitor in Lösung erzeugten Messkurven aus den beiden Segmenten von Messbereichen (zur Bestimmung des endogenen Akt bzw. zur Kontrollmessung mit 1000 ng/ml Akt in der Immobilisierungslösung) fallen exakt zusammen. Das verbleibende Restsignal (leere Symbole), welches bis zu einer Proteinkonzentration von 0.3 mg/ml ansteigt und dann konstant bleibt, entspricht dem Signalbeitrag infolge unspezifischer Bindung (an die Proteine der Probenmatrix), welcher naturgemäß mit steigender Oberflächenkonzentration der Proteine ansteigt, bis die Oberfläche der Messbereiche vollständig bedeckt ist.

Fig. 6 illustriert die Bestimmung der durch spezifische Bindung verursachten Signalanteile bei einer Proteinkonzentration von 0.1 mg/ml aus dem Vergleich der Messwerte in Anwesenheit und Abwesenheit des Kompetitors in Lösung (Fig. 6a) und die Bestimmung des Gehalts an endogenem Akt aus dem Vergleich des durch spezifische Bindung ("SB") verursachten Signalanteils mit der Kalibrationskurve (Fig. 6b). Die durch unspezifische Bindung ("NSB") hervorgerufenen Signalanteile (0.20 RFI) sind für die Signale aus Messbereichen mit nur endogenem Akt (0.373 RFI) und solchen mit zusätzlich hinzugegebenen 1000 ng/ml gereinigtem Akt (ca. 20 RFI) innerhalb der Messgenauigkeit gleich hoch. Aus Vergleich der Differenz zwischen Gesamtsignal und unspezifisch hervorgerufenem Signal mit der Kalibrationskurve wird ein Gehalt von (8.8 ± 1.3) ng/ml endogenem Akt bestimmt (Fig. 6b). Der Vergleich mit der Bestimmung von Akt in Abschnitt 7.1.2.a), in der durch unspezifische Bindung erzeugte Signalanteile nicht diskriminiert wurden, zeigt, dass etwa 56 % des dort vorläufig bestimmten Gehalts an Akt dem Beitrag unspezifischer Bindung zuzuordnen sind.

### c) Verwendung von Bindungsreagentien mit Zusatz von der Probenmatrix ähnlichen Stoffen als Kompetitoren

Zur Bestimmung des Anteils unspezifischer Bindung, welche durch Bindung der Bindungsreagentien an Komponenten der Probenmatrix verursacht werden, wird der Lösung des Antikörpers Rattenserum mit einer Gesamtproteinkonzentration von 0.1 mg/ml hinzugesetzt. Diese Lösung wird dann in ein drittes Probenbehältnis mit einem dritten Array wiederum gleichartiger Anordnung der Messbereiche wie das vorgenannte erste und zweite Array gefüllt, entsprechend dem Vorgehen gemäß Abschnitt 4.3. Es wird erwartet, dass unter diesen Voraussetzungen die Anteile durch spezifische Bindung an immobilisiertes Akt erzeugter Signale erhalten bleiben und die Anteile durch unspezifische Bindung an die Probenmatrix erzeugter Signale weitgehend verschwinden.

Die Ergebnisse des Kompetitionsexperiments mit Rattenserum in Lösung sind in Fig. 7 dargestellt, zusammen mit den Resultaten der entsprechenden, vorangehend unter 7.1.2.a) besprochenen Messungen ohne Anwesenheit eines Kompetitors in der Lösung des Bindungsreagenz. Ausgefüllte Symbole kennzeichnen jeweils die Ergebnisse ohne Anwesenheit eines Kompetitors (gemessen auf dem ersten Array), leere Symbole die in Gegenwart des Kompetitors gemessenen Signale (gemessen auf dem dritten Array). Die Fluoreszenzsignale aus Messbereichen, deren Immobilisierungslösungen aus Lysaten von Rattenherzgewebe hergestellt wurden (sowohl zur Bestimmung des natürlich vorkommenden, endogenen Akt, ohne zusätzlich hinzugefügtes Akt, Messbereichsinhaltskennzahlen 25 bis 31, als auch mit 1000 ng/ml zusätzlich hinzugefügtem Akt, Messbereichsinhaltskennzahlen 37 bis 43, gemäß Fig. 1) sind als Funktion der Proteinkonzentration der Immobilisierungslösung dargestellt (Fig. 7, obere Abszisse). Die in 7.1.1. besprochene Kalibrationskurve für den Nachweis von Akt (mit 0.1 mg/ml BSA in den Immobiliserungslösunge) ist aufgetragen als Funktion der Akt-Konzentration (Fig. 7, untere Abszisse).

Die Anwesenheit von Rattenserum in Lösung als Kompetitor für unspezifische Bindung führt zu keiner signifikanten Verschiebung der Messkurven; die in Anwesenheit und Abwesenheit von Rattenserum gemessenen referenzierten Fluoreszenzsignale sind innerhalb der Messgenauigkeit jeweils identisch.

Hieraus ist zu schließen, dass im Falle des Antikörpers "anti-Total-Akt" eine unspezifische Bindung an die Probenmatrix nicht in signifikantem Umfang stattfindet.

### 7.2. Bestimmung des Gehalts an P-Akt (Ser473)

### 7.2.1. Kalibrationskurven für den Nachweis von P-Akt (Ser473)

### a) Verwendung von Bindungsreagentien ohne Zusatz von Kompetitoren

Zur Erstellung von Kalibrationskurven für die Bestimmung der phosphoryliserten Form P-Akt (Ser473) werden Segmente gleichartiger Messbereiche wie für die vorangehend beschriebene Bestimmung von Akt verwendet.

Segment 1: Array-Reihen I und II mit Messbereichs-Inhaltsnummern 1 - 9 mit aufgebrachten unterschiedlichen Konzentrationen (zwischen 0 ng/ml und 3000 ng/ml) von gereinigtem Akt in Spotting-Puffer mit zusätzlichen 0.1 mg/ml BSA,

Segment 2: Array-Reihen III und IV mit Messbereichs-Inhaltsnummern 13 - 21 mit aufgebrachten unterschiedlichen Konzentrationen (zwischen 0 ng/ml und 3000 ng/ml) von gereinigtem Akt in Spotting-Puffer mit zusätzlichen 0.1 mg/ml Rattenserum,

Es wird angenommen, dass das gereinigte Akt in vollständig phosphorylierter Form, d.h. an Ser473 und an Thr308 phosphorylierter Form, vorliegt.

In ein viertes Probenbehältnis mit einem vierten Array von Messbereichen wird eine Lösung von anti-P-Akt (Ser473) (5 nM) aufgegeben. Die weiteren Verfahrensteilschritte wurden vorangehend unter 4.1 beschrieben. Die Kalibrationskurven des Segments 1 von Messbereichen (erzeugt mit Lösungen enthaltend unterschiedliche Konzentrationen von Akt und dem Spotting-Puffer zugesetztes 0.1 mg/ml BSA), des Segments 2 von Messbereichen (erzeugt mit Lösungen enthaltend unterschiedliche Konzentrationen von Akt und dem Spotting-Puffer zugesetztes 0.1 mg/ml Rattenserum) sind in Fig. 8 (ausgefüllte Symbole) dargestellt.

Bei Konzentrationen oberhalb von 10 ng/ml Akt fallen die Kalibrationskurven von Messbereichen mit co-immobilisiertem BSA und mit co-immobilisiertem Rattenserum zusammen; bei Konzentrationen unterhalb von 10 ng/ml weist die Kalibrationskurve von Messbereichen mit co-immobilisiertem Rattenserum höhere Signalwerte als diejenige mit co-immobilisiertem BSA auf. Der Signalunterschied wird dem Beitrag unspezifischer Bindung des Bindungsreagenz an die Inhaltsstoffe des immobilisierten Rattenserums zugeordnet. Bei Hinzufügung noch höherer Konzentrationen von Rattenserum zur Immobiliserungslösung ist der Signalunterschied zu Messbereichen, deren erzeugenden Immobilisierungslösungen lediglich BSA hinzugefügt wird, noch ausgeprägter.

Für die Bestimmung der unbekannten P-Akt (Ser473) -Konzentrationen in den aus Rattenherzgewebe hergestellten Proben werden die Kalibrationskurven benutzt, welche mithilfe von Immobilisierungslösungen, denen lediglich BSA hinzugefügt wurde, erzeugt wurden, da sie eine Konzentrationsbestimmung auch noch bei niedrigen Akt-Konzentrationen, unterhalb von 10 ng/ml, ermöglichen.

### b) Verwendung von Bindungsreagentien mit Zusatz von Akt als Kompetitor

Zur Überprüfung des Anteils von Signalen unspezifischer Bindung an den Kalibrationskurven, welche mit den Immobilisierungslösungen mit unterschiedlicher Probenmatrix erstellt wurden, wird in ein fünftes Probenbehältnis auf ein weiteres Array mit gleichartiger Anordnung der Messbereiche wie die zuvor untersuchten Arrays eine Mischung der Lösung des Antikörpers "anti-P-Akt (Ser473)" als Bindungsreagenz mit einem Überschuss von Akt (5 µg/ml entsprechend 100 nM) gefüllt und mit den Messbereichen über Nacht inkubiert, wie unter 4.2. beschrieben. Es wird erwartet, dass unter diesen Bedingungen der Anteil durch spezifische Bindung des Antikörpers an immobilisiertes P-Akt (Ser473) hervorgerufener Signale verschwindet, während der Anteil durch unspezifische Bindung hervorgerufener Signale verbleibt.

Erwartungsgemäß wird innerhalb der Messgenauigkeit eine komplette Unterdrückung des (durch spezifische Bindung verursachten) Signals innerhalb des relevanten Konzentrationsbereichs zur Bestimmung von endogenem P-Akt (Ser473), nämlich unterhalb von 100 ng/ml, beobachtet (leere Symbole in Fig. 8). Dabei sind die Signale aus Messbereichen, deren Immobilisierungslösungen Rattenserum enthielten, höher als die Signale derjenigen Messbereiche, deren Immobilisierungslösungen, außer den definierten Akt-Konzentrationen, lediglich BSA enthielten, entsprechend den erwarteten unterschiedlichen Anteilen unspezifischer Bindung an die Probenmatrixkomponenten.

Der bei den höchsten Konzentrationen an P-Akt (Ser473) (1000 ng/ml und 3000 ng/ml in der Immobilisierungslösung) beobachtete Signalanstieg wird darauf zurückgeführt, dass die Kompetitor-Konzentration in Lösung unter diesen Bedingungen offensichtlich nicht ausreichend ist, um eine spezifische Bindung des Bindungsreagenz an immobilisiertes P-Akt (Ser473) vollständig zu verhindern.

### c) Verwendung von Bindungsreagentien mit Zusatz von der Probenmatrix ähnlichen Stoffen als Kompetitoren

Zur Überprüfung des Anteils unspezifischer Bindung, welche durch Bindung der Bindungsreagentien an Komponenten der Probenmatrix verursacht werden, wird der Lösung des Antikörpers Rattenserum mit einer Gesamtproteinkonzentration von 0.1 mg/ml hinzugesetzt. Diese Lösung wird dann in ein sechstes Probenbehältnis mit einem sechsten Array wiederum gleichartiger Anordnung der Messbereiche wie die vorgenannten Arrays gefüllt, entsprechend dem Vorgehen gemäß Abschnitt 4.3. Es wird erwartet, dass unter diesen Voraussetzungen die Anteile durch spezifische Bindung an immobilisiertes P-Akt (Ser473) erzeugter Signale erhalten bleiben und die Anteile durch unspezifische Bindung an die Probenmatrix erzeugter Signale weitgehend verschwinden.

Figur 9 zeigt mit ausgefüllten Symbolen wiederum die unter Abschnitt 7.2.1.a) besprochenen Kalibrationskurve, die ohne Zusatz von Kompetitoren zu den Bindungsreagentien mit dem vierten Array erzeugt wurden. Die durch Zugabe der Rattenserum enthaltenden Lösung des Antikörpers als Bindungsreagenz mit dem sechsten Array erhaltenen Signale unterscheiden sich von diesen Kalibrationskurven nicht messbar.

Hieraus ist zu schließen, dass auch im Falle des Antikörpers "anti-P-Akt (Ser473)" die erstellte Kalibrationskurve mit hoher Genauigkeit den spezifischen Bindungsereignissen entspricht und eine unspezifische Bindung an die Probenmatrix nur in sehr geringem Umfang stattfindet.

### 7.2.2. Bestimmung des Gehalts von P-Akt (Ser473) in aus Rattenherzgewebe hergestellten Proben

### a) Verwendung von Bindungsreagentien ohne Zusatz von Kompetitoren

Der Nachweis des Gehalts von P-Akt (Ser473) in den aus Rattenherzgewebe hergestellten, komplex zusammengesetzten Proben biologischen Ursprungs erfolgt auf dem Segment von Messbereichen, welche mit den Messbereichs-Inhaltsnummern 25 bis 31 gekennzeichnet sind.

Die zur Erzeugung dieser Messbereiche erzeugten Immobilisierungslösungen stammen aus derselben Stammlösung und wurden durch Verdünnung (siehe Abschnitt 3.) auf unterschiedliche Gesamtproteinkonzentrationen (zwischen 0.025 mg/ml und 0.5 mg/ml) eingestellt.

Das Segment von Messbereichen mit den Messbereichs-Inhaltsnummern 37 bis 43 wurde durch Aufbringung von Immobilisierungsproben aus einer gleichartigen Verdünnungsreihe der Stammlösung aus dem Lysat des Rattenherzgewebes erzeugt, wobei den Immobilisierungsproben aber jeweils noch gereinigtes Akt in einer Konzentration von 1000 ng/ml zugesetzt wurde. Die mithilfe dieses Segments zu messenden Signale sollen der Kontrolle dienen, ob durch Vergleich der zu messenden Fluoreszenzsignale mit der Kalibrationskurve für P-Akt (Ser473) (erstellt mit Messbereichen mit co-immobilisiertem BSA) bei gleicher Gesamtproteinkonzentration (0.1 mg/ml) ein der eingesetzten hohen "Total-Akt"-Konzentration (welche wesentlich höher als der erwartete natürliche, endogene Akt-Gehalt ist) entsprechender hoher Gehalt an P-Akt (Ser473) wiedergefunden wird

Zusammen mit dem unter 7.2.1.a beschriebenen Teil des erfindungsgemäßen Verfahrens wird die Messung durchgeführt mittels Zugabe einer Lösung von "anti-P-Akt (Ser473)" (5 nM) in das vierte Probenbehältnis mit dem darin befindlichen vierten Array von Messbereichen. Die weiteren Verfahrensteilschritte wurden vorangehend unter 4.1 beschrieben.

Die Ergebnisse (referenzierte Fluoreszenzintensitäten) sind in Fig. 10 dargestellt. Die Fluoreszenzsignale aus Messbereichen, in denen aus Rattenherzgewebe hergestellte Immobilisierungslösungen aufgebracht wurden, sind als Funktion der Gesamtproteinkonzentration dargestellt (Fig. 10, obere Abszissenachse), und die Werte der referenzierten Fluoreszenzsignale der Kalibrationsmessung mit co-immobilisiertem BSA (Proteinkonzentration: 0.1 mg/ml) sind als Funktion der Konzentrationen an P-Akt (Ser473) der für diese Messbereiche benutzten Immobilisierungslösungen (Fig. 10, untere Abszissenachse) dargestellt.

Die Signale für P-Akt (Ser473) aus den Messbereichen ohne zugesetztes Akt (d.h. mit in den immobilisierten Proben natürlich vorkommendem, endogenem P-Akt (Ser473)) steigen zunächst erwartungsgemäß mit zunehmender Proteinkonzentration an und erreichen ab einer Proteinkonzentration von 0.2 mg/ml einen Maximalwert. Bei weiter steigender Proteinkonzentration wird keine signifikante weitere Signalerhöhung beobachtet, ähnlich zu einem Sättigungseffekt.

Die Signale aus den Messbereichen mit 1000 ng/ml zugesetztem P-Akt (Ser473) (d.h. mit in den immobilisierten Proben natürlich vorkommendem, endogenem und zusätzlich 1000 ng/ml gereinigtem P-Akt (Ser473)) erreichen entsprechend sehr hohe Werte. Hierbei wird vorausgesetzt, dass das zur Erzeugung der Kalibrationskurven benutzte gereinigte Akt in vollständig phosphorylierter Form, d.h. an Ser473 und an Thr308 phosphorylierter Form, vorliegt. Aus dem Vergleich des Signalwertes mit der gleichzeitig generierten Kalibrationskurve bei vergleichbarer Totalproteinkonzentration (0.1 mg/ml, illustriert durch die durchbrochene Linie bei einer Proteinkonzentration von 0.1 mg/ml sowie durch die vom Schnittpunkt dieser Linie mit der Messkurve für Signale aus Messbereichen mit 1000 ng/ml zugegebenem P-Akt (Ser473) in Richtung der Kalibrationskurve verlaufenden durchbrochenen Linie), kann die der Probe zugegebene Konzentration von 1000 ng/ml P-Akt (Ser473) mit (870 ± 100) ng/ml wiedergefunden und bestimmt werden (siehe gestrichelte Linie). Die erreichte Wiederfindungsrate ("Recovery") von 92 %. Der hohe Wiederauffindungswert bestätigt zugleich, dass die auf Messbereichen mit 0.1 mg/ml den Immobilisierungslösungen zugesetztem BSA (als Proteinmatrix) erzeugte Messkurve gut zur Kalibration der Messwerte von Spots, welche aus Lösungen mit Rattenherzgewebe-Lysaten hergestellt wurden, geeignet ist.

Durch Vergleich mit der Kalibrationskurve (illustriert durch die durchbrochene Linie bei einer Proteinkonzentration von 0.1 mg/ml sowie durch die vom Schnittpunkt dieser Linie mit der Messkurve für Signale aus Messbereichen mit nur endogenem P-Akt (Ser473) in Richtung der Kalibrationskurve verlaufenden durchbrochenen Linie) wird für die aus Rattenherzgewebe hergestellte Probe bei einem Proteingehalt von 0.1 mg/ml ein Gehalt von (12 ± 2) ng/ml endogenem P-Akt (Ser473) bestimmt. Hierbei wird vorausgesetzt, **dass** das zur Erzeugung der Kalibrationskurven benutzte gereinigte Akt in vollständig phosphorylierter Form, d.h. an Ser473 und an Thr308 phosphorylierter Form, vorliegt. Sollte der Anteil von P-Akt (Ser473) an dem gereinigten Akt weniger als 100 % betragen, so reduziert sich der Wert des mit dieser Messung in der aus Rattenherzgewebe hergestellten Probe entsprechend.

### b) Verwendung von Bindungsreagentien mit Zusatz von Akt als Kompetitor

Zur Bestimmung des Anteils von Signalen unspezifischer Bindung an der Messkurve aus Messbereichen, welche mit den Immobilisierungslösungen basierend auf Rattenherzgewebe als Probenmatrix erzeugt wurden, wird in ein fünftes Probenbehältnis auf ein weiteres Array mit gleichartiger Anordnung der Messbereiche wie die anderen untersuchten Arrays eine Mischung der Lösung des Antikörpers "anti-P-Akt (Ser473)" (5 nM) als Bindungsreagenz mit einem Überschuss von Akt (5 µg/ml entsprechend 100 nM) gefüllt und mit den Messbereichen über Nacht inkubiert, wie unter 4.2. beschrieben. Es wird erwartet, dass unter diesen Bedingungen der Anteil durch spezifische Bindung des Antikörpers an immobilisiertes P-Akt (Ser473) hervorgerufener Signale verschwindet, während der Anteil durch unspezifische Bindung hervorgerufener Signale verbleibt.

Die Ergebnisse des Kompetitionsexperiments sind in Fig. 11 dargestellt, zusammen mit den Resultaten der entsprechenden, vorangehend unter 7.2.2.a) besprochenen Messungen ohne Anwesenheit eines Kompetitors in der Lösung des Bindungsreagenz. Ausgefüllte Symbole kennzeichnen jeweils die Ergebnisse ohne Anwesenheit eines Kompetitors (gemessen auf dem vierten Array), leere Symbole die in Gegenwart des Kompetitors gemessenen Signale. Die Fluoreszenzsignale aus Messbereichen, deren Immobilisierungslösungen aus Lysaten von Rattenherzgewebe hergestellt wurden (sowohl zur Bestimmung des natürlich vorkommenden, endogenen P-Akt (Ser473), ohne zusätzlich hinzugefügtes Akt, Messbereichsinhaltskennzahlen 25 bis 31, als auch mit 1000 ng/ml zusätzlich hinzugefügtem Akt, Messbereichsinhaltskennzahlen 37 bis 43, gemäß Fig. 1) sind als Funktion der Proteinkonzentration der Immobilisierungslösung dargestellt (Fig. 11, obere Abszisse). Die in 7.2.1. besprochene Kalibrationskurve für den Nachweis von P-Akt (Ser473) (mit 0.1 mg/ml BSA in den Immobiliserungslösunge) ist aufgetragen als Funktion Konzentration an P-Akt (Ser473) (Fig. 11, untere Abszisse).

In Anwesenheit des Kompetitors zeigen die Signale aus den Messbereichen zur Bestimmung des endogenen p473-Akt eine deutliche Verminderung. Die Differenz zwischen den Messkurven in Abwesenheit und in Anwesenheit des Kompetitors stellt den Signalanteil dar, welcher durch spezifische Bindung hervorgerufen wird.

Für die Messbereiche, deren Immobilsierungslösung zusätzlich Akt im Überschuss (1000 ng/ml) enthielten, wird eine noch deutlichere Reduktion der referenzierten Fluoreszenzsignale beobachtet. Die in Anwesenheit von 100 nM Akt als Kompetitor in Lösung erzeugten Messkurven aus den beiden Segmenten von Messbereichen (zur Bestimmung des endogenen Akt bzw. zur Kontrollmessung mit 1000 ng/ml Akt in der Immobilisierungslösung) fallen wiederum nahezu zusammen. Das verbleibende Restsignal, welches bis zu einer Proteinkonzentration von 0.3 mg/ml deutlich und dann nur noch schwach ansteigt, entspricht dem Signalbeitrag infolge unspezifischer Bindung (an die Proteine der Probenmatrix), welcher naturgemäß mit steigender Oberflächenkonzentration der Proteine ansteigt, bis die Oberfläche der Messbereiche vollständig bedeckt ist.

Fig. 12 illustriert die Bestimmung der durch spezifische Bindung verursachten Signalanteile bei einer Proteinkonzentration von 0.1 mg/ml aus dem Vergleich der Messwerte in Anwesenheit und Abwesenheit des Kompetitors in Lösung (Fig. 12a) und die Bestimmung des Gehalts an endogenem Akt aus dem Vergleich des durch spezifische Bindung ("SB") verursachten Signalanteils mit der Kalibrationskurve (Fig. 12b). Die durch unspezifische Bindung ("NSB") hervorgerufenen Signalanteile (0.037 RFI) sind für die Signale aus Messbereichen mit nur endogenem Akt (0.073 RFI) und solchen mit zusätzlich hinzugegebenen 1000 ng/ml gereinigtem Akt (ca. 10 RFI) innerhalb der Messgenauigkeit gleich hoch. Aus Vergleich der Differenz zwischen Gesamtsignal und unspezifisch hervorgerufenem Signal mit der Kalibrationskurve wird ein Gehalt von (5.3 ± 0.5) ng/ml endogenem P-Akt (Ser473) bestimmt (Fig. 12b). Der Vergleich mit der Bestimmung von Akt in Abschnitt 7.1.2.a), in der durch unspezifische Bindung erzeugte Signalanteile nicht diskriminiert wurden, zeigt, dass etwa 57 % des dort vorläufig bestimmten Gehalts an P-Akt (Ser473) dem Beitrag unspezifischer Bindung zuzuordnen sind.

### c) Verwendung von Bindungsreagentien mit Zusatz von der Probenmatrix ähnlichen Stoffen als Kompetitoren

Zur Bestimmung des Anteils unspezifischer Bindung, welche durch Bindung der Bindungsreagentien an Komponenten der Probenmatrix verursacht werden, wird der Lösung des Antikörpers Rattenserum mit einer Gesamtproteinkonzentration von 0.1 mg/ml hinzugesetzt. Diese Lösung wird dann in ein sechstes Probenbehältnis mit einem sechsten Array wiederum gleichartiger Anordnung der Messbereiche wie das vorgenannte erste und zweite Array gefüllt, entsprechend dem Vorgehen gemäß Abschnitt 4.3. Es wird erwartet, dass unter diesen Voraussetzungen die Anteile durch spezifische Bindung an immobilisiertes P-Akt (Ser473) erzeugter Signale erhalten bleiben und die Anteile durch unspezifische Bindung an die Probenmatrix erzeugter Signale weitgehend verschwinden.

Die Ergebnisse des Kompetitionsexperiments mit Rattenserum in Lösung sind in Fig. 13 dargestellt, zusammen mit den Resultaten der entsprechenden, vorangehend unter 7.2.2.a) besprochenen Messungen ohne Anwesenheit eines Kompetitors in der Lösung des Bindungsreagenz. Ausgefüllte Symbole kennzeichnen jeweils die Ergebnisse ohne Anwesenheit eines Kompetitors (gemessen auf dem vierten Array), leere Symbole die in Gegenwart des Kompetitors gemessenen Signale (gemessen auf dem sechsten Array). Die Fluoreszenzsignale aus Messbereichen, deren Immobilisierungslösungen aus Lysaten von Rattenherzgewebe hergestellt wurden (sowohl zur Bestimmung des natürlich vorkommenden, endogenen P-Akt (Ser473), ohne zusätzlich hinzugefügtes Akt, Messbereichsinhaltskennzahlen 25 bis 31, als auch mit 1000 ng/ml zusätzlich hinzugefügtem Akt, , Messbereichsinhaltskennzahlen 37 bis 43, gemäß Fig. 1) sind als Funktion der Proteinkonzentration der Immobilisierungslösung dargestellt (Fig. 13, obere Abszisse). Die in 7.2.1.a) besprochene Kalibrationskurve für den Nachweis von P-Akt (Ser473) (mit 0.1 mg/ml BSA in den Immobiliserungslösunge) ist aufgetragen als Funktion der Konzentration an P-Akt (Ser473) (Fig. 13, untere Abszisse).

Die Anwesenheit von Rattenserum in Lösung als Kompetitor für unspezifische Bindung in Lösung führt zu keiner signifikanten Verschiebung der Messkurven; die in Anwesenheit und Abwesenheit von Rattenserum gemessenen referenzierten Fluoreszenzsignale sind innerhalb der Messgenauigkeit jeweils identisch.

Hieraus ist zu schließen, dass im Falle des Antikörpers "anti-P-Akt (Ser473)" eine unspezifische Bindung an die Probenmatrix nicht in signifikantem Umfang stattfindet.

### 7.3. Bestimmung des Gehalts an P-Akt (Thr308)

### 7.3.1. Kalibrationskurven für den Nachweis von P-Akt (Thr308)

### a) Verwendung von Bindungsreagentien ohne Zusatz von Kompetitoren

Zur Erstellung von Kalibrationskurven für die Bestimmung der phosphoryliserten Form P-Akt (Thr308) werden Segmente gleichartiger Messbereiche wie für die vorangehend beschriebene Bestimmung von Akt und von P-Akt (Ser473) verwendet.

Segment 1: Array-Reihen I und II mit Messbereichs-Inhaltsnummern 1 - 9 mit aufgebrachten unterschiedlichen Konzentrationen (zwischen 0 ng/ml und 3000 ng/ml) von gereinigtem Akt in Spotting-Puffer mit zusätzlichen 0.1 mg/ml BSA,

Segment 2: Array-Reihen III und IV mit Messbereichs-Inhaltsnummern 13 - 21 mit aufgebrachten unterschiedlichen Konzentrationen (zwischen 0 ng/ml und 3000 ng/ml) von gereinigtem Akt in Spotting-Puffer mit zusätzlichen 0.1 mg/ml Rattenserum. Es wird angenommen, dass das gereinigte Akt in vollständig phosphorylierter Form, d.h. an Ser473 und an Thr308 phosphorylierter Form, vorliegt.

In ein siebtes Probenbehältnis mit einem siebten Array von Messbereichen wird eine Lösung von anti- P-Akt (Thr308) (5 nM) aufgegeben. Die weiteren Verfahrensteilschritte wurden vorangehend unter 4.1 beschrieben. Die Kalibrationskurven des Segments 1 von Messbereichen (erzeugt mit Lösungen enthaltend unterschiedliche Konzentrationen von Akt und dem Spotting-Puffer zugesetzte 0.1 mg/ml BSA), des Segments 2 von Messbereichen (erzeugt mit Lösungen enthaltend unterschiedliche Konzentrationen von Akt und dem Spotting-Puffer zugesetztes 0.1 mg/ml Rattenserum) sind in Fig. 14 (ausgefüllte Symbole) dargestellt.

Bei Konzentrationen oberhalb von 100 ng/ml Akt fallen die Kalibrationskurven von Messbereichen mit co-immobilisiertem BSA und mit co-immobilisiertem Rattenserum zusammen; bei Konzentrationen unterhalb von 100 ng/ml weist die Kalibrationskurve von Messbereichen mit co-immobilisiertem Rattenserum höhere Signalwerte als diejenige mit co-immobilisiertem BSA auf; der Signalunterschied wird dem Beitrag unspezifischer Bindung des Bindungsreagenz an die Inhaltsstoffe des immobilisierten Rattenserums zugeordnet. Die gemessenen Signalwerte aus beiden Arten von Messbereichen sind allerdings relativ niedrig, im Vergleich zu den Fluoreszenzsignalen, die bei der Erstellung der Kalibrationskurven für Akt und P-Akt (Ser473) gemessen worden waren. Für die Bestimmung der unbekannten P-Akt (Thr308)-Konzentrationen in den aus Rattenherzgewebe hergestellten Proben werden die Kalibrationskurven benutzt, welche mithilfe von Immobilisierungslösungen, denen lediglich BSA hinzugefügt wurde, erzeugt wurden.

### b) Verwendung von Bindungsreagentien mit Zusatz von Akt als Kompetitor

Zur Überprüfung des Anteils von Signalen unspezifischer Bindung an den Kalibrationskurven, welche mit den Immobilisierungslösungen mit unterschiedlicher Probenmatrix erstellt wurden, wird in ein achtes Probenbehältnis auf ein weiteres Array mit gleichartiger Anordnung der Messbereiche wie die zuvor untersuchten Arrays eine Mischung der Lösung des Antikörpers "anti-P-Akt (Thr308)" als Bindungsreagenz mit einem Überschuss von Akt (5 µg/ml entsprechend 100 nM) gefüllt und mit den Messbereichen über Nacht inkubiert, wie unter 4.2. beschrieben. Es wird erwartet, dass unter diesen Bedingungen der Anteil durch spezifische Bindung des Antikörpers an immobilisiertes P-Akt (Thr308) hervorgerufener Signale verschwindet, während der Anteil durch unspezifische Bindung hervorgerufener Signale verbleibt.

Es wird nur bei hohen Konzentrationen der Immobilisierungslösungen (oberhalb von 30 ng/ml) eine Verringerung der Signale festgestellt (leere Symbole in Fig. 14, wobei die Signale aus Messbereichen, deren Immobilisierungslösungen Rattenserum enthielten, höher sind als die Signale derjenigen Messbereiche, deren Immobilisierungslösungen, außer den definierten Akt-Konzentrationen, lediglich BSA enthielten. Nur im Bereich von Konzentrationen von mehr als etwa 30 ng/ml übersteigt die Signalabnahme infolge der Anwesenheit von P-Akt (Thr308) als Kompetitor in der Lösung des Bindungsreagenz die experimentell bedingte Signalvariation.

Hieraus ist zu schließen, dass eine unspezifische Bindung des Antikörpers anti- P-Akt (Thr308) sowohl an Messbereiche mit co-immobilisiertem BSA als auch mit co-immobilisierten Serumbestandteilen in erheblichem Ausmaß stattfindet.

### c) Verwendung von Bindungsreagentien mit Zusatz von der Probenmatrix ähnlichen Stoffen als Kompetitoren

Zur Überprüfung des Anteils unspezifischer Bindung an den Fluoreszenzsignalen, welche durch Bindung der Bindungsreagentien an Komponenten der Probenmatrix verursacht werden, wird der Lösung des Antikörpers Rattenserum mit einer Gesamtproteinkonzentration von 0.1 mg/ml hinzugesetzt. Diese Lösung wird dann in ein neuntes Probenbehältnis mit einem neunten Array wiederum gleichartiger Anordnung der Messbereiche wie die vorgenannten Arrays gefüllt, entsprechend dem Vorgehen gemäß Abschnitt 4.3. Es wird erwartet, dass unter diesen Voraussetzungen die Anteile durch spezifische Bindung an immobilisiertes P-Akt (Thr308) erzeugter Signale erhalten bleiben und die Anteile durch unspezifische Bindung an die Probenmatrix erzeugter Signale weitgehend verschwinden.

Figur 15 zeigt mit ausgefüllten Symbolen wiederum die unter Abschnitt 7.3.1.a) besprochenen Kalibrationskurven, die ohne Zusatz von Kompetitoren zu den Bindungsreagentien mit dem siebten Array erzeugt wurden. Die durch Zugabe der Rattenserum enthaltenden Lösung des Antikörpers als Bindungsreagenz mit dem neunten Array erhaltenen Signale unterscheiden sich von diesen Kalibrationskurven nicht messbar. Eine signifikante Abhängigkeit von der angenommenen Konzentration an P-Akt (Thr308) in der Immobilisierungslösung wird erst bei Konzentrationen oberhalb 30 ng/ml festgestellt. Dieses ist konsistent damit, dass bei niedrigeren Konzentrationen gemessene Fluoreszenzsignale auf unspezifische Bindung zurückzuführen sind.

### 7.3.2. Bestimmung des Gehalts von P-Akt (Thr308) in aus Rattenherzgewebe hergestellten Proben

### a) Verwendung von Bindungsreagentien ohne Zusatz von Kompetitoren

Der Nachweis des Gehalts von P-Akt (Thr308) in den aus Rattenherzgewebe hergestellten, komplex zusammengesetzten Proben biologischen Ursprungs erfolgt auf dem Segment von Messbereichen, welche mit den Messbereichs-Inhaltsnummern 25 bis 31 gekennzeichnet sind.

Die zur Erzeugung dieser Messbereiche erzeugten Immobilisierungslösungen stammen aus derselben Stammlösung und wurden durch Verdünnung (siehe Abschnitt 3.) auf unterschiedliche Gesamtproteinkonzentrationen (zwischen 0.025 mg/ml und 0.5 mg/ml) eingestellt.

Das Segment von Messbereichen mit den Messbereichs-Inhaltsnummern 37 bis 43 wurde durch Aufbringung von Immobilisierungsproben aus einer gleichartigen Verdünnungsreihe der Stammlösung aus dem Lysat des Rattenherzgewebes erzeugt, wobei den Immobilisierungsproben aber jeweils noch gereinigtes Akt in einer Konzentration von 1000 ng/ml zugesetzt wurde. Die mithilfe dieses Segments zu messenden Signale sollen der Kontrolle dienen, ob durch Vergleich der zu messenden Fluoreszenzsignale mit der Kalibrationskurve für P-Akt (Thr308) (erstellt mit Messbereichen mit co-immobilisiertem BSA) bei gleicher Gesamtproteinkonzentration (0.1 mg/ml) ein der eingesetzten hohen "Total-Akt"-Konzentration (welche wesentlich höher als der erwartete natürliche, endogene Akt-Gehalt ist) entsprechender hoher Gehalt an P-Akt (Thr308) wiedergefunden wird

Zusammen mit dem unter 7.3.1.a beschriebenen Teil des erfindungsgemäßen Verfahrens wird die Messung durchgeführt mittels Zugabe einer Lösung von anti-P-Akt (Thr308) (5 nM) in das siebte Probenbehältnis mit dem darin befindlichen siebten Array von Messbereichen. Die weiteren Verfahrensteilschritte wurden vorangehend unter 4.1 beschrieben.

Die Ergebnisse (referenzierte Fluoreszenzintensitäten) sind in Fig. 16 dargestellt. Die Fluoreszenzsignale aus Messbereichen, in denen aus Rattenherzgewebe hergestellte Immobilisierungslösungen aufgebracht wurden, sind als Funktion der Gesamtproteinkonzentration dargestellt (Fig. 16, obere Abszissenachse), und die Werte der referenzierten Fluoreszenzsignale der Kalibrationsmessung mit co-immobilisiertem BSA (Proteinkonzentration: 0.1 mg/ml) sind als Funktion der angenommenen Konzentrationen an P-Akt (Thr308) der für diese Messbereiche benutzten Immobilisierungslösungen (Fig. 16, untere Abszissenachse) dargestellt.

Die Signale für P-Akt (Thr308) aus den Messbereichen ohne zugesetztes Akt (d.h. mit in den immobilisierten Proben natürlich vorkommendem, endogenem P-Akt (Thr308)) steigen mit zunehmender Proteinkonzentration bis 0.4 mg/ml an und erreichen dort einen Maximalwert, der sich bei einer Proteinkonzentration von 0.5 mg/ml nicht mehr wesentlich ändert.

Durch Vergleich (illustriert durch die durchbrochene Linie bei einer Proteinkonzentration von 0.1 mg/ml sowie durch die vom Schnittpunkt dieser Linie mit der Messkurve für Signale aus Messbereichen mit nur endogenem P-Akt (Thr308 in Richtung der Kalibrationskurve verlaufenden durchbrochenen Linie) mit der Kalibrationskurve wird (vorläufig) für die aus Rattenherzgewebe hergestellte Probe bei einem Proteingehalt von 0.1 mg/ml ein Gehalt von (130 ± 15) ng/ml endogenem P-Akt (Thr308) bestimmt. Dieser Wert ist allerdings von vornherein als unrealistisch anzusehen, angesichts einer zuvor unter gleichen Bedingungen bestimmten Konzentration von "Total-Akt" in Höhe von 20 ng/ml (ohne Berücksichtigung von Effekten unspezifischer Bindung) bei gleicher Gesamtproteinkonzentration. Der Vergleich (illustriert durch die durchbrochene Linie bei einer Proteinkonzentration von 0.1 mg/ml sowie durch die vom Schnittpunkt dieser Linie mit der Messkurve für Signale aus Messbereichen mit 1000 ng/ml zugegebenem Akt in Richtung der Kalibrationskurve verlaufenden durchbrochenen Linie) der Messkurve mit Signalen aus Messbereichen, zu deren Immobilisierungslösungen 1000 ng/ml gereinigtes Akt zugesetzt wurden, führt zur Bestimmung eines Wertes von (510 ± 10) ng/ml als Wiederfindung ("Recovery").

### b) Verwendung von Bindungsreagentien mit Zusatz von Akt als Kompetitor

Zur Bestimmung des Anteils von Signalen unspezifischer Bindung an der Messkurve aus Messbereichen, welche mit den Immobilisierungslösungen basierend auf Rattenherzgewebe als Probenmatrix erzeugt wurden, wird in ein achtes Probenbehältnis auf ein weiteres Array mit gleichartiger Anordnung der Messbereiche wie das zuerst untersuchte Array eine Mischung der Lösung des Antikörpers "anti P-Akt (Thr308)" (5 nM) als Bindungsreagenz mit einem Überschuss von Akt (5 µg/ml entsprechend 100 nM), gefüllt und mit den Messbereichen über Nacht inkubiert, wie unter 4.3. beschrieben. Es wird erwartet, dass unter diesen Bedingungen der Anteil durch spezifische Bindung des Antikörpers an immobilisiertes P-Akt (Thr308) hervorgerufener Signale verschwindet, während der Anteil durch unspezifische Bindung hervorgerufener Signale verbleibt.

Die Ergebnisse des Kompetitionsexperiments sind in Fig. 17 dargestellt, zusammen mit den Resultaten der entsprechenden, vorangehend unter 7.3.2.a) besprochenen Messungen ohne Anwesenheit eines Kompetitors in der Lösung des Bindungsreagenz. Ausgefüllte Symbole kennzeichnen jeweils die Ergebnisse ohne Anwesenheit eines Kompetitors (gemessen auf dem ersten Array), leere Symbole die in Gegenwart des Kompetitors gemessenen Signale. Die Fluoreszenzsignale aus Messbereichen, deren Immobilisierungslösungen aus Lysaten von Rattenherzgewebe hergestellt wurden (sowohl zur Bestimmung des natürlich vorkommenden, endogenen P-Akt (Thr308), ohne zusätzlich hinzugefügtes Akt, Messbereichsinhaltskennzahlen 25 bis 31, als auch mit 1000 ng/ml zusätzlich hinzugefügtem Akt, , Messbereichsinhaltskennzahlen 37 bis 43, gemäß Fig. 1) sind als Funktion der Proteinkonzentration der Immobilisierungslösung dargestellt (Fig. 15, obere Abszisse). Die in 7.3.1. besprochene Kalibrationskurve für den Nachweis von P-Akt (Thr308) (mit 0.1 mg/ml BSA in den Immobiliserungslösunge) ist aufgetragen als Funktion der angenommenen Konzentration an P-Akt (Thr308) (Fig. 17, untere Abszisse).

In Anwesenheit des Kompetitors zeigen die Signale aus den Messbereichen zur Bestimmung des endogenen P-Akt (Thr308) keinerlei Verminderung. Die Differenz zwischen den Messkurven in Abwesenheit und in Anwesenheit des Kompetitors, welche den durch spezifische Bindung hervorgerufenen Signalanteil darstellen würde, ist innerhalb der Messgenauigkeit gleich Null.

Für die Messbereiche, deren Immobilisierungslösung zusätzlich Akt im Überschuss (1000 ng/ml) enthielten, wird eine vergleichsweise schwache Verminderung der referenzierten Fluoreszenzsignale vor allem im Bereich niedriger Proteinkonzentrationen beobachtet.

Fig. 18 illustriert die Bestimmung der durch spezifische Bindung verursachten Signalanteile bei einer Proteinkonzentration von 0.1 mg/ml aus dem Vergleich der Messwerte in Anwesenheit und Abwesenheit des Kompetitors in Lösung. Für die aus Lösungen von Rattenherzgewebe-Lysaten erzeugten Messbereiche, wo höchstens das Vorkommen endogenen P-Akt (Thr308) erwartet wird, werden Signale von 0.068 RFI aus der Messung ohne Kompetitor und von 0.073 RFI aus der Messung mit 100 nM Kompetitor bestimmt. Der Unterschied zwischen diesen beiden Werten entspricht etwa der experimentell bedingten Signalvariation. Dieses bestätigt, dass unter den vorliegenden Bedingungen kein durch spezifische Bindung erzeugtes Signal gemessen wird, woraus geschlossen werden kann, dass der Gehalt an P-Akt (Thr308) gleich Null ist.

### c) Verwendung von Bindungsreagentien mit Zusatz von der Probenmatrix ähnlichen Stoffen als Kompetitoren

Zur Bestimmung des Anteils unspezifischer Bindung, welche durch Bindung der Bindungsreagentien an Komponenten der Probenmatrix verursacht werden, wird der Lösung des Antikörpers Rattenserum mit einer Gesamtproteinkonzentration von 0.1 mg/ml hinzugesetzt. Diese Lösung wird dann in ein neuntes Probenbehältnis mit einem neunten Array wiederum gleichartiger Anordnung der Messbereiche wie die vorgenannten Arrays gefüllt, entsprechend dem Vorgehen gemäß Abschnitt 4.3. Es wird erwartet, dass unter diesen Voraussetzungen die Anteile durch spezifische Bindung an immobilisiertes P-Akt (Thr308) erzeugter Signale erhalten bleiben und die Anteile durch unspezifische Bindung an die Probenmatrix erzeugter Signale weitgehend verschwinden.

Die Ergebnisse des Kompetitionsexperiments mit Rattenserum in Lösung sind in Fig. 19 dargestellt, zusammen mit den Resultaten der entsprechenden, vorangehend unter 7.3.2.a) besprochenen Messungen ohne Anwesenheit eines Kompetitors in der Lösung des Bindungsreagenz. Ausgefüllte Symbole kennzeichnen jeweils die Ergebnisse ohne Anwesenheit eines Kompetitors (gemessen auf dem siebten Array), leere Symbole die in Gegenwart des Kompetitors gemessenen Signale. Die Fluoreszenzsignale aus Messbereichen, deren Immobilisierungslösungen aus Lysaten von Rattenherzgewebe hergestellt wurden (sowohl zur Bestimmung des natürlich vorkommenden, endogenen P-Akt (Thr308), ohne zusätzlich hinzugefügtes Akt, Messbereichsinhaltskennzahlen 25 bis 31, als auch mit 1000 ng/ml zusätzlich hinzugefügtem Akt, gemäß Fig. 1) sind als Funktion der Proteinkonzentration der Immobilisierungslösung dargestellt (Fig. 19, obere Abszisse). Die in 7.3.1.a) besprochene Kalibrationskurve für den Nachweis von P-Akt (Thr308) (mit 0.1 mg/ml BSA in den Immobiliserungslösunge) ist aufgetragen als Funktion der angenommenen Konzentration an P-Akt (Thr308) (Fig. 19, untere Abszisse).

Die Anwesenheit von Rattenserum in Lösung als Kompetitor für unspezifische Bindung in Lösung führt zu messbaren Abnahmen der Fluoreszenzsignale, was nochmals den Ursprung unspezifischer Bindung bestätigt.

## Patentansprüche

1. Ein Mikroarray zur quantitativen Bestimmung eines oder mehrerer Analyten in einer oder mehreren komplex zusammengesetzten Proben biologischen Urspungs, umfassend
- einen festen Träger,
- eine erste Vielzahl von diskreten Messbereichen, in denen direkt oder vermittelt über eine Haftvermittlungsschicht jeweils kleine Mengen komplex zusammengesetzter Proben biologischen Ursprungs in verdünnter oder unverdünnter Form immobilisiert sind,
**dadurch gekennzeichnet,**
**dass** mindestens eine zweite Vielzahl von Messbereichen in besagtem Array auf dem festen Träger bereitgestellt ist, in welchen Messbereichen den nachzuweisenden Analyten gleichartige Stoffe in unterschiedlichen Konzentrationen immobilisiert sind, welche geeignet sind, mittels Kontaktierung des Mikroarrays mit einer ersten Lösung enthaltend ein oder mehrere Bindungsreagentien als spezifische Bindungspartner für die in der ersten Vielzahl von diskreten Messbereichen in den aufgebrachten komplex zusammengesetzten Proben biologischen Ursprungs enthaltenen, nachzuweisenden Analyten und die in der zweiten Vielzahl von diskreten Messbereichen enthaltenen, besagten nachzuweisenden Analyten gleichartigen Stoffe, und, gegebenenfalls falls erforderlich, ein oder mehr Detektionsreagentien, wobei die Aufbringung von Bindungs- und Detektionsreagentien gleichzeitig oder sequentiell erfolgen kann, und nachfolgende ortsaufgelöste Messung von ersten optischen Signalen, welche von diskreten Messbereichen eines oder mehrerer Arrays, welche mit der ersten Lösung in Kontakt gebracht wurden, ausgehen, sowie Aufzeichnung dieser ersten optischen Signale, eine Kalibrationskurve für besagte in den immobilisierten komplex zusammengesetzten Proben enthaltenen, quantitativ nachzuweisenden Analyten zu erzeugen.

2. Ein Mikroarray nach Anspruch 1, **dadurch gekennzeichnet, dass** eine dritte Vielzahl von Messbereichen in besagtem Array auf dem festen Träger bereitgestellt ist, in denen jeweils kleine Mengen komplex zusammengesetzter Proben biologischen Ursprungs in verdünnter oder unverdünnter Form und zusätzlich diesen zugesetzte bekannte Mengen von den nachzuweisenden Analyten gleichartigen Stoffen immobilisiert sind.

3. Ein Mikroarray nach einem der Ansprüche 1 oder 2, **dadurch gekennzeichnet, dass** eine vierte Vielzahl von Messbereichen in besagtem Array auf dem festen Träger bereitgestellt ist, in welchen Stoffe, welche ähnlicher Art wie in der Probenmatrix der in der ersten Vielzahl von Messbereichen aufgebrachten Proben enthaltene Stoffe sind, immobilisiert sind.

4. Ein Mikroarray nach einem der Ansprüche 1 bis 3, **dadurch gekennzeichnet, dass** die Messbereiche der zweiten Vielzahl von Messbereichen Stoffe umfassen, welche ähnlicher Art wie in der Probenmatrix der in der ersten Vielzahl von Messbereichen aufgebrachten Proben enthaltene Stoffe sind.

5. Ein Mikroarray nach einem der Ansprüche 3 oder 4, **dadurch gekennzeichnet, dass** die Stoffe, welche ähnlicher Art wie in der Probenmatrix der in der ersten Vielzahl von Messbereichen aufgebrachten Proben enthaltene Stoffe sind, aus der Gruppe stammen, welche Albumine, insbesondere Rinderserumalbumin, Immunoglobuline oder verdünntes Serum umfasst.

6. Ein Mikroarray nach einem der Ansprüche 1 bis 5, **dadurch gekennzeichnet, dass** eine fünfte Vielzahl von Messbereichen in besagtem Array auf dem festen Träger bereitgestellt ist, welche Zwecken einer Referenzierung dienen.

7. Ein Mikroarray nach Anspruch 6, **dadurch gekennzeichnet, dass** die Messbereiche der fünften Vielzahl von Messbereichen Stoffe umfassen, welche ausgewählt sind aus der Gruppe, welche Massenlabel und Lumineszenzlabel umfasst.

8. Ein Verfahren zur quantitativen Bestimmung eines oder mehrerer Analyten in einer komplex zusammengesetzten Probe biologischen Ursprungs umfassend die folgenden Schritte:
(1) Bereitstellung einer oder mehrerer komplex zusammengesetzter Proben biologischen Ursprungs,
(2) Bereitstellung einer Vielzahl von Lösungen von den in den komplex zusammengesetzten Proben nachzuweisenden Analyten gleichartigen Stoffen in unterschiedlichen Konzentrationen,
(3) Bereitstellung mindestens eines festen Trägers,
(4) Erzeugung einer ersten Vielzahl von diskreten Messbereichen als Teil eines Mikroarrays durch Aufbringung kleiner Mengen der komplex zusammengesetzten Proben biologischen Ursprungs, in verdünnter oder unverdünnter Form, an diskreten Orten direkt auf dem festen Träger oder nach vorheriger Aufbringung einer Haftvermittlungsschicht auf besagter Haftvermittlungsschicht auf dem festen Träger,
(5) Erzeugung einer zweiten Vielzahl von diskreten Messbereichen als Teil besagten Mikroarrays durch Aufbringung jeweils kleiner Mengen der Vielzahl von Lösungen von den in den komplex zusammengesetzten Proben nachzuweisenden Analyten gleichartigen Stoffen in unterschiedlichen Konzentrationen,
(6) Kontaktierung des Mikroarrays mit einer ersten Lösung enthaltend ein oder mehrere Bindungsreagentien als spezifische Bindungspartner für die in der ersten Vielzahl von diskreten Messbereichen in den aufgebrachten komplex zusammengesetzten Proben biologischen Ursprungs enthaltenen, nachzuweisenden Analyten und die in der zweiten Vielzahl von diskreten Messbereichen enthaltenen, besagten nachzuweisenden Analyten gleichartigen Stoffe, und, gegebenenfalls falls erforderlich, ein oder mehr Detektionsreagentien, wobei die Aufbringung von Bindungs- und Detektionsreagentien gleichzeitig oder sequentiell erfolgen kann,
(7) ortsaufgelöste Messung von ersten optischen Signalen, welche von der ersten und zweiten Vielzahl diskreter Messbereiche des Mikroarrays ausgehen, Aufzeichnung der ersten optischen Signale aus der ersten Vielzahl diskreter Messbereiche als für deren Kontaktierung mit der ersten Lösung charakteristische Signale, Aufzeichnung der ersten optischen Signale aus der zweiten Vielzahl diskreter Messbereiche als für deren Kontaktierung mit der ersten Lösung in Abhängigkeit von der Konzentration der in besagten Messbereichen enthaltenen, den in den komplex zusammengesetzten Proben enthaltenen Analyten gleichartigen Stoffen, charakteristische Signale,
(8) Erstellung von Kalibrationskurven für die in den komplex zusammengesetzten Proben nachzuweisenden Analyten aus den aufgezeichneten optischen Signalen aus der zweiten Vielzahl diskreter Messbereiche, sofern erforderlich nach vorheriger Subtraktion von Hintergrundsignalen und geeigneter Referenzierung,
(9) quantitative Bestimmung der in den komplex zusammengesetzten Proben enthaltenen nachzuweisenden Analyten durch Vergleich der aufgezeichneten ersten optischen Signale aus der ersten Vielzahl diskreter Messbereiche mit den Kalibrationskurven für die jeweiligen Analyten, sofern erforderlich nach vorheriger Subtraktion von Hintergrundsignalen und geeigneter Referenzierung.

9. Verfahren nach Anspruch 8 umfassend die weiteren folgenden Schritte:
(10a) Aufbringung einer zweiten Lösung, enthaltend, zusätzlich zu den ein oder mehr Bindungsreagentien und gegebenenfalls ein oder mehr Detektionsreagentien der in Schritt (6) hinzugegebenen ersten Lösung, eine bekannte hohe Konzentration von Verbindungen, welche gleichartig zu den in diskreten Messbereichen aufgebrachten komplex zusammengesetzten Proben biologischen Ursprungs enthaltenen, nachzuweisenden Analyten sind, als Kompetitoren zu den in diskreten Messbereichen aufgebrachten komplex zusammengesetzten Proben biologischen Ursprungs enthaltenen, nachzuweisenden Analyten um die spezifische Bindung besagter Bindungsreagentien und gegebenenfalls zusätzlich hinzugegebener Detektionsreagentien, auf ein oder mehr der in Schritt (4) erzeugten Arrays von diskreten Messbereichen und/oder
(10b) Aufbringung einer dritten Lösung, enthaltend, zusätzlich zu den ein oder mehr Bindungsreagentien und gegebenenfalls ein oder mehr Detektionsreagentien der in Schritt (6) hinzugegebenen ersten Lösung, eine bekannte hohe Konzentration von Stoffen, welche ähnlicher Art wie in der Probenmatrix der in Schritt (4) aufgebrachten Proben enthaltene Stoffe sind, zwecks Kompetition zu den in diskreten Messbereichen aufgebrachten komplex zusammengesetzten Proben biologischen Ursprungs enthaltenen Stoffen der Probenmatrix um unspezifische Bindung besagter Bindungsreagentien und gegebenenfalls zusätzlich hinzugegebener Detektionsreagentien, auf ein oder mehr der in Schritt (4) erzeugten Arrays von diskreten Messbereichen,
(11) ortsaufgelöste Messung von zweiten und/oder dritten optischen Signalen, welche von diskreten Messbereichen eines oder mehrerer Arrays, welche in Schritt (10a) mit der zweiten Lösung bzw. in Schritt (10b) mit der dritten Lösung in Kontakt gebracht wurden, ausgehen,
(12) Aufzeichnung dieser zweiten und/oder dritten optischen Signale und
(13) Vergleich der ersten und zweiten und/oder dritten optischen Signale.

10. Verwendung eines Mikroarrays nach einem der Ansprüche 1 bis 7 und/oder eines Verfahrens nach einem der Ansprüche 8 oder 9 zu quantitativen und/oder qualitativen Analysen zur Bestimmung chemischer, biochemischer oder biologischer Analyten in Verfahren des Screenings von Wirkstoffbibliotheken zur Effizienzbestimmung in der Pharmaforschung, der Kombinatorischen Chemie, der Klinischen und Präklinischen Entwicklung, zur Identifizierung, Validierung und Kontrolle ("monitoring") von biologischen oder chemischen Markerstoffen ("biomarkers"), zur Identifizierung und Verifizierung von Signaltransduktionswegen in der Proteomforschung und Systembiologie, zum Affinitätsscreening und insbesondere zum Antikörperscreening, zu Echtzeitbindungsstudien und zur Bestimmung kinetischer Parameter im Affinitätsscreening und in der Forschung, zu qualitativen und quantitativen Analytbestimmungen, insbesondere für die DNA- und RNA-Analytik und die Bestimmung von genomischen oder proteomischen Unterschieden im Genom bzw. Proteom, wie beispielsweise Einzelnukleotid-Polymorphismen, zur Messung von Protein-DNA-Wechselwirkungen, zur Bestimmung von Steuerungsmechanismen für die m-RNA-Expression und für die Protein(bio)synthese, für die Erstellung von Toxizitätsstudien sowie für die Bestimmung von Expressionsprofilen, insbesondere zur Erstellung von zellulären Expressionsprofilen von krankhaften und gesunden Zellpopulationen, mit und ohne äußere Stimulation, und deren Vergleich, zur Untersuchung der zeitlichen Entwicklung derartiger Expressionsprofile über Zeiträume von Minuten, Stunden, Tagen, Wochen, Monaten oder Jahren, zur Bestimmung von biologischen und chemischen Markerstoffen, wie mRNA, Proteinen, Peptiden oder niedermolekularen organischen (Boten)Stoffen, sowie zum Nachweis von Antikörpern, Antigenen, Pathogenen oder Bakterien in der pharmazeutischen Produktforschung und -entwicklung, der Human-und Veterinärdiagnostik, der Agrochemischen Produktforschung und -entwicklung, der symptomatischen und präsymptomatischen Pflanzendiagnostik, zur Patientenstratifikation in der pharmazeutischen Produktentwicklung und für die therapeutische Medikamentenauswahl, zum Nachweis von Pathogenen, Schadstoffen und Erregern, insbesondere von Salmonellen, Prionen, Viren und Bakterien, insbesondere in der Lebensmittel- und Umweltanalytik.
